(19) Europäisches Patentamt
European Patent Office
Office européen des brevets

(11) EP 4 578 464 A1

(12) **EUROPEAN PATENT APPLICATION**
published in accordance with Art. 153(4) EPC

(43) Date of publication:
**02.07.2025 Bulletin 2025/27**

(21) Application number: **23856675.6**

(22) Date of filing: **24.08.2023**

(51) International Patent Classification (IPC):
***A61K 47/68** (2017.01)* ***A61K 47/65** (2017.01)*
***A61K 9/08** (2006.01)* ***A61P 35/00** (2006.01)*

(52) Cooperative Patent Classification (CPC):
**A61K 9/08; A61K 47/65; A61K 47/68; A61P 35/00**

(86) International application number:
**PCT/CN2023/114566**

(87) International publication number:
**WO 2024/041587 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.08.2022 CN 202211029543**

(71) Applicants:
- **Genequantum Healthcare (Suzhou) Co., Ltd.**
  **Suzhou, Jiangsu 215123 (CN)**
- **GENEQUANTUM MEDICINE (SUZHOU) CO., LTD.**
  **Suzhou, Jiangsu 215124 (CN)**

(72) Inventors:
- **LI, Xuesong**
  **Suzhou, Jiangsu 215123 (CN)**
- **QIN, Gang**
  **Suzhou, Jiangsu 215123 (CN)**
- **SONG, Paul H.**
  **Suzhou, Jiangsu 215123 (CN)**

(74) Representative: **Zwicker, Jörk**
**ZSP Patentanwälte PartG mbB**
**Hansastraße 32**
**80686 München (DE)**

Remarks:
The complete document including Reference Table(s) and the Sequence Listing(s) can be downloaded from the EPO website

(54) **PHARMACEUTICAL COMPOSITION OF ANTIBODY DRUG CONJUGATE**

(57) The present disclosure relates to the field of biopharmaceuticals, and in particular, to a pharmaceutical composition of an antibody-conjugated drug.

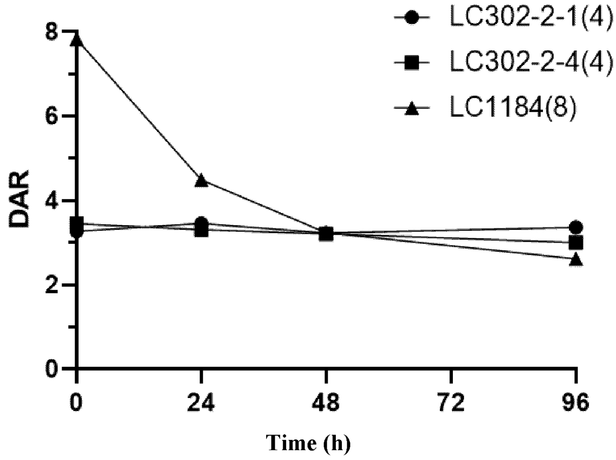


FIG. 8



EP 4 578 464 A1

## Description

### TECHNICAL FIELD

The present disclosure relates to the field of biopharmaceuticals and, in particular, to a pharmaceutical composition of an antibody-conjugated drug.

### BACKGROUND

HER2 has been found to be overexpressed in several types of cancer, including breast cancer and gastric cancer, and it has been proved to be a promising target for cancer therapy. Several HER2-targeting therapeutic modalities have been approved, including HER2 tyrosine kinase inhibitors (lapatinib and tucatinib), therapeutic HER2 antibodies (Herceptin and Perjeta), and HER2 antibody-conjugated drugs (ADCs) (Kadcyla and Enhertu). These therapeutic agents have significantly improved the survival rate of the patients with HER2-positive breast cancer and gastric cancer. Notably, Enhertu has demonstrated significant efficacy not only in patients with high expression of HER2 but also shown signs of clinical benefit in patients with moderate/low expression of HER2. This could potentially benefit a broader population of patients with HER2-expressing cancers. Enhertu, although highly effective, causes interstitial lung disease in no less than 10% of patients, which limits its use in some patients.

Enhertu, other commercially available ADCs, and most ADCs in clinical trials are prepared through chemical conjugation, which uses a thiosuccinimide structure (thiosuccinimide linkage) to conjugate small-molecule drugs to targeting antibodies or proteins. The thiosuccinimide structure is formed by the reaction between sulfhydryl and maleimide. However, the thiosuccinimide linkage is not stable. In biological systems, the retro-Michael addition or the sulfhydryl-sulfhydryl exchange leads to the shedding of cytotoxins from ADCs, which may result in off-target toxicity and thereby reduce the safety and limit the clinical applications.

### SUMMARY

The inventors developed a new ADC that addresses the above problems in the prior art. During the research and development, the inventors further found that the stability of the ADC is affected by the formulation. Therefore, the inventors have developed a pharmaceutical composition based on the ADC. The pharmaceutical composition has the advantages of high stability, long-term storage, and prevention of toxicity due to degradation of the ADC and can be used as a pharmaceutical formulation of the ADC.

In a first aspect, the present disclosure provides an antibody-conjugated drug (also referred to herein as "antibody-drug conjugate", "conjugate", or "ADC") having the structure of formula (III):

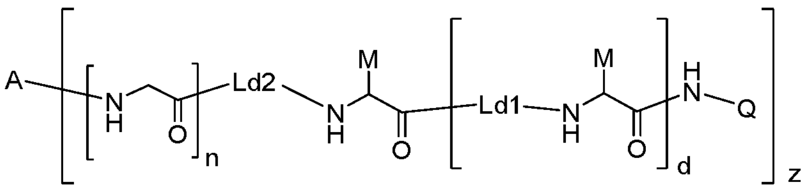
formula (III)

wherein,

Q is hydrogen or $L^2$-$L^1$-B-P;

M is hydrogen or LKa-$L^2$-$L^1$-B-P;

each LKa is independently selected from

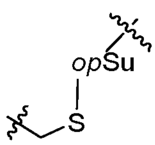 , 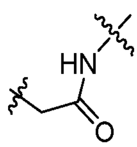 , and 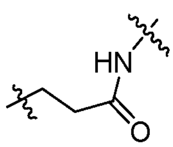 ;

*opSu* is

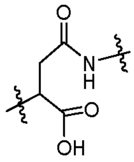,
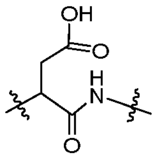,

or a mixture thereof;

each B is independently absent or a combination of 1) and 2) below: 1) a self-immolative spacer Sp1; and 2) a bond, or one divalent group, or a combination of two or more divalent groups, wherein the divalent group is selected from: $-CR^1R^2-$, $C_{1-10}$ alkylene, and -(CO)-; preferably, B is $-NH-CH_2-U-$, or is absent, or is $-NH-CH_2-U-(CH_2)_g-(CO)-$; g is any integer from 1 to 6, preferably 1;

provided that Q and M are not both hydrogen;

P is a payload linked to the B moiety or the $L^1$ moiety;

A is a targeting molecule linked to the $G_n$ moiety; G is glycine;

$L^1$ is cleavable sequence 1 comprising an amino acid sequence capable of being cleaved by an enzyme, and the cleavable sequence 1 comprises 1-10 amino acids;

$L^2$ is a bond or $C_{2-20}$ alkylene, wherein one or more $-CH_2-$ structures in the alkylene are optionally replaced by the following groups: $-CR^3R^4-$, -O-, -(CO)-, $-S(=O)_2-$, $-NR^5-$, $-N^{\oplus}R^6R^7-$, $C_{4-10}$ cycloalkylene, $C_{4-10}$ heterocyclylene, and phenylene, wherein the cycloalkylene, heterocyclylene, and phenylene are each independently unsubstituted or substituted with at least one substituent selected from halogen, $-C_{1-10}$ alkyl, $-C_{1-10}$ haloalkyl, $-C_{1-10}$ alkylene-NH-$R^8$, and $-C_{1-10}$ alkylene-O-$R^9$;

Ld2 and each Ld1 are independently a bond, or are selected from $-NH-C_{1-20}$ alkylene-(CO)- and $NH-(PEG)_i-(CO)-$; or are natural amino acids or oligomeric natural amino acids with a degree of polymerization of 2-10 each independently unsubstituted or substituted with $-(PEG)_j-R^{11}$ on the side chain; or are natural amino acids or oligomeric natural amino acids with a degree of polymerization of 2-10 substituted with $-(PEG)_j-R^{11}$ on the side chain, wherein $-(PEG)_j-R^{11}$ has carbonyl linked therebetween;

$-(PEG)_i-$ and $-(PEG)_j-$ are each a PEG fragment comprising a specified number of consecutive $-(O-C_2H_4)-$ structural units or consecutive $-(C_2H_4-O)-$ structural units, optionally with additional $C_{1-10}$ alkylene at one terminal;

$R^1$, $R^2$, $R^3$, $R^4$, $R^5$, $R^6$, $R^7$, $R^8$, and $R^9$ are each independently selected from hydrogen, halogen, $-C_{1-10}$ alkyl, $-C_{1-10}$ haloalkyl, and $C_{4-10}$ cycloalkylene;

$R^{11}$ is $C_{1-10}$ alkyl;

n is any integer from 2 to 20;

d is 0 or any integer from 1 to 6;

each i is independently an integer from 1 to 100, preferably from 1 to 20; preferably, each i is independently an integer from 1 to 12, more preferably from 2 to 8, particularly 4;

each j is independently an integer from 1 to 100, preferably from 1 to 20; preferably, each j is independently an integer from 1 to 12, more preferably from 8 to 12 or from 8 to 13, particularly 8, 9, 12, or 13.

z is an integer from 1 to 20.

In a second aspect, the present disclosure provides a pharmaceutical composition comprising an active ingredient, a buffering agent, a stabilizer, and optionally a surfactant, wherein the active ingredient comprises the conjugate of formula (III) described above.

In some embodiments, the buffer is selected from an acetic acid buffer, a histidine buffer, a citric acid buffer, and a phosphoric acid buffer. In some embodiments, the buffer is an acetic acid buffer or a histidine buffer. In some embodiments, the buffer is a histidine buffer. In some embodiments, the concentration of the buffer is 10-50 mM; preferably, the pH of the buffer is 5.0-6.5 or 5.5-6.4.

In some embodiments, the stabilizer is selected from sucrose and trehalose, preferably sucrose. In some embodiments, the concentration of the stabilizer is 1-20% (w:v).

In some embodiments, the pH of the pharmaceutical composition is 5.0-6.5, preferably 5.2-6.0, more preferably about 5.5.

In some embodiments, the surfactant is a polysorbate; preferably, the concentration of the surfactant is 0.01-10 mg/mL.

In some embodiments, the present disclosure provides a pharmaceutical formulation comprising an active ingredient of formula (III), wherein the pharmaceutical formulation comprises:

20 mM acetic acid buffer at pH 5.0, 9% (w:v) sucrose, 20 mg/mL active ingredient; or

20 mM histidine buffer at pH 5.5, 9% (w:v) sucrose, 20 mg/mL active ingredient; or

20 mM histidine buffer at pH 6.0, 9% (w:v) sucrose, 20 mg/mL active ingredient; or

20 mM citric acid buffer at pH 6.0, 9% (w:v) sucrose, 20 mg/mL active ingredient; or

20 mM phosphoric acid buffer at pH 7.0, 9% (w:v) sucrose, 20 mg/mL active ingredient; or

20 mM histidine buffer at pH 5.5, 8.1% (w:v) trehalose (or 9% trehalose dihydrate), 0.2 mg/mL polysorbate 20, 20 mg/mL active ingredient; or

20 mM histidine buffer at pH 5.5, 9% (w:v) sucrose, 0.2 mg/mL polysorbate 20, 20 mg/mL active ingredient; or

20 mM histidine buffer at pH 5.5, 9% (w:v) sucrose, 0.2 mg/mL polysorbate 80, 20 mg/mL active ingredient;

wherein the pharmaceutical formulation is a powder formulation or a liquid formulation.

In a third aspect, the present disclosure provides use of the pharmaceutical composition of the present disclosure in the manufacture of a medicament for treating a disease, wherein the disease is a tumor or an autoimmune disease.

## BRIEF DESCRIPTION OF THE DRAWINGS

FIG. 1 shows the efficacy of conjugates and corresponding payloads in the SK-BR-3 cell line with high expression of HER2.

FIG. 2 shows the efficacy of conjugates and corresponding payloads in the NCI-N87 cell line with high expression of HER2.

FIG. 3 shows the efficacy of conjugates in the CFPAC-1 cell line with low expression of HER2.

FIG. 4 shows the efficacy of conjugates in the NCI-H2110 cell line with low expression of HER2.

FIG. 5 shows the efficacy of conjugates in the HER2-negative MDA-MB-468 cell line.

FIG. 6 shows the change of mean tumor volume over time in SCID Beige mice of the JIMT1 CDX model to which 5 mg/kg LC1184(8), LC1184(4), LC301-2-1(2), LC302-2-1(4), and LC302-2-4(4) are administrated.

FIG. 7 shows the change of mean tumor volume over time in BALB/c nude mice of the Capan-1 CDX model to which 5 mg/kg LC1184(8), LC1184(4), LC301-2-1(2), LC302-2-1(4), and LC302-2-4(4) are administrated.

FIG. 8 shows the *ex vivo* serum stability of conjugates after 0, 24, 48 and 96 h of incubation in mixed human serum.

## DETAILED DESCRIPTION

Specific embodiments are provided below to illustrate the technical content of the present disclosure. Other advantages and effects of the present disclosure will be readily understood by those skilled in the art from the content disclosed in the specification. The present disclosure may also be implemented or applied through other different embodiments. Various modifications and changes may be made by those skilled in the art without departing from the spirit of the present disclosure.

### Definitions

Unless otherwise defined hereinafter, all technical and scientific terms used herein have the same meaning as commonly understood by those skilled in the art. The techniques used herein are those commonly understood in the art, including variants and equivalent substitutions that are apparent to those skilled in the art. While the following terms are believed to be readily understood by those skilled in the art, the following definitions are set forth to better illustrate the present disclosure. When referring to a trade name herein, it is intended to refer to its corresponding commercial product or its active ingredient. All patents, published patent applications, and publications cited herein are incorporated herein by reference.

When a certain amount, concentration, or another value or parameter is set forth in the form of a range, a preferred range, or a preferred upper limit or a preferred lower limit, it should be understood that it is equivalent to specifically indicating any range formed by combining any upper limit or preferred value with any lower limit or preferred value, regardless of whether the range is explicitly recited. Unless otherwise indicated, the numerical ranges set forth herein are intended to include the endpoints of the ranges, as well as all integers and fractions (decimals) within the ranges. For example, the expression "i is an integer from 1 to 20" means that i is any integer from 1 to 20. For example, i may be 1, 2, 3, 4, 5, 6, 7, 8, 9, 10, 11, 12, 13, 14, 15, 16, 17, 18, 19, or 20. Other similar expressions such as j, k, and g should also be understood in a similar manner.

As used herein, the "%" related to a component in the present disclosure specifically refers to weight/volume (w/v) percentage, wherein the weight unit may be g and the volume unit may be mL. For example, 1% stabilizer in a solution means that 1 g of stabilizer is present in 100 mL of solution or the stabilizer has a content of 0.01 g/mL.

The amount of the buffering agent in the present disclosure means the total amount of the buffer pair in the buffer system constituting the buffering agent. In some embodiments, the molar concentration is taken as the unit of the amount of the buffering agent (or buffer), the value of which refers to the molar concentration of the buffer pair in the buffer system of the buffering agent (or buffer). For example, when the histidine buffering agent consisting of histidine and histidine hydrochloride is used as the buffering agent, a given concentration of histidine buffering agent (e.g., 20 mM) is the combined concentration of histidine and histidine hydrochloride (e.g., histidine at 15 mM and histidine hydrochloride at 5 mM, or histidine at 3.6 mM and histidine hydrochloride at 16.4 mM).

Trehalose is generally present as trehalose dihydrate in a solid state, so in some embodiments, the formulation is prepared using trehalose dihydrate, or using other forms of trehalose in the corresponding amounts, and the resulting formulation contains the same concentration of trehalose. When trehalose dihydrate is used herein to describe the amount of trehalose in the formulation, the formulation may contain such an amount of trehalose dihydrate or a corresponding amount of trehalose or other forms of trehalose or combinations thereof, and vice versa.

Unless the context clearly dictates otherwise, the singular forms "a", "an", and "the" include the plural forms. The expression "one or more" or "at least one" may denote 1, 2, 3, 4, 5, 6, 7, 8, 9, or more.

The terms "about" and "approximately", when used together with a numerical variable, generally mean that the value of the variable and all values of the variable are within experimental error (e.g., within a 95% confidence interval of the mean) or within $\pm 10\%$ or more of a specified value.

The term "stoichiometric ratio" refers to matching various substances according to a certain amount by weight. For example, in the present disclosure, the active ingredient is mixed with a filler, a binder, and a lubricant in a specified weight ratio.

The term "optional" or "optionally" means that the subsequently described event may, but not necessarily, occur, and that the description includes instances where the event or circumstance occurs or does not occur.

The expressions "comprise", "include", "contain" and "have" are open-ended and do not exclude additional unrecited elements, steps, or ingredients. The expression "consisting of" excludes any element, step, or ingredient not designated. The expression "consisting essentially of" means that the scope is limited to the designated elements, steps, or ingredients, as well as elements, steps, or ingredients that are optionally present and do not substantially affect the essential and novel characteristics of the claimed subject matter. It should be understood that the expression "comprise" encompasses the expressions "consisting essentially of" and "consisting of".

The term "targeting molecule" refers to a molecule that has affinity for a particular target (e.g., a receptor, a cell surface protein, a cytokine, a tumor-specific antigen, or the like). A targeting molecule can deliver the payload to a specific

site *in vivo* by targeted delivery. A targeting molecule can recognize one or more targets. The specific target sites are defined by the target it recognizes. For example, a targeting molecule that targets a receptor can deliver the cytotoxin to sites containing a large number of receptors. Examples of targeting molecules include, but are not limited to, antibodies, antibody fragments, binding proteins for a given antigen, antibody mimetics, scaffold proteins having affinity for a given target, ligands, and the like.

As used herein, the term "antibody" is used in a broad manner and specifically includes intact monoclonal antibodies, polyclonal antibodies, monospecific antibodies, multispecific antibodies (e.g., bispecific antibodies), and antibody fragments so long as they possess the desired biological activity. An antibody may be of any subtype (e.g., IgG, IgE, IgM, IgD, and IgA) or subclass and may be derived from any suitable species. In some embodiments, the antibody is of human or murine origin. The antibody may also be a fully human antibody, a humanized antibody, or a chimeric antibody prepared by recombinant methods.

The monoclonal antibody used herein refers to an antibody obtained from a population of substantially homogeneous antibodies, i.e., the individual antibodies composing the population are identical except for a few possible natural mutations. Monoclonal antibodies are highly specific for a single antigenic site, different epitopes of the same antigen, multiple antigenic sites. "Monoclonal" means that the characteristics of the antibody are derived from a population of substantially homogeneous antibodies and it should not be construed as requiring some particular methods to produce the antibody.

An intact or full-length antibody essentially comprises an antigen-binding variable region, a light chain constant region (CL), and a heavy chain constant region (CH), and it may include CH1, CH2, CH3, and CH4, depending on the subtype of the antibody. The antigen-binding variable region (also referred to as fragment variable region or Fv fragment) typically comprises a light chain variable region (VL) and a heavy chain variable region (VH). The constant region may be a constant region having a natural sequence (e.g., a constant region having a human natural sequence) or an amino acid sequence variant thereof. The variable region recognizes and interacts with the target antigen. The constant region can be recognized by and interact with the immune system.

An antibody fragment may comprise a portion of an intact antibody, preferably the antigen-binding region or variable region thereof. Examples of antibody fragments include Fab, Fab', $F(ab')_2$, an Fd fragment consisting of the VH and CH1 domains, an Fv fragment, a single-domain antibody (dAb) fragment, and an isolated complementarity determining region (CDR). The Fab fragment is an antibody fragment obtained by digesting a full-length immunoglobulin with papain or a fragment of the same structure produced, for example, by recombinant expression. The Fab fragment comprises a light chain (comprising a VL and a CL) and another chain comprising the variable region of the heavy chain (VH) and one constant region of the heavy chain (CH1). The $F(ab')_2$ fragment is an antibody fragment obtained by digesting an immunoglobulin with pepsin at pH 4.0-4.5 or a fragment of the same structure produced, for example, by recombinant expression. The $F(ab')_2$ fragment substantially comprises two Fab fragments, wherein each heavy chain moiety comprises a few additional amino acids, including cysteines that form a disulfide bond linking the two fragments. The Fab' fragment is a fragment that comprises half of an $F(ab')_2$ fragment (one heavy chain and one light chain). The antibody fragment may comprise multiple chains linked together, for example, by a disulfide bond and/or by a peptide linker. Examples of antibody fragments also include single-chain Fv (scFv), Fv, dsFv, diabody, Fd, and Fd' fragments, as well as other fragments, including modified fragments. An antibody fragment typically comprises at least or about 50 amino acids, typically at least or about 200 amino acids. The antigen-binding fragment may include any antibody fragment that, when inserted into an antibody framework (e.g., by replacing the corresponding region), can result in an antibody that immunospecifically binds to an antigen.

The antibodies of the present disclosure can be prepared using techniques well known in the art, such as the following techniques or a combination thereof: recombinant techniques, phage display techniques, synthetic techniques, or other techniques known in the art. For example, a genetically engineered recombinant antibody (or an antibody mimetic) can be expressed through a suitable culture system (e.g., *E.coli* or mammalian cells). The engineering may refer to, for example, the introduction of a ligase-specific recognition sequence at its terminals.

HER2 refers to human epidermal growth factor receptor-2, which belongs to the epidermal growth factor receptor (EGFR) tyrosine kinase family. In the present application, the terms ErbB2 and HER2 have the same meaning and can be used interchangeably.

As used herein, the term "antibody-conjugated drug" is referred to as a "conjugate". Examples of conjugates include, but are not limited to, antibody-drug conjugates.

A small molecule compound refers to a molecule that is comparable in size to organic molecules commonly used in pharmaceuticals. The term does not encompass biological macromolecules (e.g., proteins, nucleic acids, etc.), but encompasses low molecular weight peptides or derivatives thereof, such as dipeptides, tripeptides, tetrapeptides, pentapeptides, etc. Typically, the small molecule compound may have a molecular weight of, for example, about 100 to about 2000 Da, about 200 to about 1000 Da, about 200 to about 900 Da, about 200 to about 800 Da, about 200 to about 700 Da, about 200 to about 600 Da, or about 200 to about 500 Da.

The cytotoxin refers to a substance that inhibits or prevents the expression activity of a cell and the function of a

cell and/or causes the destruction of a cell. Cytotoxins currently commonly used in ADCs are more toxic than chemotherapeutic drugs. Examples of cytotoxins include, but are not limited to, drugs that target the following targets: microtubule cytoskeleton, DNA, RNA, kinesin-mediated protein transport, and regulation of apoptosis. The drug targeting the microtubule cytoskeleton may be, e.g., a microtubule stabilizer or a tubulin polymerization inhibitor. Examples of microtubule stabilizers include, but are not limited to, taxanes. Examples of tubulin polymerization inhibitors include, but are not limited to, maytansinoids, auristatins, vinca alkaloids, colchicines, and dolastatins. The drug targeting DNA may be, e.g., a drug that directly destroys the DNA structure or a topoisomerase inhibitor. Examples of drugs that directly destroy the structure of DNA include, but are not limited to, a DNA double strand breaker, a DNA alkylator, and a DNA intercalator. The DNA double strand breaker may be, e.g., an enediyne antibiotic, including but not limited to dynemicin, esperamicin, neocarzinostatin, uncialamycin, and the like. The DNA alkylator may be, e.g., a DNA bis-alkylator (i.e., DNA crosslinker) or a DNA mono-alkylator. Examples of DNA alkylators include, but are not limited to, a pyrrolo[2,1-c][1,4] benzodiazepine (PBD) dimer, a 1-(chloromethyl)-2,3-dihydro-1H-benzo[e]indole (CBI) dimer, a CBI-PBD heterodimer, an indolino-benzodiazepine (IGN) dimer, a duocarmycin-like compound, and the like. Examples of topoisomerase inhibitors include, but are not limited to, exatecan and derivatives thereof (e.g., DX8951f, DXd-(1), and DXd-(2), the structures of which are described below), camptothecins, and anthracyclines. The drug targeting RNA may be, e.g., a drug that inhibits splicing, and examples thereof include, but are not limited to, pladienolide. The drug targeting kinesin-mediated protein transport may be, e.g., mitotic kinesin inhibitors, including but not limited to kinesin spindle protein (KSP) inhibitors.

"Spacer" refers to a structure that is located between different structural modules and can spatially separate the structural modules. The definition of spacer does not limit whether it has a certain function or whether it can be cut off or degraded *in vivo.* Examples of spacers include, but are not limited to, amino acids and non-amino acid structures, wherein the non-amino acid structures may be, but are not limited to, amino acid derivatives or analogs. "Spacer sequence" refers to an amino acid sequence as a spacer, and examples thereof include, but are not limited to, single amino acids and sequences containing a plurality of amino acids, e.g., a sequence containing two amino acids, such as GA, or, e.g., GGGGS, GGGGSGGGGS, or GGGGSGGGGSGGGGS. The self-immolative spacer is a covalent assembly that enables the sequential cleavage of two chemical bonds after the activation of the protective moiety in the precursor: the protective moiety (e.g., cleavable sequence) is removed upon activation, triggering a cascade of decomposition reactions and thereby resulting in the sequential release of relatively small molecules. Examples of self-immolative spacers include, but are not limited to, PABC (p-benzyloxycarbonyl), acetal, heteroacetal, and combinations thereof.

The term "alkyl" refers to a linear or branched saturated aliphatic hydrocarbon group consisting of carbon atoms and hydrogen atoms, which is linked to the rest of the molecule by a single bond. Alkyl may have 1-20 carbon atoms and is referred to as "$C_1$-$C_{20}$ alkyl", such as $C_1$-$C_4$ alkyl, $C_1$-$C_3$ alkyl, $C_1$-$C_2$ alkyl, $C_3$ alkyl, $C_4$ alkyl, or $C_3$-$C_6$ alkyl. Non-limiting examples of alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, or isomers thereof. A divalent radical refers to a group obtained by removing one hydrogen atom from a carbon atom having a free valence electron of a corresponding monovalent radical. Divalent radicals have two linking sites for linking to the rest of the molecule. For example, "alkylene" or "alkylidene" refers to a saturated linear or branched divalent hydrocarbon group.

Examples of "alkylene" include, but are not limited to, methylene ($-CH_2-$), ethylene ($-C_2H_4-$), propylene ($-C_3H_6-$), butylene ($-C_4H_8-$), pentylene ($-C_3H_{10}-$), hexylene ($-C_6H_{12}-$), 1-methylethylene ($-CH(CH_3)CH_2-$), 2-methylethylene ($-CH_2CH(CH_3)-$), methylpropylene, ethylpropylene, or the like.

As used herein, when a group is combined with another group, the linkage between the groups may be linear or branched, provided that a chemically stable structure is formed. The structure formed by such a combination may be linked to the rest of the molecule through any suitable atom in the structure, preferably through a designated chemical bond. For example, when two or more divalent groups selected from $-CR^1R^2-$, $C_{1-10}$ alkylene, $C_{4-10}$ cycloalkylene, $C_{4-10}$ heterocyclylene, and -(CO)- are combined together to form a combination, the two or more divalent groups may form a linear linkage with each other, such as $-CR^1R^2$-$C_{1-10}$ alkylene-(CO)-, $-CR^1R^2$-$C_{4-10}$ cycloalkylene-(CO)-, $-CR^1R^2$-$C_{4-10}$ cycloalkylene-$C_{1-10}$ alkylene-(CO)-, $-CR^1R^2-CR^1R^{2'}$-(CO)-, or $-CR^1R^2-CR^{1'}R^{2'}-CR^{1''}R^{2''}$-(CO)-. The resulting divalent structure may be further linked to other parts of the molecule.

As used herein, the expressions "antibody-conjugated drug" and "antibody-drug conjugate" have the same meaning.

Compound of Formula (I)

In a first aspect, the present disclosure provides a compound of formula (I):

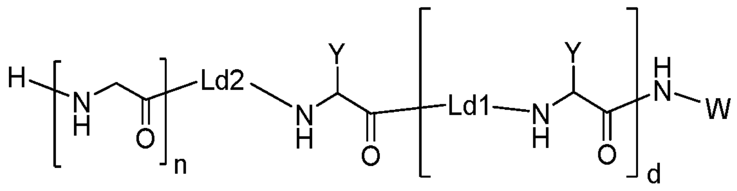

wherein,

W is hydrogen or LKb;

Y is hydrogen or LKa-LKb;

provided that W and Y are not both hydrogen;

each LKa is independently selected from

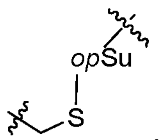 , 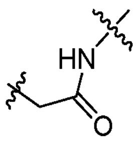 , and 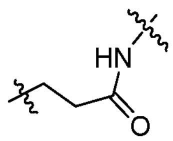 ;

*opSu* is

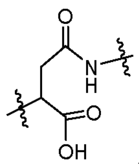 , 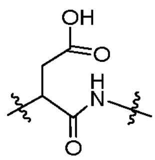 ,

or a mixture thereof;

each LKb is independently selected from $L^2$-$L^1$-B;

each B is independently a terminal group $R^{10}$ or a combination of 1), 2), and 3) below: 1) a self-immolative spacer Sp1; 2) a bond, or one divalent group, or a combination of two or more divalent groups, wherein the divalent group is selected from: $-CR^1R^2-$, $C_{1-10}$ alkylene, $C_{4-10}$ cycloalkylene, $C_{4-10}$ heterocyclylene, and -(CO)-; and 3) a terminal group $R^{10}$;

$R^{10}$ is hydrogen or a group that can leave when reacting with a group in the payload;

$L^1$ is a cleavable sequence 1 comprising an amino acid sequence capable of being cleaved by an enzyme, and the cleavable sequence 1 comprises 1-10 amino acids;

$L^2$ is a bond or $C_{2-20}$ alkylene, wherein one or more $-CH_2-$ structures in the alkylene are optionally replaced by the following groups: $-CR^3R^4-$, -O-, -(CO)-, $-S(=O)_2-$, $-NR^5-$, $-N^{\oplus}R^6R^7-$, $C_{4-10}$ cycloalkylene, $C_{4-10}$ heterocyclylene, and phenylene, wherein the cycloalkylene, heterocyclylene, and phenylene are each independently unsubstituted or substituted with at least one substituent selected from halogen, $-C_{1-10}$ alkyl, $-C_{1-10}$ haloalkyl, $-C_{1-10}$ alkylene-NH-$R^8$, and $-C_{1-10}$ alkylene-O-$R^9$;

Ld2 and each Ld1 are independently a bond, or are selected from -NH-$C_{1-20}$ alkylene-(CO)- and NH-$(PEG)_i$-(CO)-; or are natural amino acids or oligomeric natural amino acids with a degree of polymerization of 2-10 each independently unsubstituted or substituted with $-(PEG)_j-R^{11}$ on the side chain; or are natural amino acids or oligomeric natural amino acids with a degree of polymerization of 2-10 substituted with $-(PEG)_j-R^{11}$ on the side chain, wherein the $-(PEG)_j-R^{11}$ has carbonyl linked therebetween;

the $-(PEG)_i-$ and $-(PEG)_j-$ are each a PEG fragment comprising a specified number of consecutive $-(O-C_2H_4)-$

structural units or consecutive -($C_2H_4$-O)- structural units, optionally with additional $C_{1-10}$ alkylene at one terminal;

$R^1$, $R^2$, $R^3$, $R^4$, $R^5$, $R^6$, $R^7$, $R^8$, and $R^9$ are each independently selected from hydrogen, halogen, -$C_{1-10}$ alkyl, -$C_{1-10}$ haloalkyl, and $C_{4-10}$ cycloalkylene;

$R^{11}$ is $C_{1-10}$ alkyl;

n is any integer from 2 to 20;

d is 0 or any integer from 1 to 6;

each i is independently an integer from 1 to 100, preferably from 1 to 20; preferably, each i is independently an integer from 1 to 12, more preferably from 2 to 8, particularly 4;

each j is independently an integer from 1 to 100, preferably from 1 to 20; preferably, each j is independently an integer from 1 to 12, more preferably from 8 to 12 or 13, particularly 8, 9, 12, or 13.

In an embodiment $L^2$ is selected from: -$(CH_2)_p$-$(CH_2)_2$(CO)-, wherein p is 0 or an integer from 1 to 5;

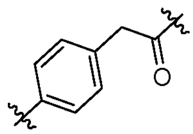 ; 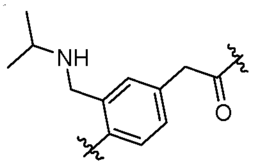 ; 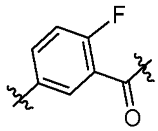 ; 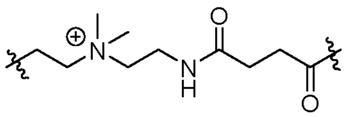 ;

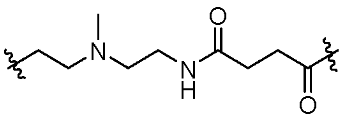 ; 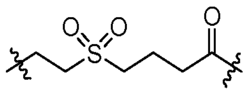 ; 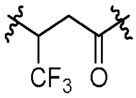 ; 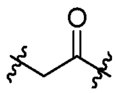 ; 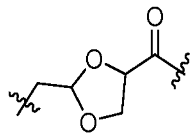 ;

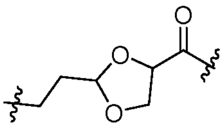 ; 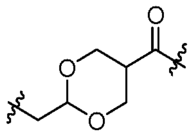 ; 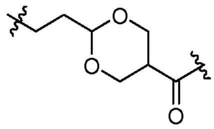 ; and 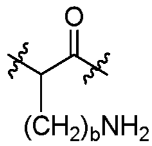 ,

wherein b is an integer from 1 to 10.

In an embodiment, the carbonyl in the above $L^2$ structure is linked to $L^1$, and the other linking site is linked to *opSu.*

In an embodiment, p is 0-3; preferably 3.

Ld2 and each Ld1 are independently selected from a bond,

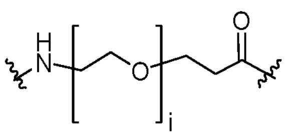 , 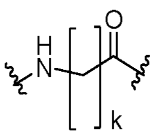 , 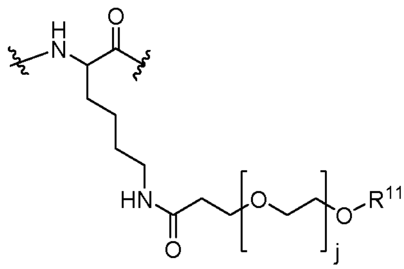 ,

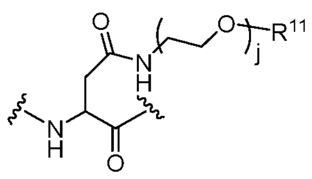
, or
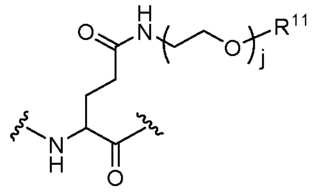
;

each i, j, and k is independently selected from integers from 1 to 100.

In an embodiment, each i, j, and k is independently selected from integers from 1 to 20. In an embodiment, each i, j, and k is independently selected from integers from 1 to 12.

In an embodiment, each i is independently selected from integers from 2 to 8, particularly 4.

In an embodiment, each j is independently selected from integers from 8 to 12 or 8 to 13, particularly 8, 9, 12, or 13.

In an embodiment, each k is independently selected from integers from 1 to 7, particularly 1, 3, or 5.

In an embodiment, Ld2 and each Ld1 are independently selected from a bond, or $C_{1-20}$ alkylene with amino and carbonyl at two terminals, respectively, a PEG fragment of a certain length (denoted as - $(PEG)_i$-) with amino and carbonyl at two terminals, respectively, or one or more natural amino acids each independently unsubstituted or substituted with a PEG fragment of a certain length (denoted as - $(PEG)_j$-) on the side chain.

In an embodiment, the -$(PEG)_i$- comprises -$(O-C_2H_4)_i$- or -$(C_2H_4-O)_i$-, optionally with additional $C_{1-10}$ alkylene at one terminal; the -$(PEG)_j$- comprises -$(O-C_2H_4)_j$- or -$(C_2H_4-O)_j$-, optionally with additional $C_{1-10}$ alkylene at one terminal. In a very specific embodiment, the -$(PEG)_i$- comprises -$C_2H_4-(O-C_2H_4)_i$- or -$(C_2H_4O)_i-C_2H_4$-.

It should be understood that when two or more Ld1, B, $L^2$, or $L^1$ structures are present in the molecule, the structure of each Ld1, B, $L^2$, or $L^1$ is independently selected. When two or more $R^x$ (x is 1, 2, 3, 4, 5, 6, 7, 8, 9, etc.) are present in the molecule, each $R^x$ is independently selected. In some embodiments, "x" in the molecule is represented with or without an additional single apostrophe (') or apostrophes (e.g., ", ‴, or ⁗), such as R, $R^{1'}$, $R^{1''}$, $R^{1'''}$, $R^{2'}$, $R^{2''}$, or $R^{2'''}$, wherein each $R^x$, with or without an additional single apostrophe or apostrophes, is independently selected. Other $R^x$, such as $R^3$, $R^4$, $R^5$, $R^6$, $R^7$, $R^8$, and $R^9$, and "Ld1", "B", "$L^2$" and "$L^1$", should be understood in a similar way. In some embodiments, "i" in the molecule is represented with or without additional numbers, such as i1, i2, i3, or i4, wherein the numbers do not represent any order, but are merely used to distinguish "i". In addition, each "i", with or without additional numbers, is independently selected.

In an embodiment, the cleavable sequence 1 is selected from GLy-GLy-Phe-Gly, Phe-Lys, Val-Cit, Val-Lys, GLy-Phe-Leu-Gly, Ala-Leu-Ala-Leu, Ala-Ala-Ala, and combinations thereof; a preferred cleavable sequence 1 is GLy-GLy-Phe-Gly.

In an embodiment, W is hydrogen.

In an embodiment, $R^{11}$ is $C_{1-6}$ alkyl, preferably methyl.

In an embodiment, n is an integer from 2 to 5, particularly 3.

In an embodiment, d is 0 or any integer from 1 to 4, preferably 0, 1, 2, or 3.

Thiosuccinimide is unstable under physiological conditions and is prone to retro-Michael addition, leading to cleavage at the conjugation site. In addition, thiosuccinimide may also undergo the exchange of sulfhydryl with another sulfhydryl compound when another sulfhydryl compound is present in the system. Both reactions will lead to the shedding of the payload and thereby result in toxic side effects. In the present disclosure, when applied to a linker, the ring-opened succinimide structure no longer undergoes retro-Michael addition or sulfhydryl exchange, and thus the product is more stable. Reference may be made to WO2015165413A1 for the method for the ring-opening reaction.

Compounds comprising the ring-opened succinimide moiety may be purified by semi-preparative/preparative HPLC or other suitable separation means to result in high purity and defined ingredients, regardless of the efficiency of the succinimide ring-opening reaction.

Moiety Comprising Recognition Sequence for Ligase Receptor or Donor Substrate

In an embodiment, the $G_n$ moiety of the compound of formula (I) is a recognition sequence for ligase receptor substrate, which facilitates the enzymatic coupling of the compound of formula (I) to a targeting molecule under catalysis of the ligase. The targeting molecule is optionally modified and comprises a corresponding recognition sequence for ligase donor substrate.

In an embodiment, the ligase is a transpeptidase. In an embodiment, the ligase is selected from the group consisting of: a natural transpeptidase, a non-natural transpeptidase, variants thereof, and combinations thereof. The non-natural transpeptidase may be, but is not limited to, those obtained by engineering a natural transpeptidase. In a preferred embodiment, the ligase is selected from the group consisting of: a natural Sortase, a non-natural Sortase, and

combinations thereof. The natural Sortase includes Sortase A, Sortase B, Sortase C, Sortase D, Sortase *L. plantarum,* etc. (US20110321183A1). The type of ligase corresponds to the ligase recognition sequence and thus serves to achieve specific conjugation between different molecules or structural fragments.

In some embodiments, the ligase is a Sortase selected from Sortase A, Sortase B, Sortase C, Sortase D, and Sortase *L. plantarum.* In these embodiments, the recognition sequence for ligase receptor substrate is selected from oligoglycine, oligoalanine, and oligomeric glycine/alanine mixtures with a degree of polymerization of 3-10. In a particular embodiment, the recognition sequence for ligase receptor substrate is $G_n$, wherein G is glycine (Gly) and n is an integer from 2 to 10.

In another particular embodiment, the ligase is Sortase A from *Staphylococcus aureus.* Thus, the ligase recognition sequence may be the recognition sequence LPXTG, which is typical of this enzyme. In yet another particular embodiment, the recognition sequence for ligase donor substrate is LPXTGJ, and the recognition sequence for ligase receptor substrate is $G_n$, wherein X may be any natural or non-natural single amino acid; J is absent or is an amino acid fragment comprising 1-10 amino acids, and may optionally be tagged. In an embodiment, J is absent. In yet another embodiment, J is an amino acid fragment comprising 1-10 amino acids, wherein each amino acid is independently any natural or non-natural amino acid. In another embodiment, J is $G_m$, where m is an integer from 1 to 10. In yet another particular embodiment, the recognition sequence for ligase donor substrate is LPETG. In another particular embodiment, the recognition sequence for ligase donor substrate is LPETGG.

In an embodiment, the ligase is Sortase B from *Staphylococcus aureus,* and the corresponding recognition sequence for donor substrate may be NPQTN. In another embodiment, the ligase is Sortase B from *Bacillus anthracis,* and the corresponding recognition sequence for donor substrate may be NPKTG.

In yet another embodiment, the ligase is Sortase A from *Streptococcus pyogenes,* and the corresponding recognition sequence for donor substrate may be LPXTGJ, wherein J is as defined above. In another embodiment, the ligase is Sortase subfamily 5 from *Streptomyces coelicolor,* and the corresponding recognition sequence for donor substrate may be LAXTG, wherein X may be any natural or non-natural single amino acid.

In yet another embodiment, the ligase is Sortase A from *Lactobacillus plantarum,* and the corresponding recognition sequence for donor substrate may be LPQTSEQ.

The ligase recognition sequence may also be other brand-new transpeptidase recognition sequences optimized by artificial screening.

<u>Moiety Comprising Reactive Group</u>

**Reactive groups for linking to payload**

In an embodiment, B is a terminal group $R^{10}$, and the cleavable sequence 1 in $L^1$ is linked to the payload. In this case, B is not present in the resulting molecule in which the cleavable sequence 1 is linked to the payload. In an embodiment, B is used to link to the payload. For linking to the payload, the compound of formula (I) comprises a reactive group. In an embodiment, B in the compound of formula (I) is linked to the payload via an amide bond, an ester bond, or an ether bond. In an embodiment, the reactive group in B of formula (I) is independently a reactive group for condensation reactions, nucleophilic addition, or electrophilic addition (e.g., reactive C=O moiety, reactive C=C-C=O moiety, amino, amine group, hydroxyl, or sulfhydryl), or a reactive group for substitution reactions (e.g., a leaving group linked to O, C, N, or S atom). In an embodiment, the reactive group in B is independently selected from carboxyl, active ester, formyl, amino, amine group, hydroxyl, and sulfhydryl. In a specific embodiment, the reactive group in B used for linking to the payload is independently selected from amino, amine group, hydroxyl, sulfhydryl, carboxyl, and active ester.

In an embodiment, the reactive group in B is independently amino, amine group, or hydroxyl, which reacts with the corresponding group in the payload (e.g., carboxyl, a sulfonic acid group, a phosphoryl group having a free-OH end, active ester, acyl chloride, or isocyanate). In another embodiment, the reactive group in B is independently carboxyl or active ester, which reacts with the corresponding group (e.g., amino, amine group, or hydroxyl) in the payload.

In an embodiment, the reactive group in B is independently amino, hydroxyl, or sulfhydryl, which reacts with the corresponding group (e.g., halogen, hydroxyl, or formyl) in the payload. In another embodiment, the reactive group in B is independently hydroxyl, which reacts with the corresponding group (e.g., halogen or hydroxyl) in the payload.

In an embodiment, each B is independently $R^{10}$ or a combination of 1), 2), and 3) below: 1) a self-immolative spacer Sp1; 2) a bond, or one divalent group, or a combination of two or more divalent groups, wherein the divalent group is selected from: $-CR^1R^2-$, $C_{1-10}$ alkylene, and -(CO)-; and 3) a terminal group $R^{10}$.

In an embodiment, Sp1 is selected from PABC, acetal, heteroacetal, and combinations thereof. In an embodiment, Sp1 is an acetal, heteroacetal, or PABC. In an embodiment, the heteroacetal is selected from N,O-heteroacetal. In an embodiment, Sp1 is $-O-CH_2-U-$ or $-NH-CH_2-U-$, wherein -O- or -NH- is linked to the cleavable sequence 1 and U is O, S, or NH, preferably O or S. In an embodiment, U is O, S, or N, preferably O or S.

In an embodiment, B is $R^{10}$, $-NH-CH_2-U-R^{10}$, or $-NH-CH_2-U-(CH_2)_g-(CO)-R^{10}$.

In an embodiment, $R^{10}$ is hydrogen, hydroxyl, or

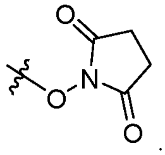

In an embodiment, $R^{10}$ is hydrogen. In an embodiment, $R^{10}$ is hydroxyl or

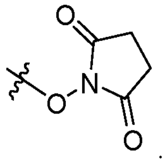

In an embodiment, $R^{10}$ represents a structural moiety that will not be present in the product molecule resulting from the reaction of B with the payload.

<u>Specific Embodiments of Compound of Formula (I)</u>

In an embodiment, W is hydrogen and each Lka is

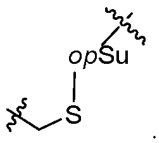

In an embodiment, the compound of formula (I) has the structure of formula (I-1):

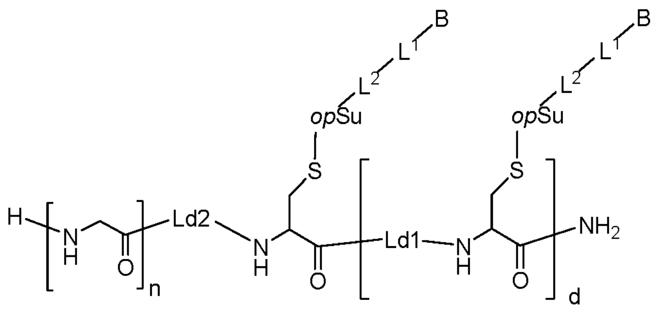

formula (I-1).

In an embodiment, Ld2 is a bond, and d is 0. In an embodiment, the compound of formula (I-1) is as follows:

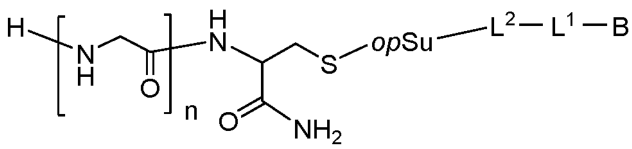

(linker LA301-1).

In an embodiment, d is 0, and Ld2 is

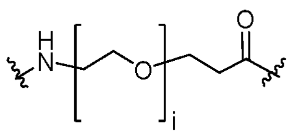

In an embodiment, the compound of formula (I-1) is as follows:

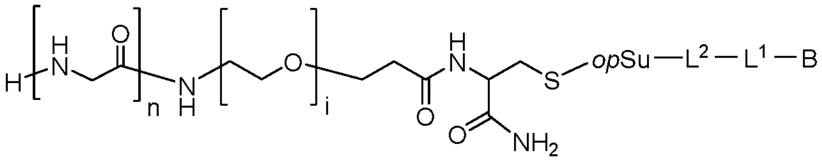

(linker LA301-2).

In an embodiment, d is 1, 2, or 3, and Ld2 and each Ld1 are independently selected from

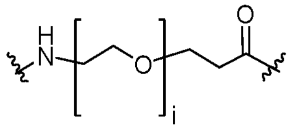

.

In an embodiment, the compound of formula (I-1) is as follows:

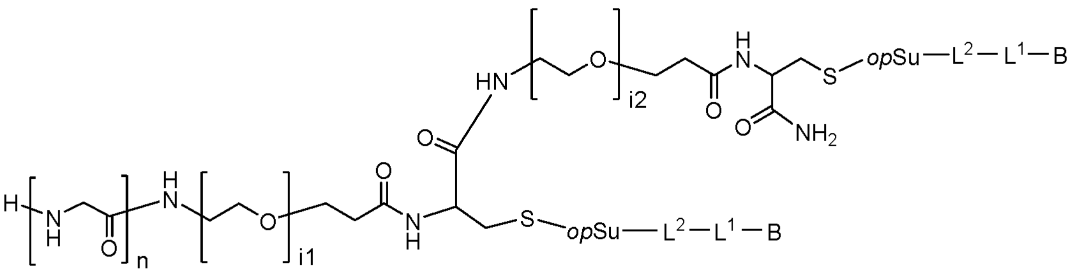

(linker LA301-3),

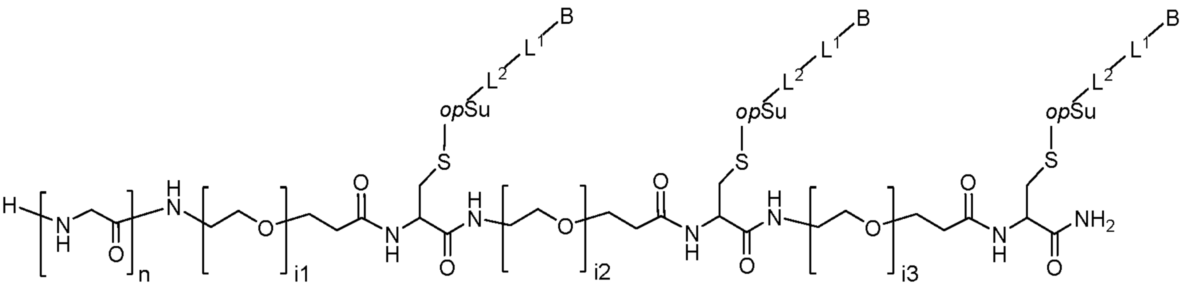

(linker LA301-4),

or

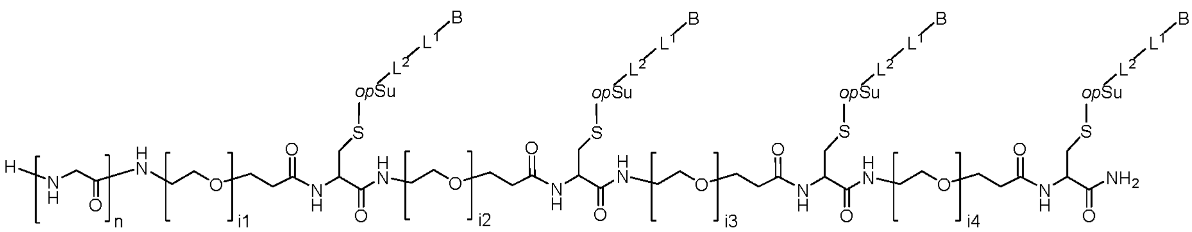

(linker LA301-5).

In an embodiment, Ld2 is

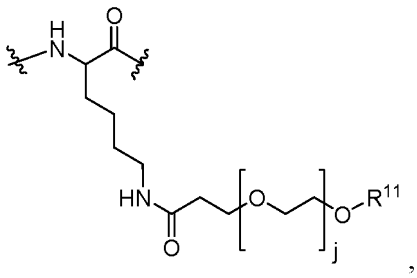

and d is 0. In an embodiment, the compound of formula (I-1) is as follows:

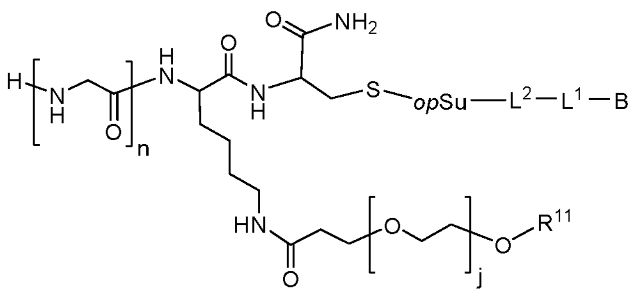

(linker LA302-1).

In an embodiment, d is 1, 2, or 3, Ld2 is

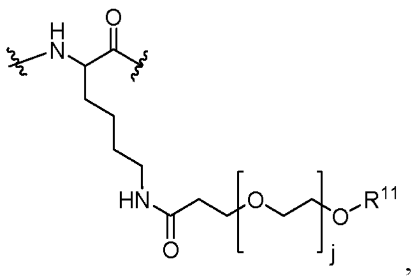

and each Ld1 is independently selected from

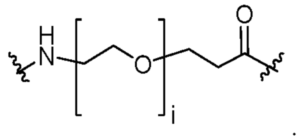

In an embodiment, the compound of formula (I-1) is as follows:

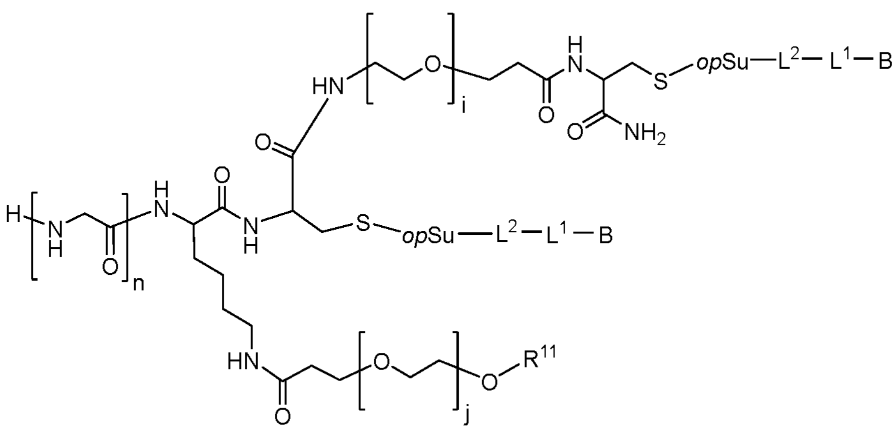

(linker LA302-2),

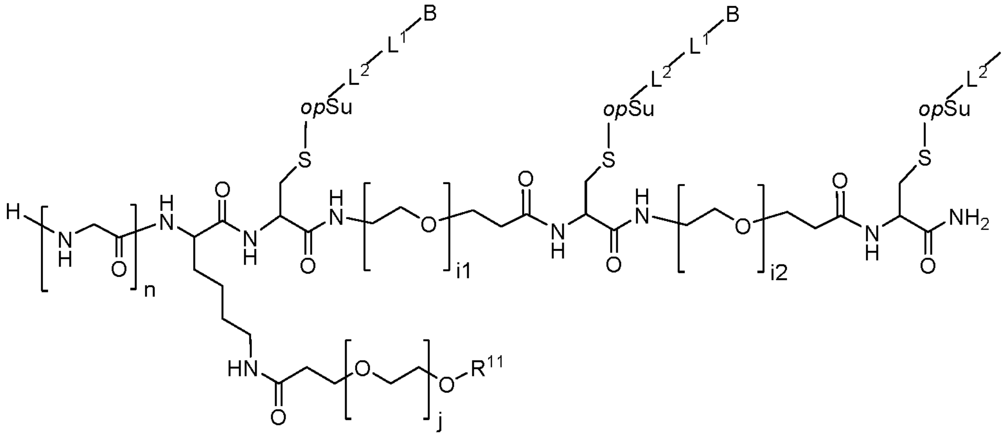

(linker LA302-3),

or

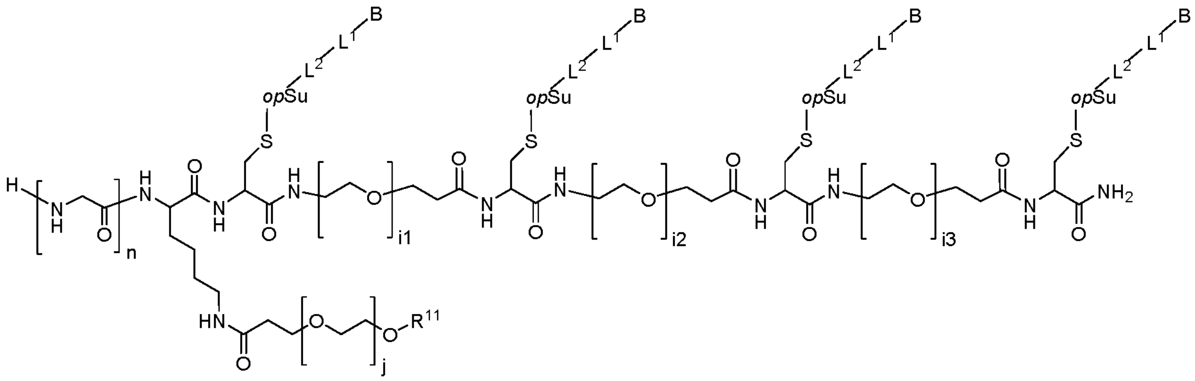

(linker LA302-4).

In an embodiment, n is 3, $L^2$ is $-(CH_2)_p-(CH_2)_2(CO)-$, p is 3, $L^1$ is GGFG, B is $-NH-CH_2-U-R^{10}$, $-R^{10}$, or $-NH-CH_2-U-(CH_2)_g-(CO)-R^{10}$, U is O, and g is 1. In an embodiment, the linker LA301-1 has the following structure:

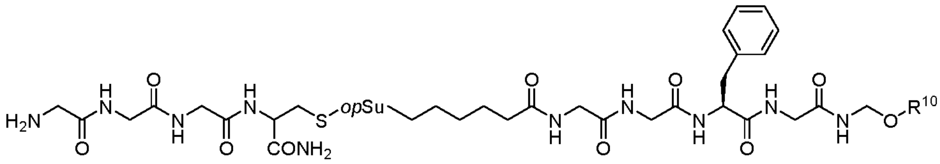

,

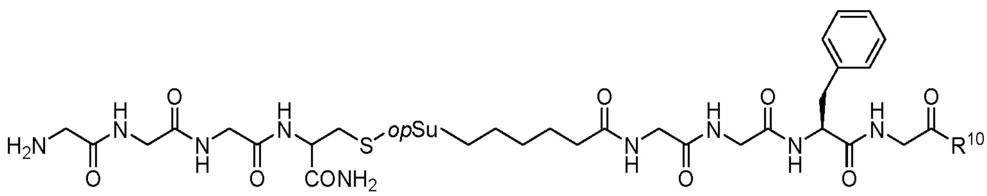

,

or 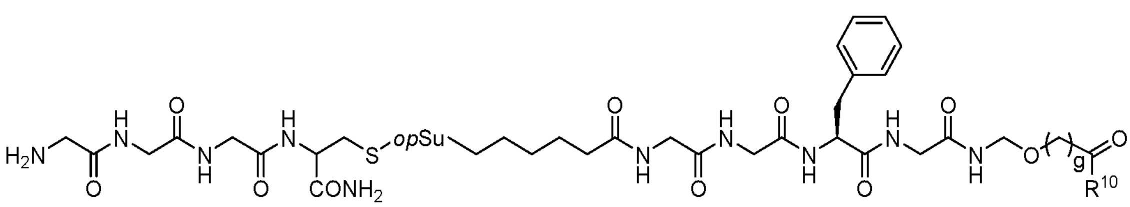 .

In an embodiment, the linker LA301-2 has the following structure:

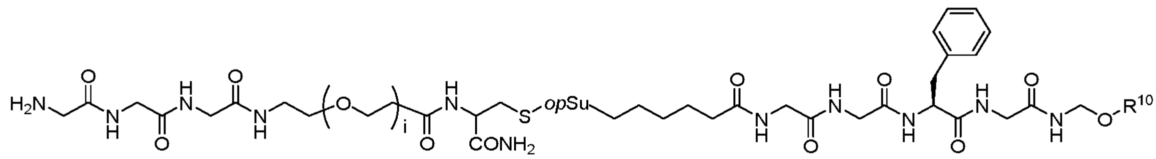

,

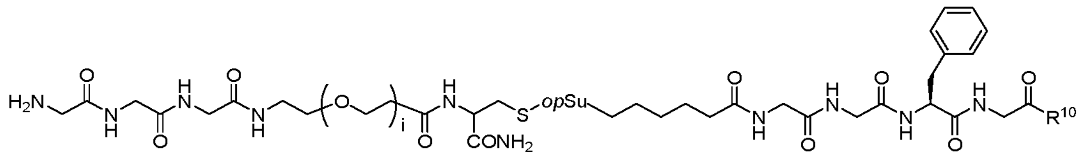

,

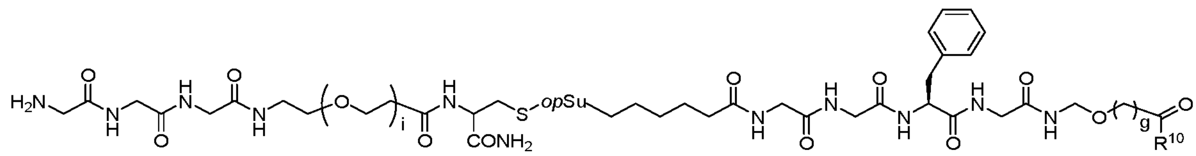

.

In an embodiment, the linker LA301-3 has the following structure:

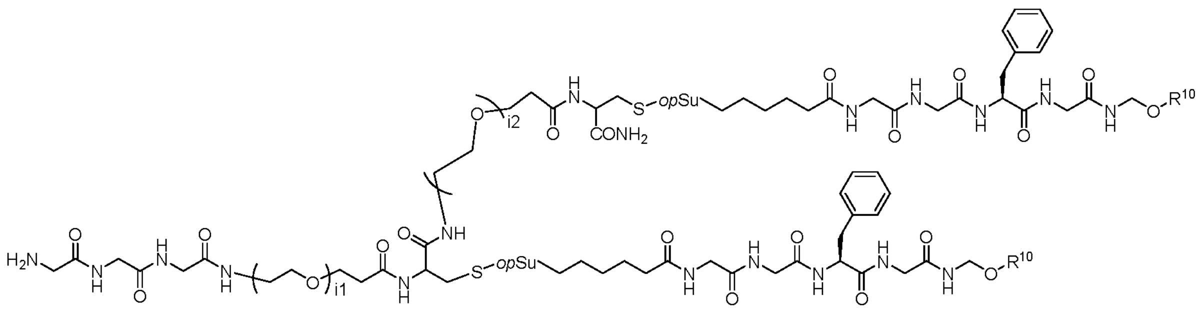

,

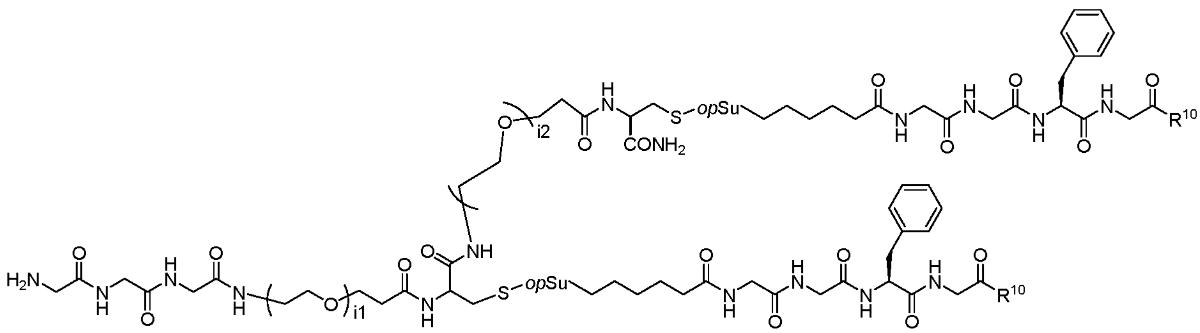

,

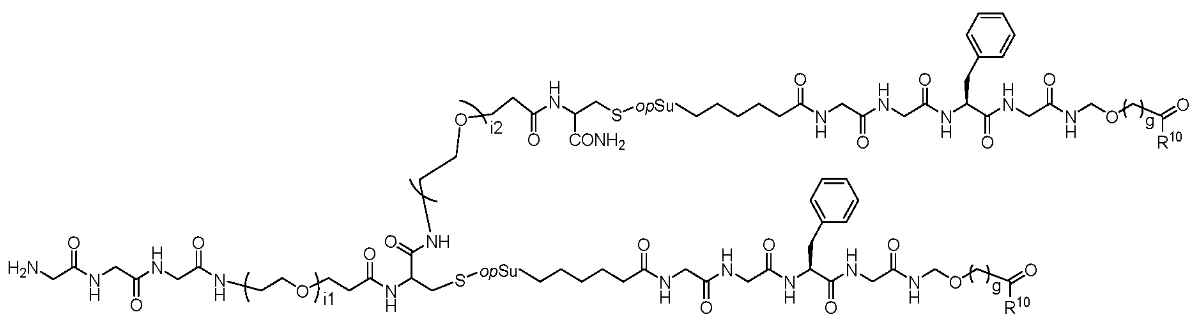

.

In an embodiment, the linker LA301-4 has the following structure:

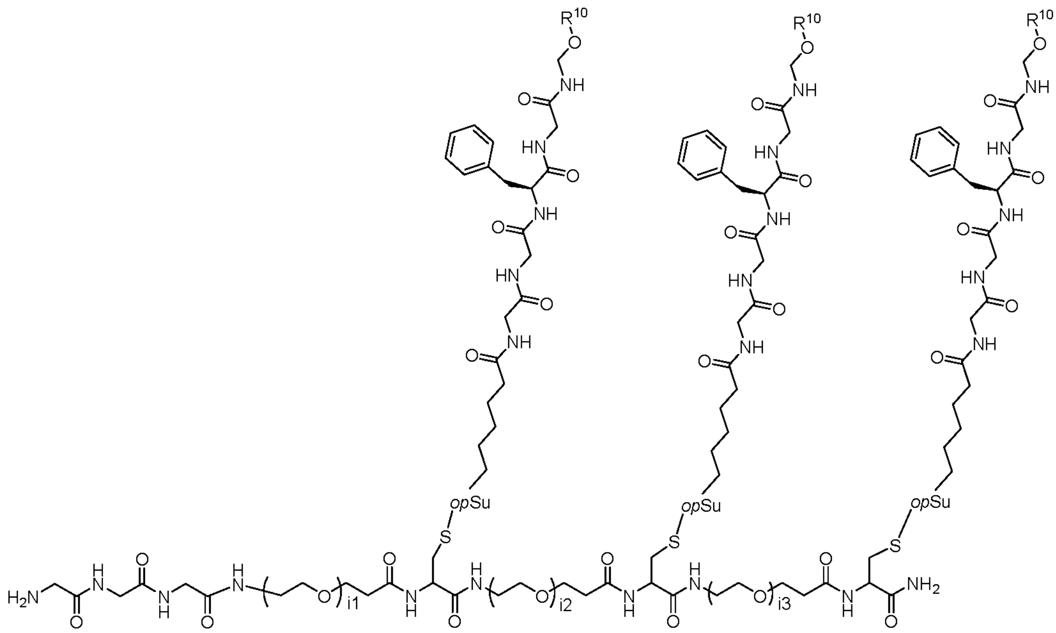

,

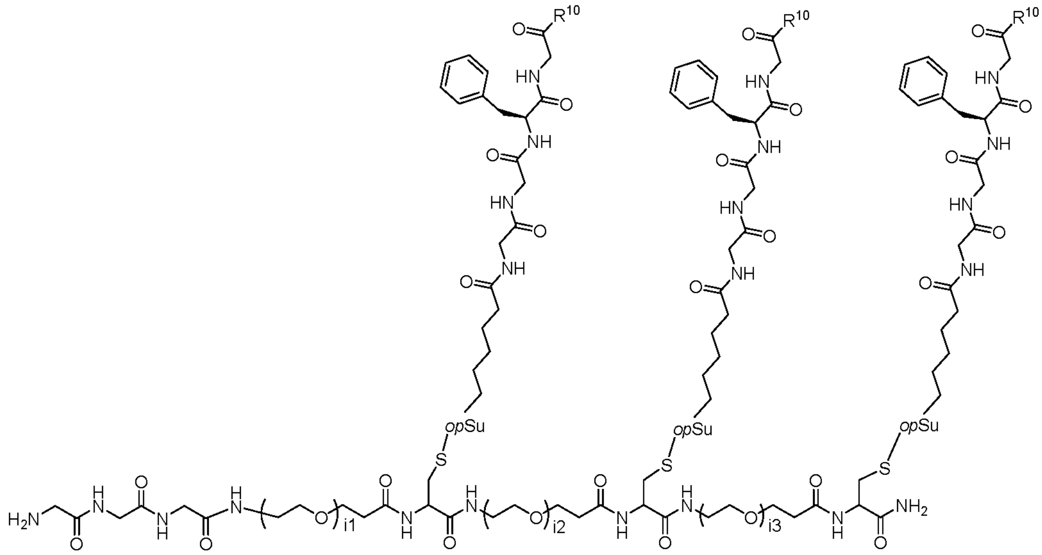

,

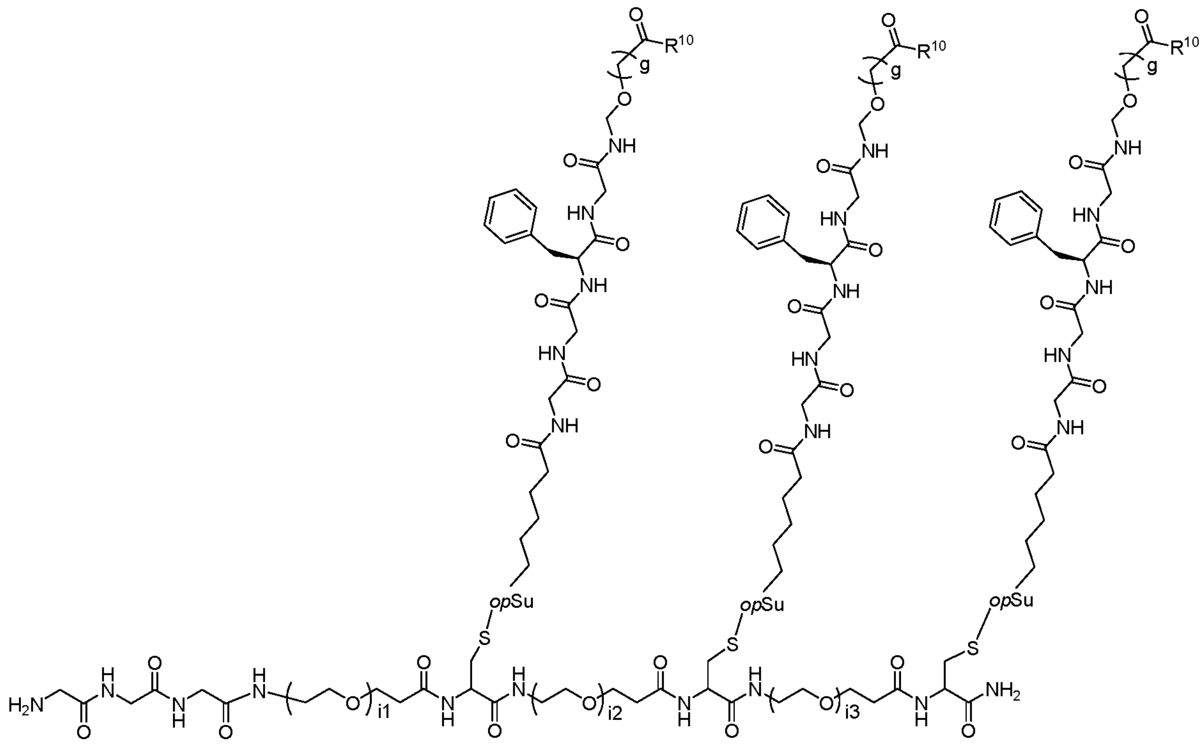

.

In an embodiment, the linker LA301-5 has the following structure:

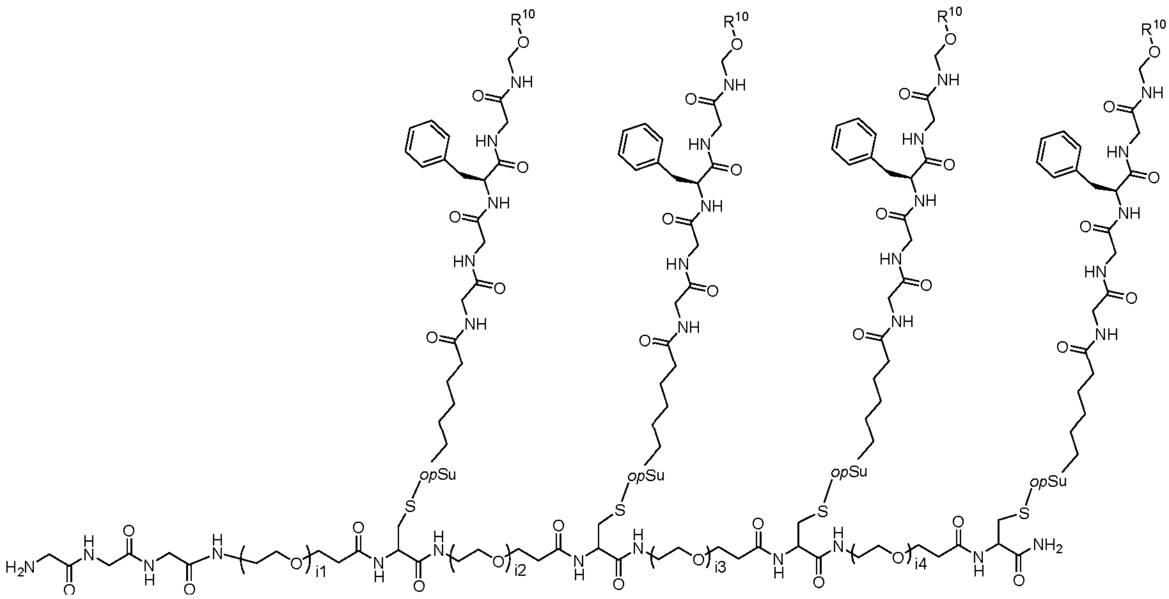

,

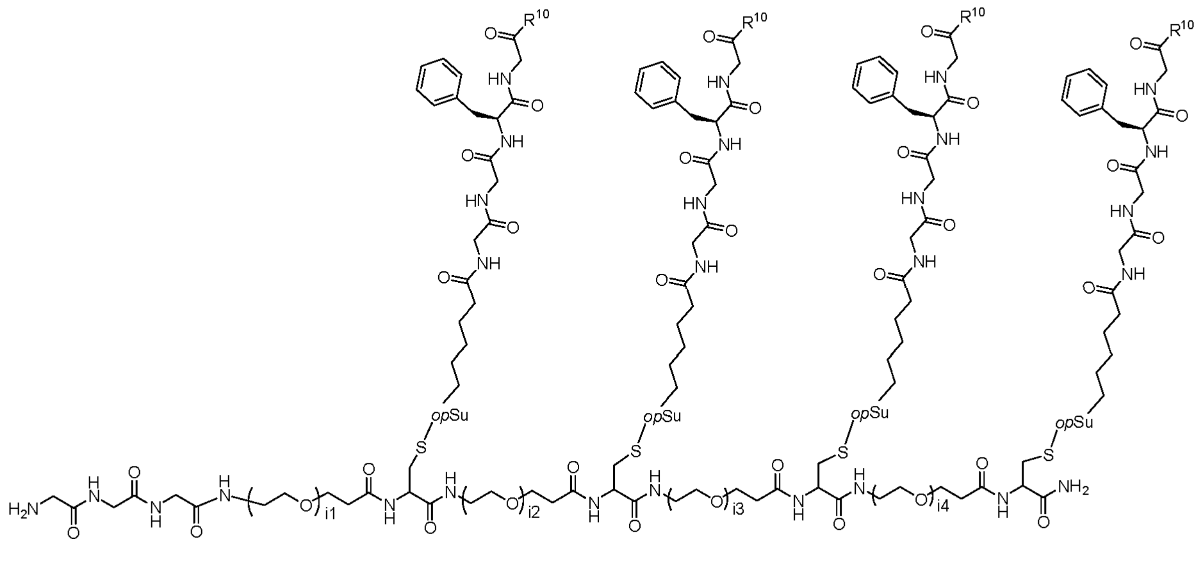

,

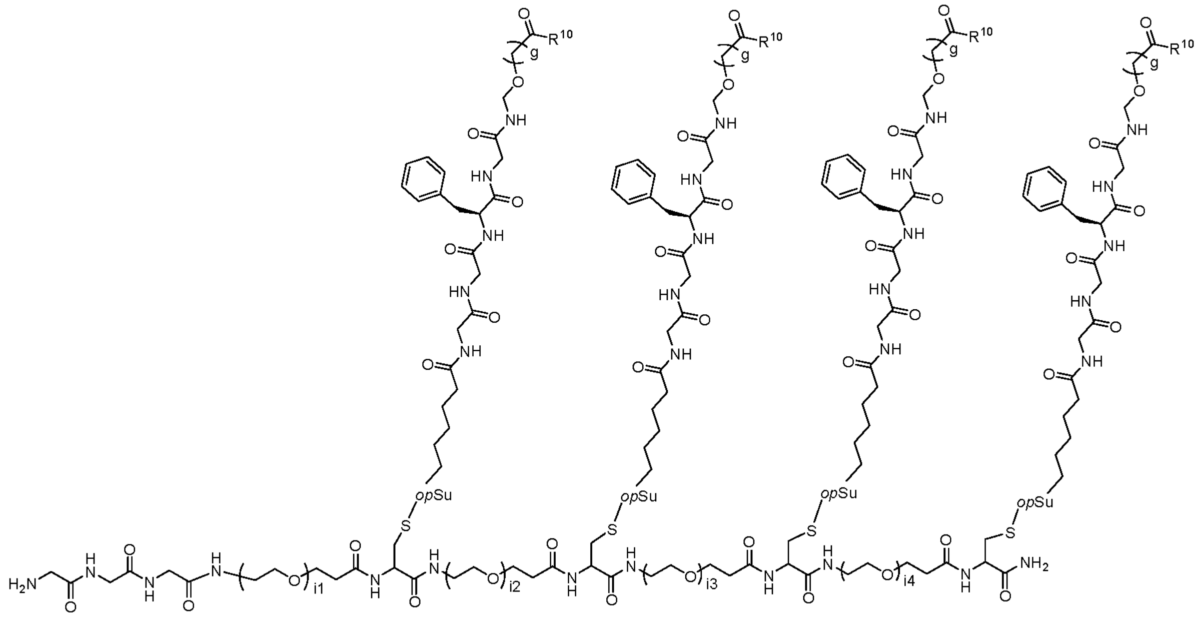

.

In an embodiment, the linker LA302-1 has the following structure:

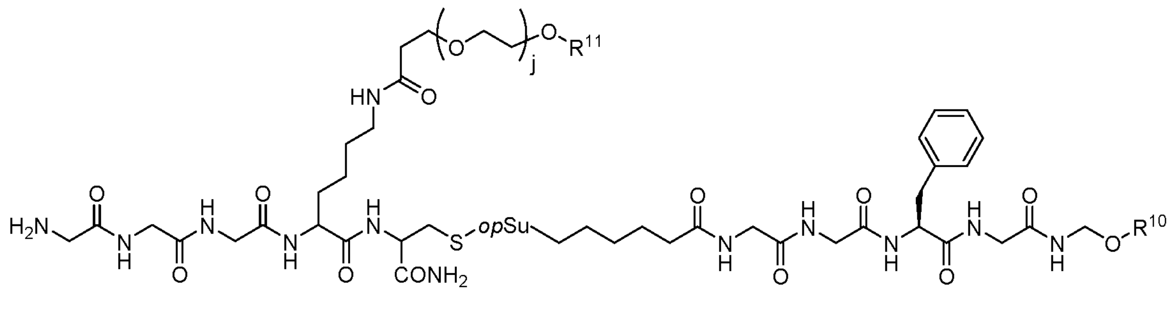

,

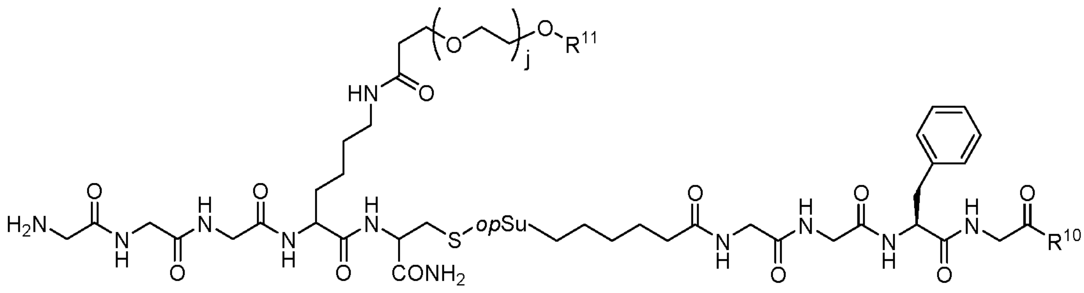

,

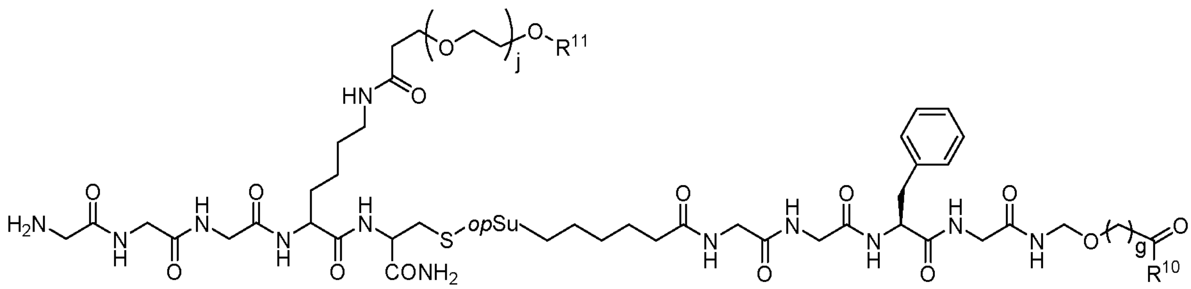

.

In an embodiment, the linker LA302-2 has the following structure:

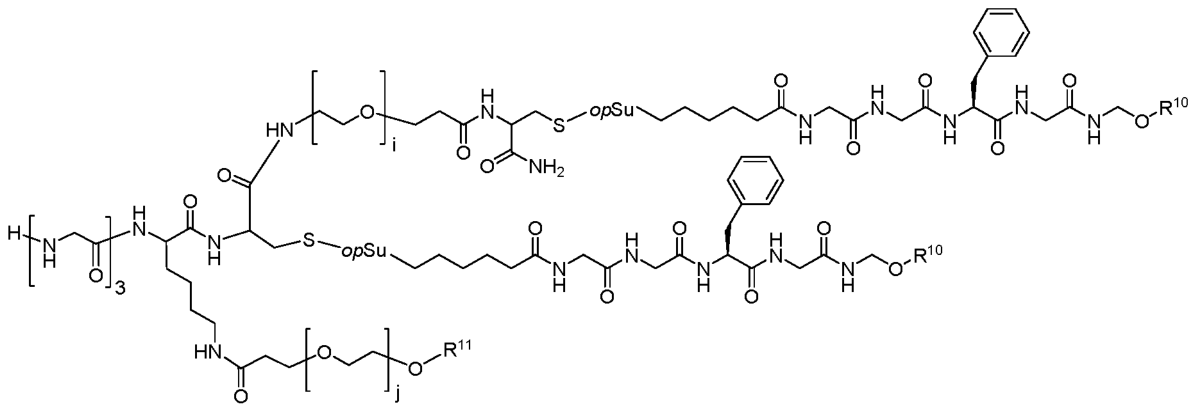

,

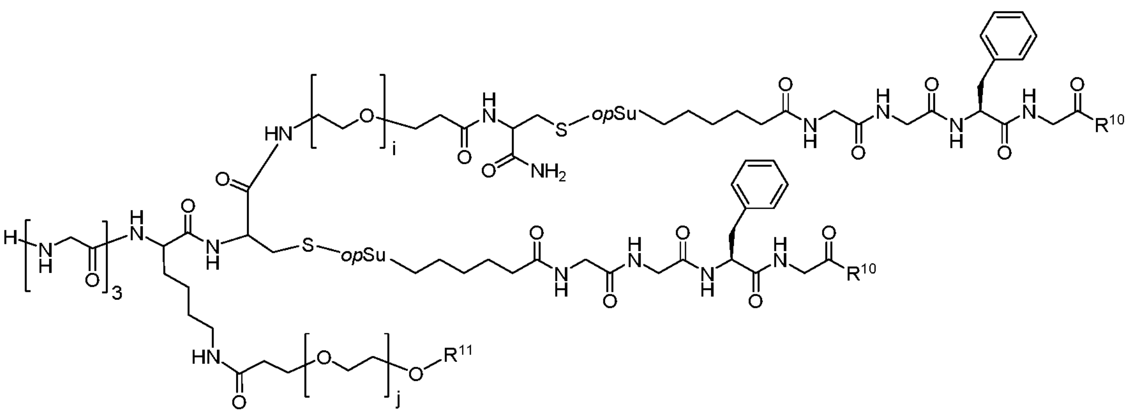

,

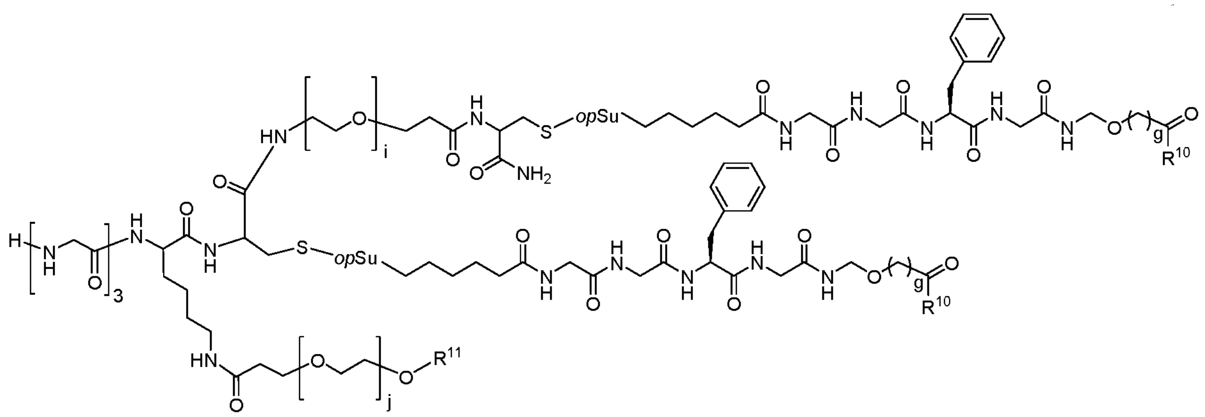

In an embodiment, the linker LA302-3 has the following structure:

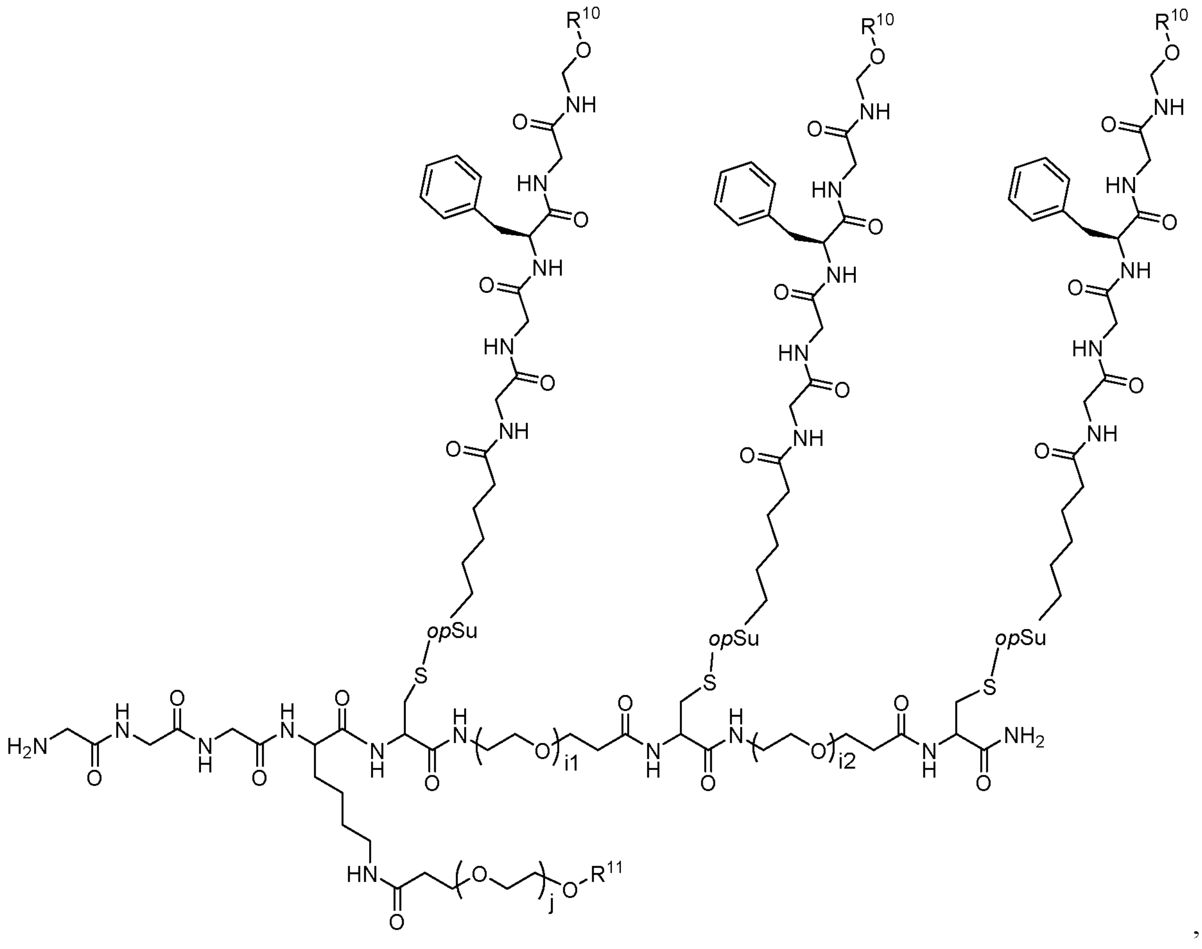

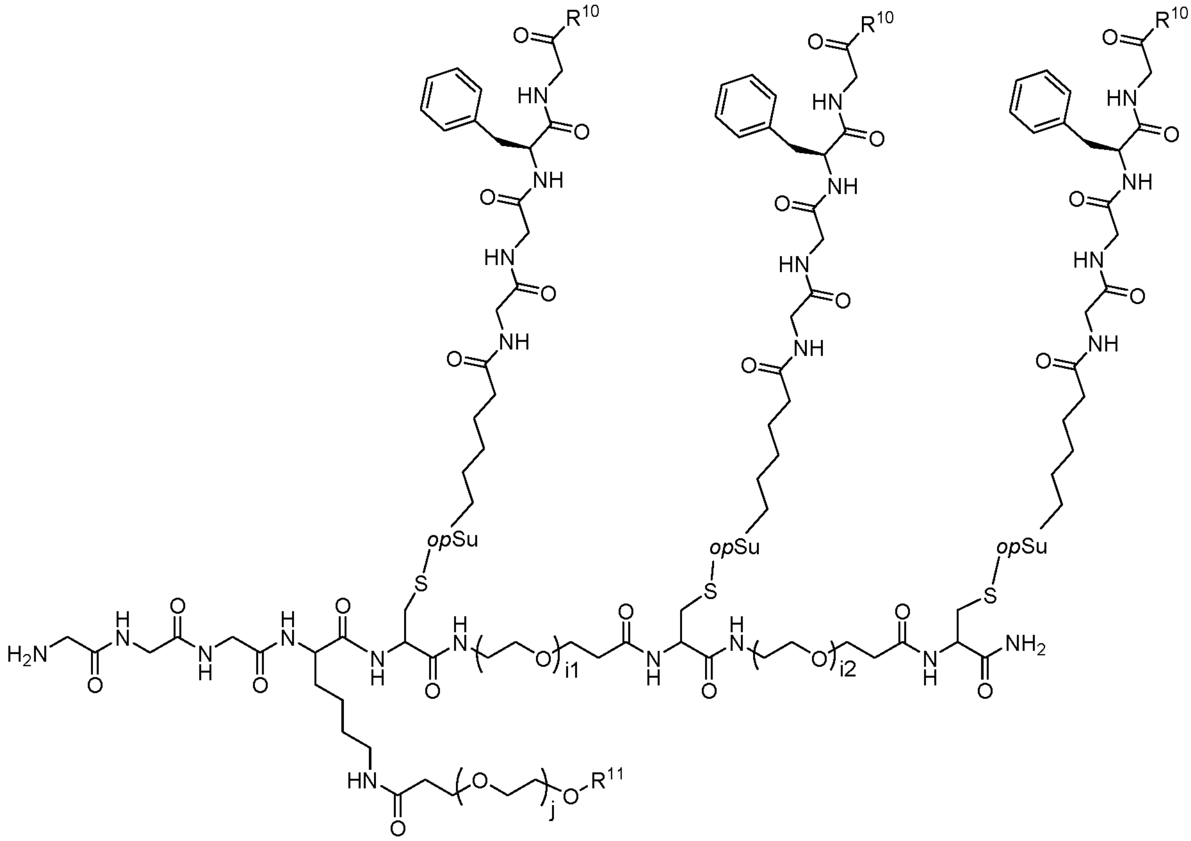

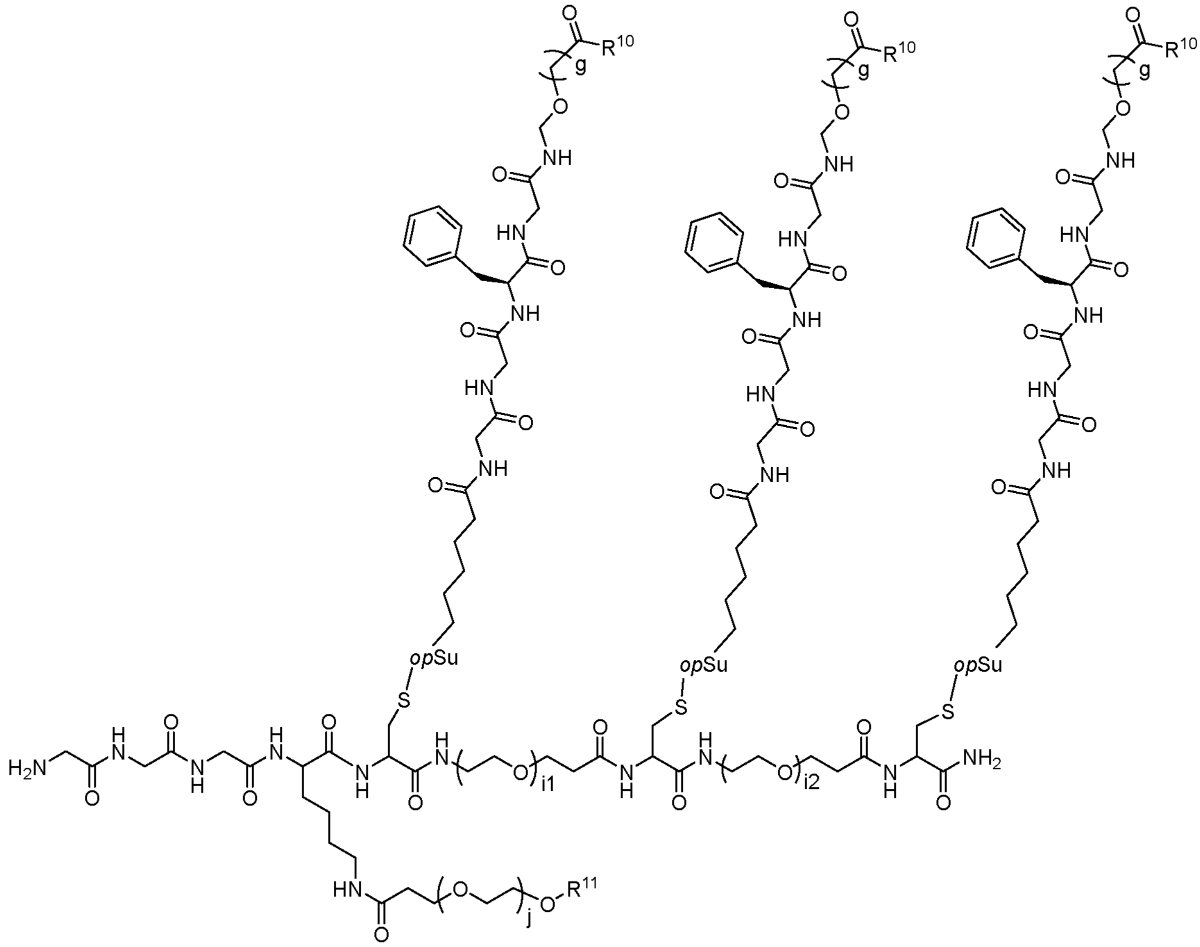

In an embodiment, the linker LA302-4 has the following structure:

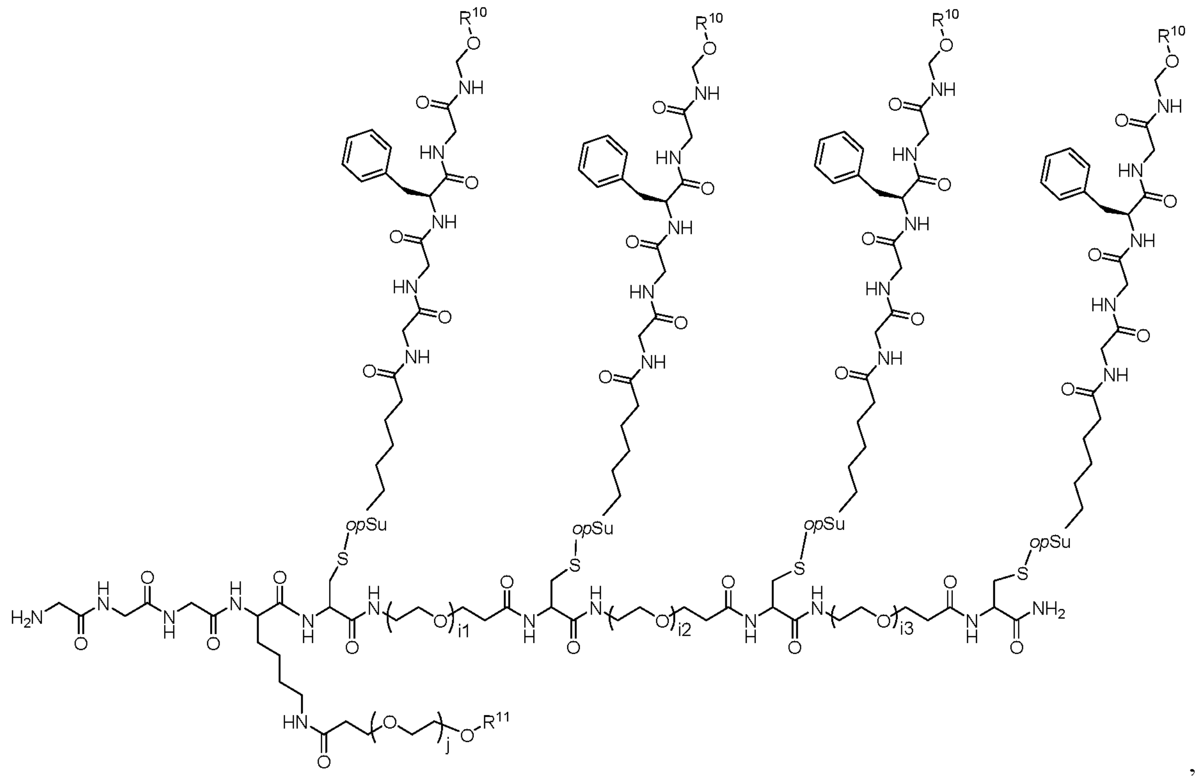

,

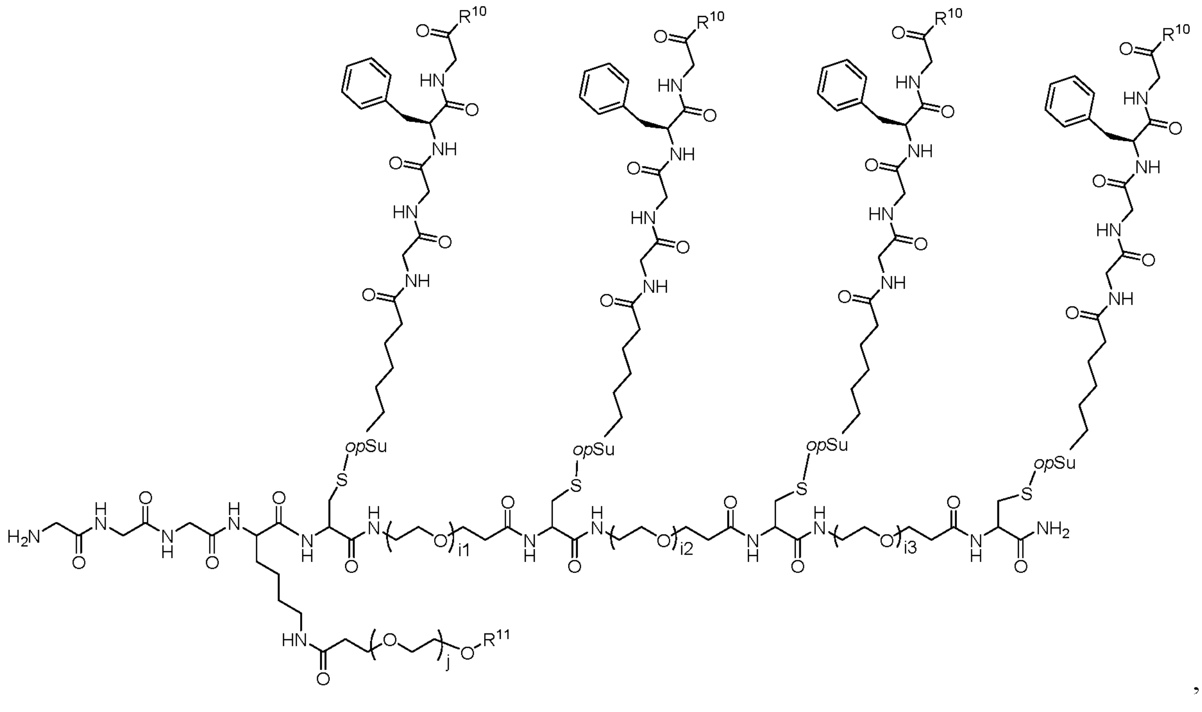

,

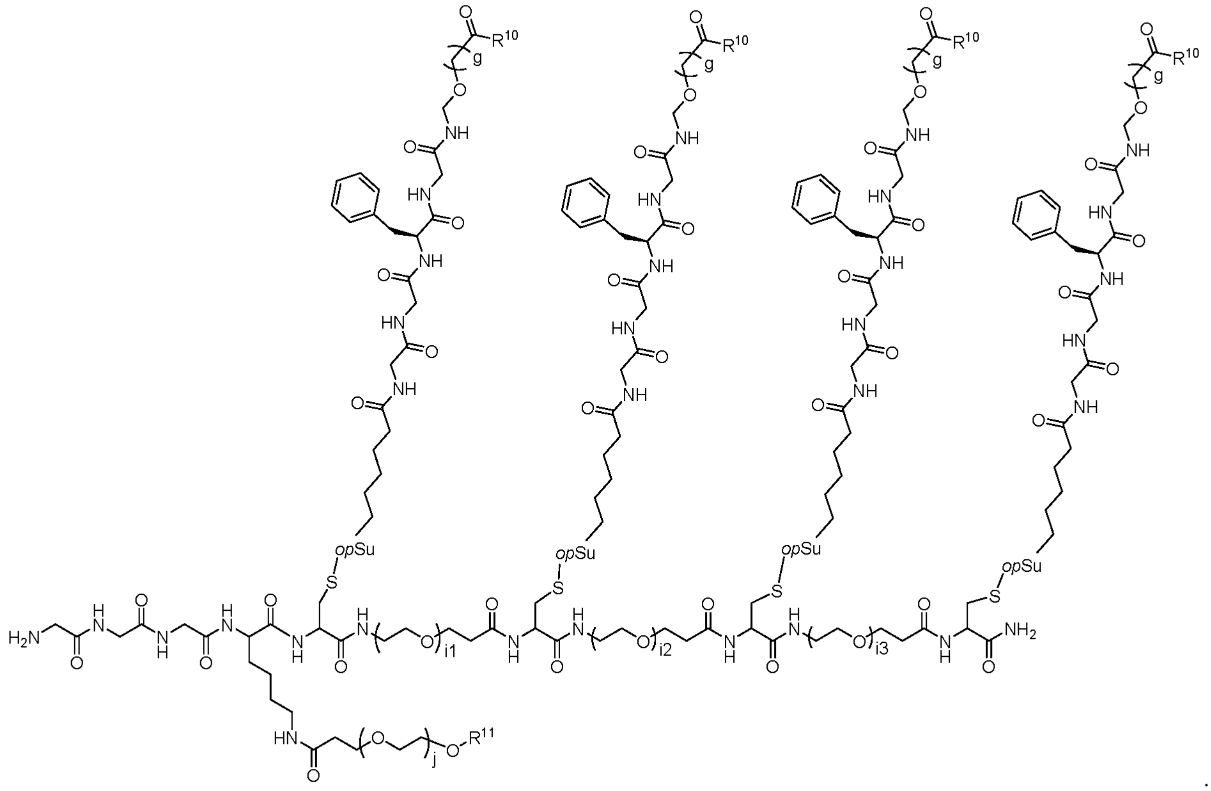

In an embodiment, i is 4, g is 1, and $R^{11}$ is methyl.

In an embodiment, the linker LA301-2 is as follows (linkers LA301-2-1 to LA301-2-3):

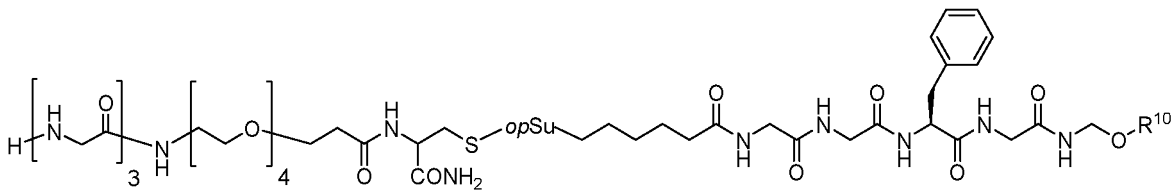

(linker LA301-2-1),

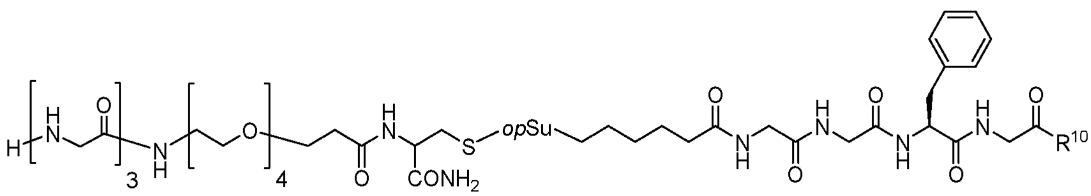

(linker LA301-2-2),

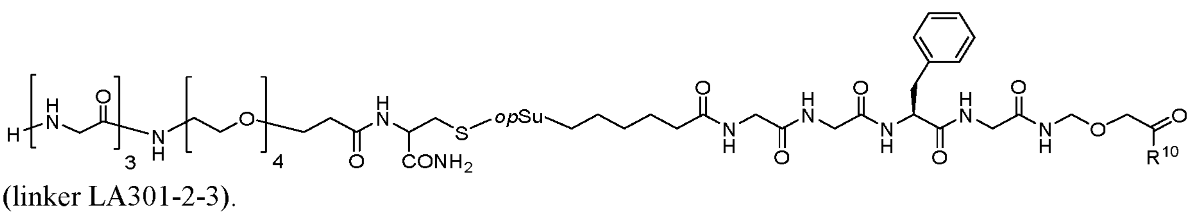

(linker LA301-2-3).

In an embodiment, i is 4, j is 8, g is 1, and $R^{11}$ is methyl. In an embodiment, the linker LA302-2 is as follows (linkers LA302-2-1 to LA302-2-3):

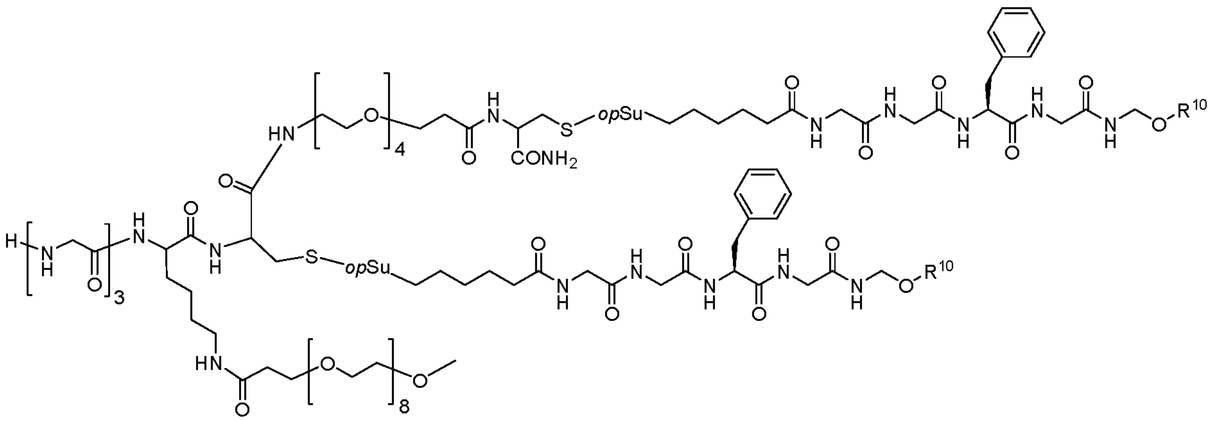

(linker LA302-2-1),

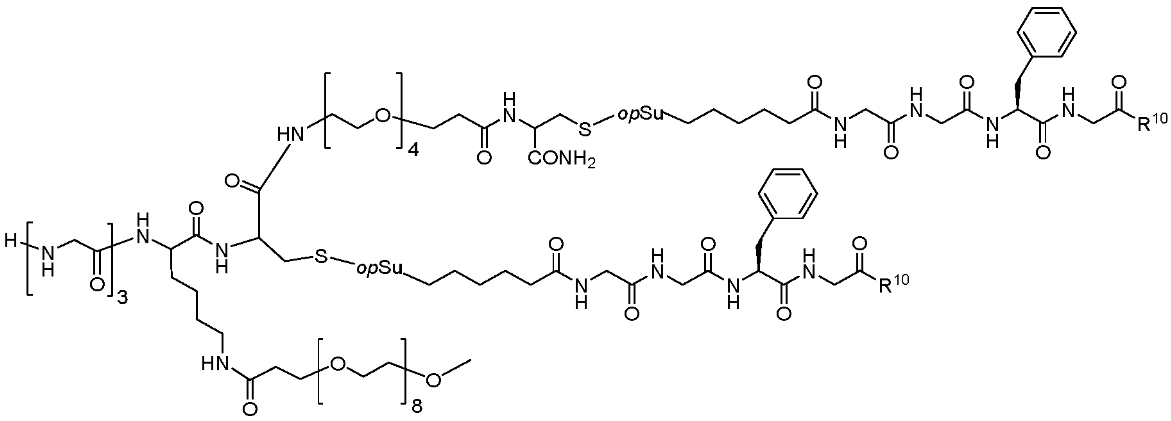

(linker LA302-2-2),

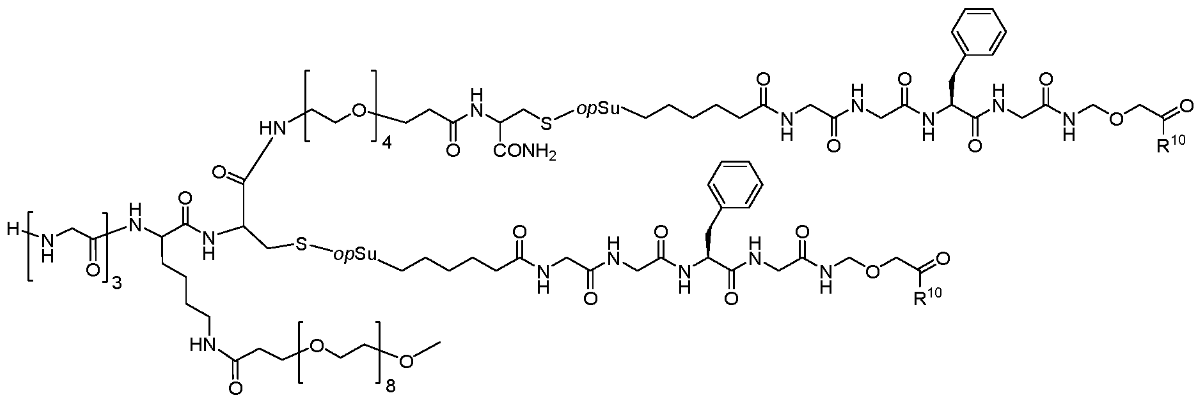

(linker LA302-2-3).

In an embodiment, i is 4, j is 12, g is 1, and $R^{11}$ is methyl. In an embodiment, the linker LA302-2 is as follows (linkers LA302-2-4 to LA302-2-6):

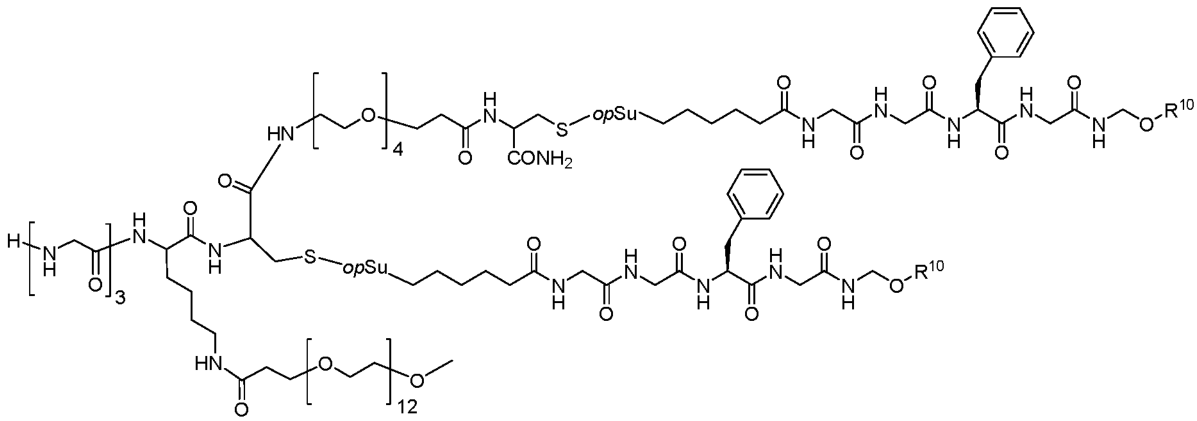

(linker LA302-2-4),

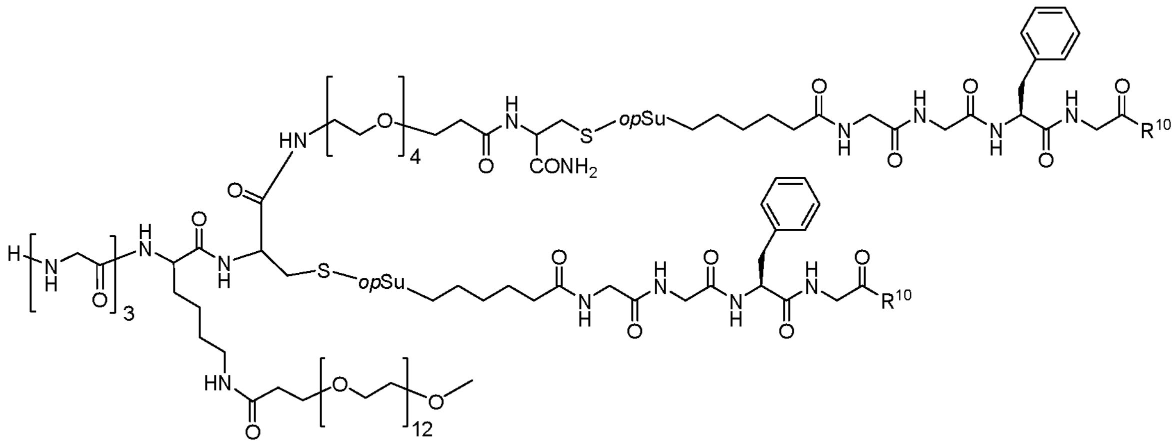

(linker LA302-2-5),

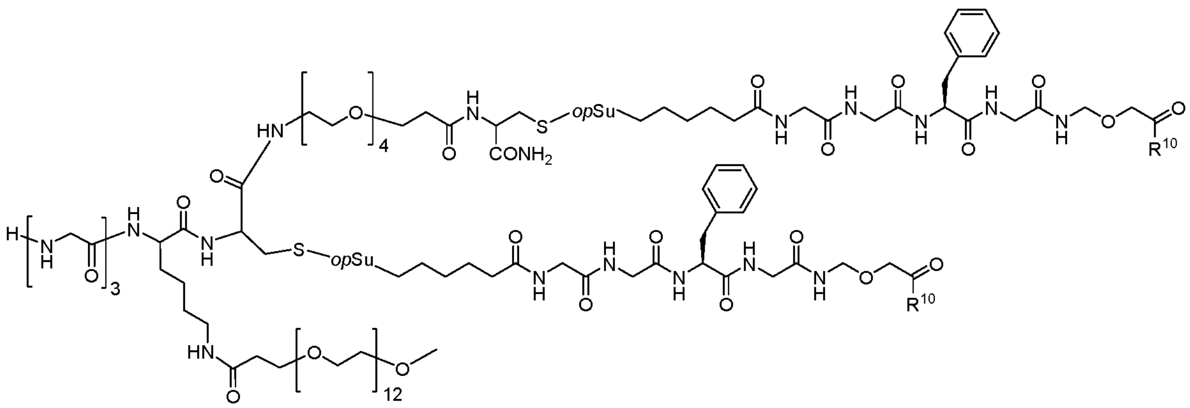

(linker LA302-2-6),

wherein *opSu* is

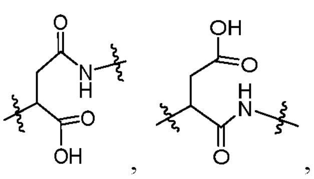

or a mixture thereof.

Payload-Bearing Compound of formula (I)

The reactive group contained in B is covalently conjugated with a payload containing another reactive group to obtain the payload-bearing compound of formula (I).

In yet another aspect, the present disclosure provides a compound having the structure of formula (II):

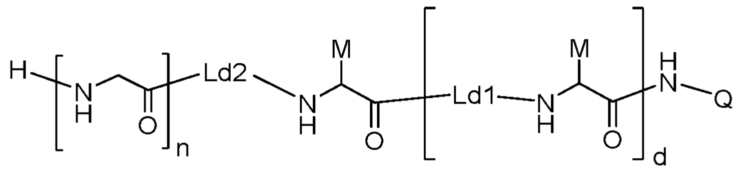

formula (II),

wherein

Q is hydrogen or LKb-P;

M is hydrogen or LKa-LKb-P;

provided that Q and M are not both hydrogen;

P is a payload linked to the B moiety or the $L^1$ moiety of the compound of formula (I);

n, d, Ld1, Ld2, LKa, and LKb are as defined in formula (I).

As defined above, in the compound of formula (I), each LKb is independently selected from $L^2$-$L^1$-B; each B is independently a terminal group $R^{10}$ or a combination of 1), 2), and 3) below: 1) a self-immolative spacer Sp1; 2) a bond, or one divalent group, or a combination of two or more divalent groups, wherein the divalent group is selected from: -$CR^1R^2$-, $C_{1-10}$ alkylene, $C_{4-10}$ cycloalkylene, $C_{4-10}$ heterocyclylene, and -(CO)-; and 3) a terminal group $R^{10}$; $R^{10}$ is hydrogen or a group that can leave when reacting with a group in the payload. In an embodiment, $R^{10}$ represents a structural moiety that will not be present in the product molecule resulting from the reaction of B with the payload.

In an embodiment, P is linked to the B moiety of the compound of formula (I) to form the compound of formula (II). As described above, $R^{10}$ will not be present in the B-P structure of the compound of formula (II).

It should be understood that when B in the compound of formula (I) is the terminal group $R^{10}$, $R^{10}$ will not be present in the compound of formula (II) and therefore B in the B-P structure of the compound of formula (II) will be correspondingly absent.

In an embodiment, M is hydrogen or LKa-$L^2$-$L^1$-B-P, wherein P is a payload linked to the B moiety or the $L^1$ moiety of the compound of formula (I); each B is independently a terminal group $R^{10}$ or a combination of 1), 2), and 3) below: 1) a self-immolative spacer Sp1; 2) a bond, or one divalent group, or a combination of two or more divalent groups, wherein the divalent group is selected from: - $CR^1R^2$-, $C_{1-10}$ alkylene, $C_{4-10}$ cycloalkylene, $C_{4-10}$ heterocyclylene, and -(CO)-; and 3) a terminal group $R^{10}$; $R^{10}$ is hydrogen or a group that can leave when reacting with a group in the payload; $R^{10}$ represents a structural moiety that will not be present in the product molecule resulting from the reaction of B with the payload.

In an embodiment, M is hydrogen or LKa-$L^2$-$L^1$-B-P, wherein each B is independently absent or is a combination of 1) and 2) below: 1) a self-immolative spacer Sp1; and 2) a bond, or one divalent group, or a combination of two or more divalent groups, wherein the divalent group is selected from: - $CR^1R^2$-, $C_{1-10}$ alkylene, and -(CO)-. In a preferred embodiment, M is hydrogen or LKa-$L^2$-$L^1$-B-P, wherein each B is independently absent or is -NH-$CH_2$-U- or -NH-$CH_2$-U-$(CH_2)_g$-(CO)-; g is any integer from 1 to 6, preferably 1. In another embodiment, M is LKa-$L^2$-$L^1$-B-P. In an embodiment, B in LKa-$L^2$-$L^1$-B-P is absent. In an embodiment, B in LKa-$L^2$-$L^1$-B-P is a combination of 1) and 2) below: 1) a self-immolative spacer Sp1; and 2) a bond, or one divalent group, or a combination of two or more divalent groups, wherein the divalent group is selected from: -$CR^1R^2$-, $C_{1-10}$ alkylene, and -(CO)-. In an embodiment, B in LKa-$L^2$-$L^1$-B-P is -NH-$CH_2$-U- or -NH-$CH_2$-U-$(CH_2)_g$-(CO)-; g is any integer from 1 to 6, preferably 1. U is O, S, or NH, preferably O or S. In an embodiment, B in the compound of formula (I) is linked to the payload via an amide bond, an ester bond, or an ether bond.

As described above, when B in the compound of formula (I) is the terminal group $R^{10}$, B in the B-P structure of the compound of formula (II) is correspondingly absent. In this case, it can also be understood as follows: the cleavable sequence 1 in $L^1$ is linked to the payload to form the compound of formula (II), wherein B is not present in the molecule resulting from the linking of the cleavable sequence 1 to the payload. Thus, in an embodiment, P is linked to the $L^1$ moiety of the compound of formula (I) to form the compound of formula (II). Thus, in an embodiment, M is LKa-$L^2$-$L^1$-B-P and B is absent, and M may also be denoted as LKa-$L^2$-$L^1$-P.

**Payload**

In the present disclosure, the payload may be selected from the group consisting of small molecule compounds, nucleic acids and nucleic acid analogs, tracer molecules (including fluorescent molecules, etc.), short peptides, polypeptides, peptidomimetics, and proteins. In an embodiment, the payload is selected from the group consisting of small molecule compounds, nucleic acid molecules, and tracer molecules. In a preferred embodiment, the payload is selected from small molecule compounds. In a more preferred embodiment, the payload is selected from the group consisting of cytotoxin and fragments thereof.

In an embodiment, the cytotoxin is selected from the group consisting of drugs targeting the microtubule cytoskeleton. In a preferred embodiment, the cytotoxin is selected from the group consisting of taxanes, maytansinoids, auristatins, epothilones, combretastatin A-4 phosphate, combretastatin A-4 and derivatives thereof, indole-sulfonamides, vinca alkaloids such as vinblastine, vincristine, vindesine, vinorelbine, vinflunine, vinglycinate and anhydrovinblastine, dolastatin 10 and analogs thereof, halichondrin B, eribulin, indole-3-oxoacetamides, podophyllotoxins, 7-diethylamino-3-(2'-benzoxazolyl)-coumarin (DBC), discodermolide, and laulimalide. In another embodiment, the cytotoxin is selected from the group consisting of DNA topoisomerase inhibitors such as camptothecins and derivatives thereof, mitoxantrone, and mitoguazone. In a preferred embodiment, the cytotoxin is selected from the group consisting of nitrogen mustards such as chlorambucil, chlomaphazine, cholophosphamide, estramustine, ifosfamide, mechlorethamine, mechlorethamine oxide hydrochloride, melphalan, novembichin, phenamet, phenesterine, prednimustine, trofosfamide, and uracil mustard. In yet another preferred embodiment, the cytotoxin is selected from the group consisting of nitrosoureas such as carmustine, flubenzuron, formoterol, lomustine, nimustine, and ranimustine. In an embodiment, the cytotoxin is selected from the group consisting of aziridines. In a preferred embodiment, the cytotoxin is selected from the group consisting of benzodopa, carboquone, meturedepa, and uredepa. In an embodiment, the cytotoxin is selected from the group consisting of anti-tumor antibiotics. In a preferred embodiment, the cytotoxin is selected from the group consisting of enediyne antibiotics. In a more preferred embodiment, the cytotoxin is selected from the group consisting of dynemicin, esperamicin, neocarzinostatin, and aclacinomycin. In another preferred embodiment, the cytotoxin is selected from the group consisting of actinomycin, anthramycin, bleomycins, actinomycin C, carabicin, carminomycin, cardinophyllin, carminomycin, actinomycin D, daunorubicin, detorubicin, adriamycin, epirubicin, esorubicin, idarubicin, marcellomycin, mitomycins, nogalamycin, olivomycin, peplomycin, porfiromycin, puromycin, ferric adriamycin, rodorubicin, rufocromomycin, streptozocin, zinostatin, and zorubicin. In yet another preferred embodiment, the cytotoxin is selected from the group consisting of trichothecenes. In a more preferred embodiment, the cytotoxin is selected from the group consisting of T-2 toxin, verracurin A, bacillocporin A, and anguidine. In an embodiment, the cytotoxin is selected from the group consisting of anti-tumor amino acid derivatives. In a preferred embodiment, the cytotoxin is selected from the group consisting of ubenimex, azaserine, and 6-diazo-5-oxo-L-norleucine. In another embodiment, the cytotoxin is selected from the group consisting of folic acid analogs. In a preferred embodiment, the cytotoxin is selected from the group consisting of denopterin, methotrexate, pteropterin, trimetrexate, and edatrexate. In an embodiment, the cytotoxin is selected from the group consisting of purine analogs. In a preferred embodiment, the cytotoxin is selected from the group consisting of fludarabine, 6-mercaptopurine, thiamiprine, and thioguanine. In yet another embodiment, the cytotoxin is selected from the group consisting of pyrimidine analogs. In a preferred embodiment, the cytotoxin is selected from the group consisting of ancitabine, gemcitabine, enocitabine, azacitidine, 6-azauridine, carmofur, cytarabine, dideoxyuridine, doxifluridine, and floxuridine. In an embodiment, the cytotoxin is selected from the group consisting of androgens. In a preferred embodiment, the cytotoxin is selected from the group consisting of calusterone, dromostanolone propionate, epitiostanol, mepitiostane, and testolactone. In another embodiment, the cytotoxin is selected from the group consisting of anti-adrenals. In a preferred embodiment, the cytotoxin is selected from the group consisting of aminoglutethimide, mitotane, and trilostane. In an embodiment, the cytotoxin is selected from the group consisting of anti-androgens. In a preferred embodiment, the cytotoxin is selected from the group consisting of flutamide, nilutamide, bicalutamide, leuprorelin acetate, and goserelin. In yet another embodiment, the cytotoxin is selected from the group consisting of protein kinase inhibitors and proteasome inhibitors. In another embodiment, the cytotoxin is selected from the group consisting of vinca alkaloids, colchicines, taxanes, auristatins, maytansinoids, calicheamicin, doxorubicin, duocarmycin, SN-38, cryptophycin analogs, deruxtecan, duocarmazine, calicheamicin, centanamycin, dolastansine, and pyrrolobenzodiazepine (PBD). In a particular embodiment, the cytotoxin is selected from the group consisting of vinca alkaloids, colchicines, taxanes, auristatins, and maytansinoids.

In a particular embodiment, the cytotoxin is exatecan or a derivative thereof, such as DX8951f.

In a particular embodiment, the cytotoxin is a maytansinoid, such as DM1. It should be noted that when a cytotoxin comprising a sulfhydryl moiety is used, the sulfhydryl moiety is capable of reacting with a maleimide moiety to form thiosuccinimide, e.g., a maytansinoid, such as DM1, and the cytotoxin may be directly linked through the thiosuccinimide. In this case, it can be understood that in some embodiments, the payload and the sulfhydryl moiety together constitute the cytotoxin, and thus in this case, the payload represents the remainder of the cytotoxin molecule except for the sulfhydryl moiety.

In a particular embodiment, the cytotoxin is an auristatin, such as MMAE (monomethyl auristatin E), MMAF (monomethyl auristatin F), or MMAD (monomethyl auristatin D). The synthesis and structure of auristatin compounds are described in US20060229253, the entire disclosure of which is incorporated herein by reference.

The payload contains a reactive group that can react with the reactive group in the compound of formula (I) and thereby covalently conjugate the payload to the compound of formula (I). Compounds that do not contain a reactive group require appropriate derivatization to obtain a payload.

In a particular embodiment, the cytotoxin is a compound of formula (i) below:

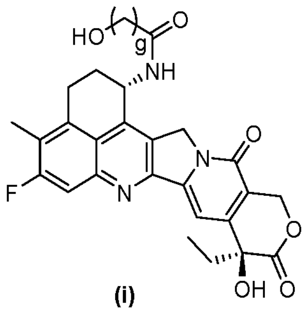

**(i)** ,

wherein, g is any integer from 1 to 6;

in an embodiment, g is any integer from 1 to 3, preferably 1.

In an embodiment, the cytotoxin is selected from the group consisting of compounds 1-14 below, wherein the wavy bond indicates the linking site for linking to the compound of formula (I).

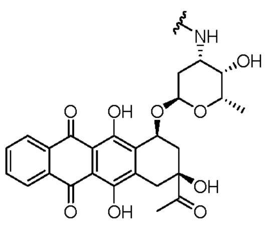

**1**
(Idarubicin)

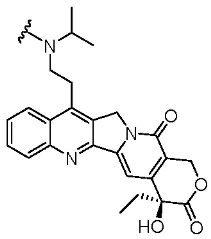

**2**
(Belotecan)

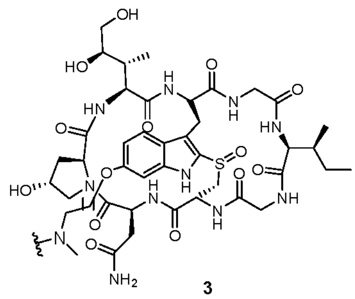

**3**
(Amanitin)

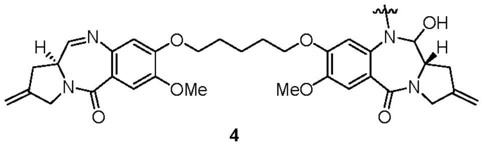

4
(PBD dimer)

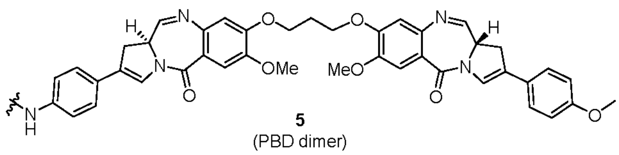

5
(PBD dimer)

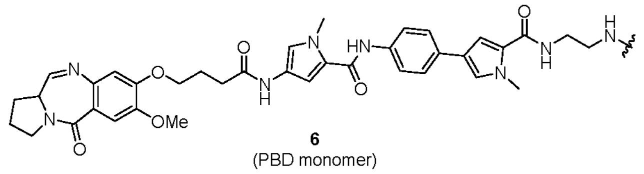

6
(PBD monomer)

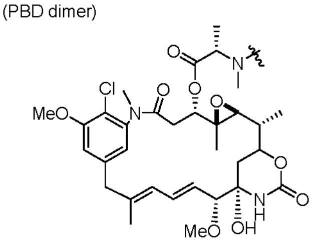

7
(DM)

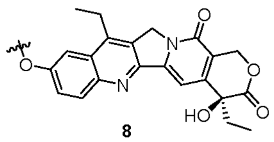

8
(SN-38)

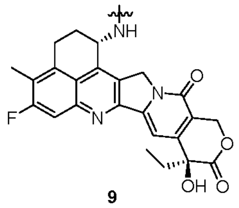

9
(DX8951f)

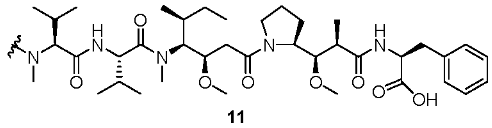

11
(MMAF)

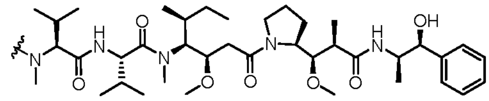

12
(MMAE)

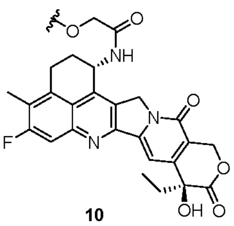

10
(Dxd-(1))

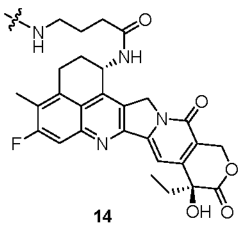

14
(Dxd-(2))

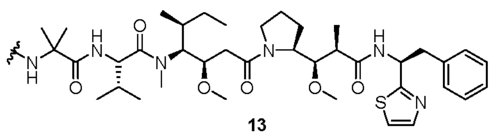

13
(Auristatin 0101)

In some embodiments, the payload is selected from DX8951f (compound 9), DXd-(1) (compound 10), and DXd-(2) (compound 14), preferably DX8951f or DXd-(1), more preferably DXd-(1).

**Preparation of Payload-Bearing Compound of Formula (I)**

In an embodiment, the linking unit and the payload are linked through the reactive groups as defined above using any reaction known in the art, including but not limited to a condensation reaction, nucleophilic addition, electrophilic addition, and the like.

In an embodiment, the payload is a cytotoxin. In an embodiment, the linking unit-payload intermediates (numberbed as LBx) are shown in the table below.

| Compound of formula (II) | Linker | Structural formula of linker | Values of i and j (if applicable)* | Payload |
|---|---|---|---|---|
| LB301-1-1 | LA301-1-1 | LA301-1 | - | Compound 10 |
| LB301-2-1 | LA301-2-1 | LA301-2 | i is 4 | Compound 10 |
| LB301-3-1 | LA301-3-1 | LA301-3 | i1 is 4, and i2 is 4 | Compound 10 |
| LB301-4-1 | LA301-4-1 | LA301-4 | i1, i2, and i3 are each 4 | Compound 10 |
| LB301-5-1 | LA301-5-1 | LA301-5 | i1, i2, i3, and i4 are each 4 | Compound 10 |
| LB302-1-1 | LA302-1-1 | LA302-1 | j is 8 | Compound 10 |
| LB302-1-4 | LA302-1-4 | LA302-1 | j is 12 | Compound 10 |
| LB302-2-1 | LA302-2-1 | LA302-2 | i is 4, and j is 8 | Compound 10 |
| LB302-2-4 | LA302-2-4 | LA302-2 | i is 4, and j is 12 | Compound 10 |
| LB302-3-1 | LA302-3-1 | LA302-3 | i1 is 4, i2 is 4, and j is 8 | Compound 10 |

(continued)

| Compound of formula (II) | Linker | Structural formula of linker | Values of i and j (if applicable)* | Payload |
|---|---|---|---|---|
| LB302-3-4 | LA302-3-4 | LA302-3 | i1 is 4, i2 is 4, and j is 12 | Compound 10 |
| LB302-4-1 | LA302-4-1 | LA302-4 | i1, i2, and i3 are each 4, and j is 8 | Compound 10 |
| LB302-4-4 | LA302-4-4 | LA302-4 | i1, i2, and i3 are each 4, and j is 12 | Compound 10 |
| *: for all listed linkers, n is 3. | | | | |

Conjugates and Preparation Thereof

Furthermore, the payload-bearing compound of formula (I) having a moiety comprising a ligase recognition sequence may be conjugated to other molecules comprising a ligase recognition sequence and thus can be used, e.g., in the preparation of an antibody-conjugated drug, such as an antibody-drug conjugate. Thus, in yet another aspect, the present disclosure provides a conjugate comprising a compound of formula (I), a targeting molecule, and a payload.

In yet another aspect, the present disclosure provides a conjugate having the structure of formula (III):

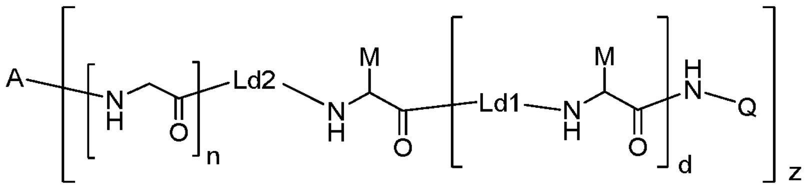 formula (III),

wherein,

n, d, Ld1, and Ld2 are as defined in formula (I);

Q is hydrogen or LKb-P;

M is hydrogen or LKa-LKb-P;

provided that Q and M are not both hydrogen;

P is a payload linked to the B moiety or the $L^1$ moiety of the compound of formula (I);

A is a targeting molecule linked to the $G_n$ moiety of the compound of formula (I); G is glycine;

z is an integer from 1 to 20.

In an embodiment, LKa and LKb are as defined in formula (I).

As defined above, in an embodiment, the $G_n$ moiety of the compound of formula (I) is a recognition sequence for ligase receptor or donor substrate, which facilitates the enzymatic coupling of the compound of formula (I) to a targeting molecule under catalysis of the ligase. The targeting molecule is optionally modified and comprises a corresponding recognition sequence for ligase receptor or donor substrate.

It should be understood that when the targeting molecule A is conjugated to the $G_n$ moiety of the compound of formula (I) under catalysis of the ligase, the recognition sequence for ligase receptor substrate and the recognition sequence for ligase donor substrate react to obtain the corresponding product sequence.

In an embodiment, the targeting molecule A comprises the recognition sequence LPXTGJ for ligase donor substrate, wherein X and J are as defined above. When conjugated to the corresponding recognition sequence $G_n$ for ligase receptor substrate, the peptide bond upstream of glycine in LPXTGJ sequence is cleaved by Sortase A, and the generated intermediate is linked to the free N-terminus of $G_n$ to generate a new peptide bond. The resulting amino acid sequence is LPXTG$_n$. The sequences $G_n$ and LPXTGJ are as defined above.

In an embodiment, P is linked to the B moiety or the $L^1$ moiety of the compound of formula (I), and A is linked to the $G_n$ moiety of the compound of formula (I), thus forming a compound of formula (III).

As described above, $R^{10}$ will not be present in the B-P structure of the compound of formula (III). As described

above, when B in the compound of formula (I) is the terminal group $R^{10}$, B in the B-P structure of the compound of formula (III) is correspondingly absent.

As described above, in the A-$G_n$ structure of the compound of formula (III), A optionally comprises the corresponding product sequence obtained by the reaction of the recognition sequence for ligase receptor substrate and the recognition sequence for ligase donor substrate.

In an embodiment, M is hydrogen or LKa-$L^2$-$L^1$-B-P, wherein P is a payload linked to the B moiety or the $L^1$ moiety of the compound of formula (I); each B is independently a terminal group $R^{10}$ or a combination of 1), 2), and 3) below: 1) a self-immolative spacer Sp1; 2) a bond, or one divalent group, or a combination of two or more divalent groups, wherein the divalent group is selected from: - $CR^1R^2$-, $C_{1-10}$ alkylene, $C_{4-10}$ cycloalkylene, $C_{4-10}$ heterocyclylene, and -(CO)-; and 3) a terminal group $R^{10}$; $R^{10}$ is hydrogen or a group that can leave when reacting with a group in the payload; $R^{10}$ represents a structural moiety that will not be present in the product molecule resulting from the reaction of B with the payload.

In an embodiment, M is hydrogen or LKa-$L^2$-$L^1$-B-P, wherein each B is independently absent or is a combination of 1) and 2) below: 1) a self-immolative spacer Sp1; and 2) a bond, or one divalent group, or a combination of two or more divalent groups, wherein the divalent group is selected from: - $CR^1R^2$-, $C_{1-10}$ alkylene, and -(CO)-. In a preferred embodiment, M is hydrogen or LKa-$L^2$-$L^1$-B-P, wherein each B is independently absent or is -NH-$CH_2$-U- or -NH-$CH_2$-U-$(CH_2)_g$-(CO)-; g is any integer from 1 to 6, preferably 1. In another embodiment, M is LKa-$L^2$-$L^1$-B-P. In an embodiment, B in LKa-$L^2$-$L^1$-B-P is absent. In an embodiment, B in LKa-$L^2$-$L^1$-B-P is a combination of 1) and 2) below: 1) a self-immolative spacer Sp1; and 2) a bond, or one divalent group, or a combination of two or more divalent groups, wherein the divalent group is selected from: -$CR^1R^2$-, $C_{1-10}$ alkylene, and -(CO)-. In an embodiment, B in LKa-$L^2$-$L^1$-B-P is -NH-$CH_2$-U- or -NH-$CH_2$-U-$(CH_2)_g$-(CO)-. U is O, S, or NH, preferably O or S. In an embodiment, B in the compound of formula (I) is linked to the payload via an amide bond, an ester bond, or an ether bond. In an embodiment, M is LKa-$L^2$-$L^1$-B-P and B is absent, and M may also be denoted as LKa-$L^2$-$L^1$-P.

**Targeting Molecule**

In an embodiment, the targeting molecule is an antibody or an antigen-binding fragment thereof.

In an embodiment, the targeting molecule is an anti-human HER2 antibody or an antigen-binding fragment thereof. Examples of anti-human HER2 antibodies include, but are not limited to, pertuzumab and trastuzumab. Pertuzumab binds to the second extracellular domain (ECD2) of HER2 and is approved for the treatment of HER2-positive breast cancer. Trastuzumab binds to the fourth extracellular domain (ECD4) of HER2 and is approved for the treatment of HER2-positive breast cancer and gastric cancer.

In a preferred embodiment, the anti-human HER2 antibody is one or more antibodies selected from trastuzumab-based engineered anti-HER2 antibodies.

In a preferred embodiment, the anti-human HER2 antibody is a recombinant antibody selected from a monoclonal antibody, a chimeric antibody, a humanized antibody, an antibody fragment, and an antibody mimetic. In an embodiment, the antibody mimetic is selected from a scFv, a minibody, a diabody, and a nanobody. For conjugation to the compound of formula (I), the targeting molecule of the present disclosure may comprise a modification moiety to link to $G_n$ in the compound of formula (I). The position for the introduction of such a modification moiety is not limited. For example, when the targeting molecule is an antibody, the position for its introduction may be, but is not limited to, the C-terminus or N-terminus of the heavy chain or light chain of the antibody.

In an alternative embodiment, the modification moiety for conjugating to $G_n$ in the compound of formula (I) may be introduced at a non-terminal position of the heavy chain or light chain of the antibody, using, e.g., a chemical modification method.

In an embodiment, the targeting molecule of the present disclosure is an antibody or an antigen-binding fragment thereof, which may comprise a terminal modification. A terminal modification refers to the modification at the C-terminus or N-terminus of the heavy chain or light chain of an antibody, which, for example, comprises a ligase recognition sequence. In another embodiment, the terminal modification may further comprise a spacer Sp2 having 2-100 amino acids, wherein the antibody, the Sp2, and the ligase recognition sequence are linked sequentially. In a preferred embodiment, Sp2 is a spacer sequence comprising 2-20 amino acids. In a particular embodiment, Sp2 is a spacer sequence selected from GA, GGGGS, GGGGSGGGGS, and GGGGSGGGGSGGGGS, particularly GA.

In a preferred embodiment, the light chain of the antibody or the antigen-binding fragment thereof comprises 3 types: wild type (LC); C-terminus-modified light chain in which the ligase recognition sequence LPXTG is introduced directly for modification (LCCT); and C-terminus-modified light chain in which a short peptide spacer and the recognition sequence LPXTG for ligase donor substrate are introduced for modification ($LCCT_L$). The heavy chain of the antibody or the antigen-binding fragment thereof comprises 3 types: wild type (HC); C-terminus-modified heavy chain in which the ligase recognition sequence LPXTG is introduced directly for modification (HCCT); and C-terminus-modified heavy chain in which a short peptide spacer and the recognition sequence LPXTG for ligase donor substrate are introduced for modification ($HCCT_L$). X may be any natural or non-natural single amino acid. When z in the compound of formula (III) is 1

or 2, the above heavy and light chain combinations can form 8 preferred antibody molecules (see the amino acid sequence listing).

The conjugate of the present disclosure may further comprise a payload. The payload is as described above.

**Specific Embodiments of Conjugates**

In an embodiment, Q is hydrogen and each Lka is

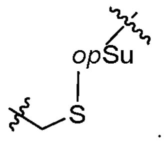

In an embodiment, the compound of formula (III) has the structure of formula (III-1):

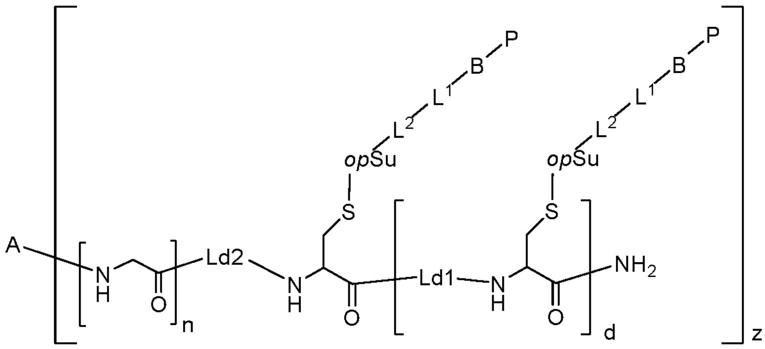

formula (III-1).

In an embodiment, Ld2 is a bond, and d is 0. In an embodiment, the compound of formula (III-1) is as follows:

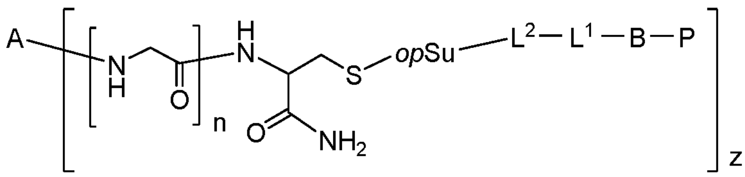

(conjugate LC301-1).

In an embodiment, d is 0, and Ld2 is

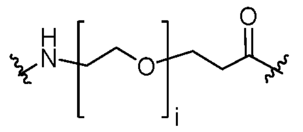

In an embodiment, the compound of formula (III-1) is as follows:

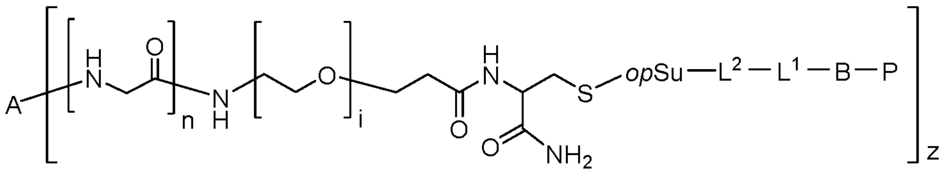

(conjugate LC301-2).

In an embodiment, d is 1, 2, or 3, and Ld2 and each Ld1 are independently selected from

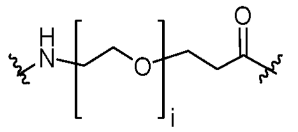

In an embodiment, the compound of formula (III-1) is as follows:

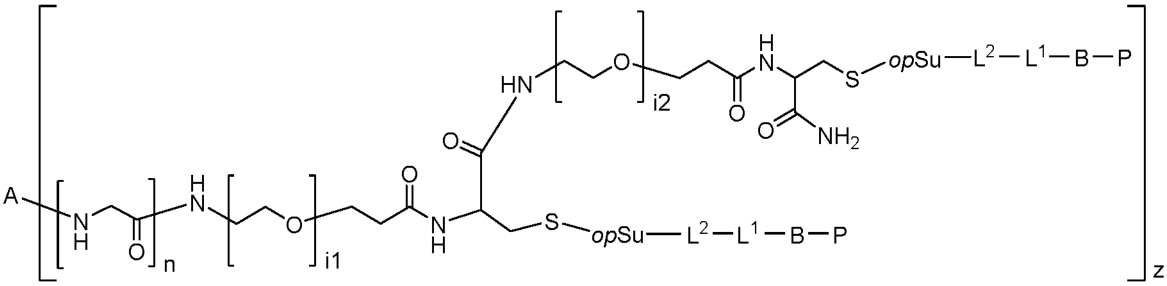

(conjugate LC301-3),

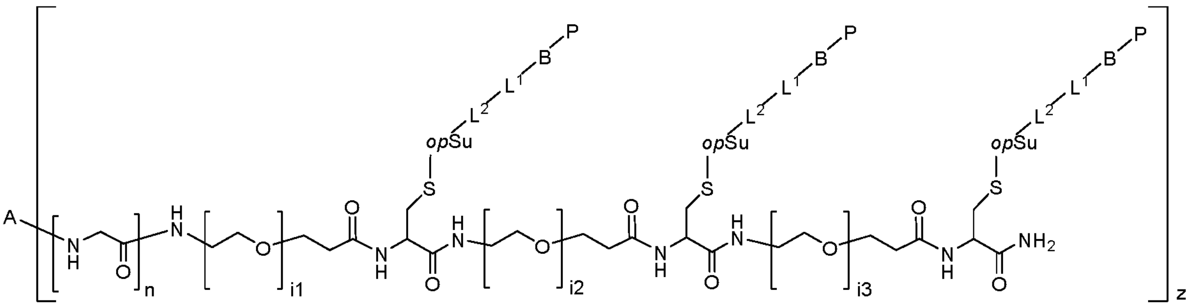

(conjugate LC301-4),

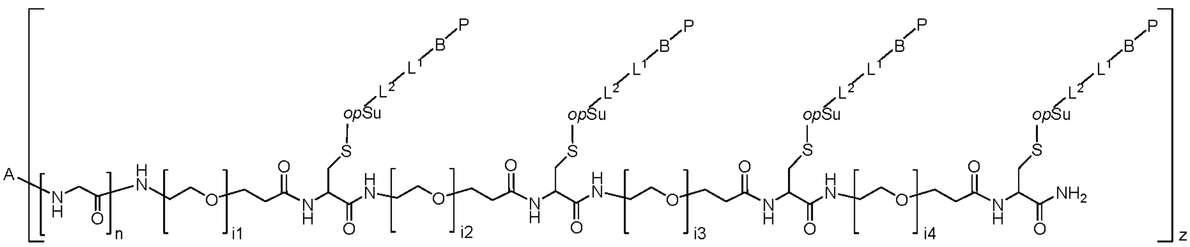

(conjugate LC301-5).

In an embodiment, Ld2 is

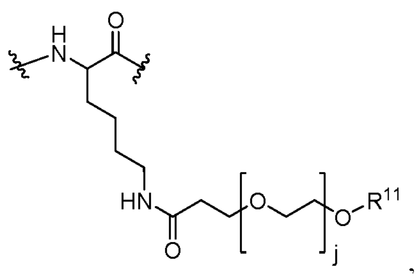

and d is 0. In an embodiment the compound of formula (III-1) is as follows:

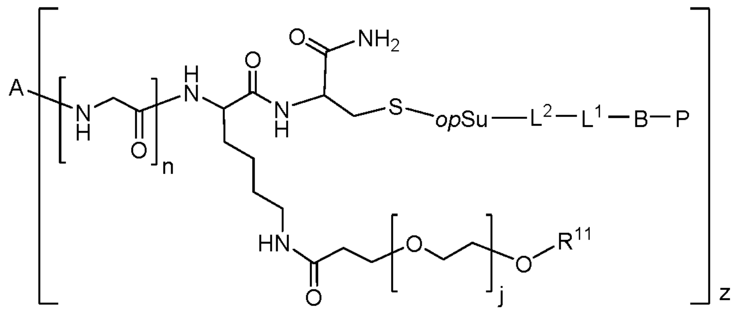

(conjugate LC302-1).

In an embodiment, d is 1, 2, or 3, Ld2 is

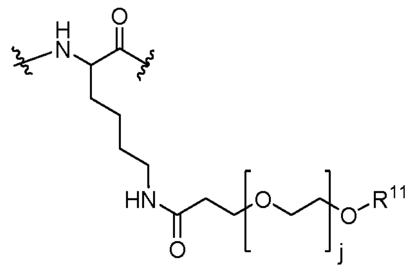

,

and each Ld1 is independently selected from

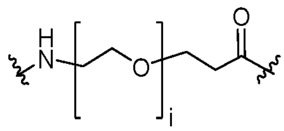

.

In an embodiment, the compound of formula (III-1) is as follows:

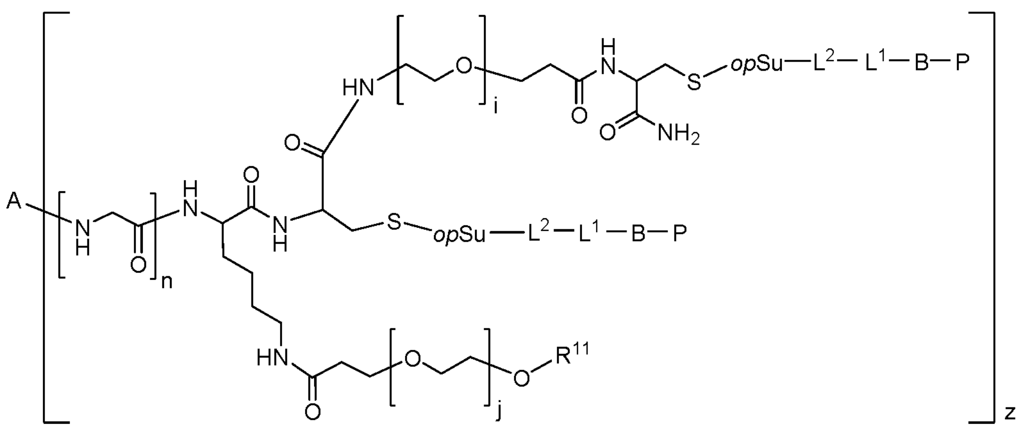

(conjugate LC302-2),

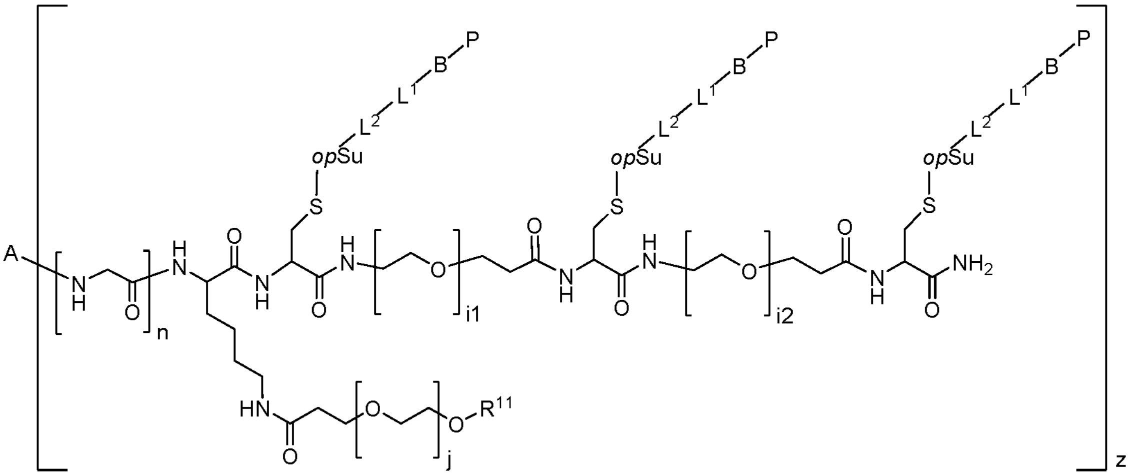

(conjugate LC302-3),

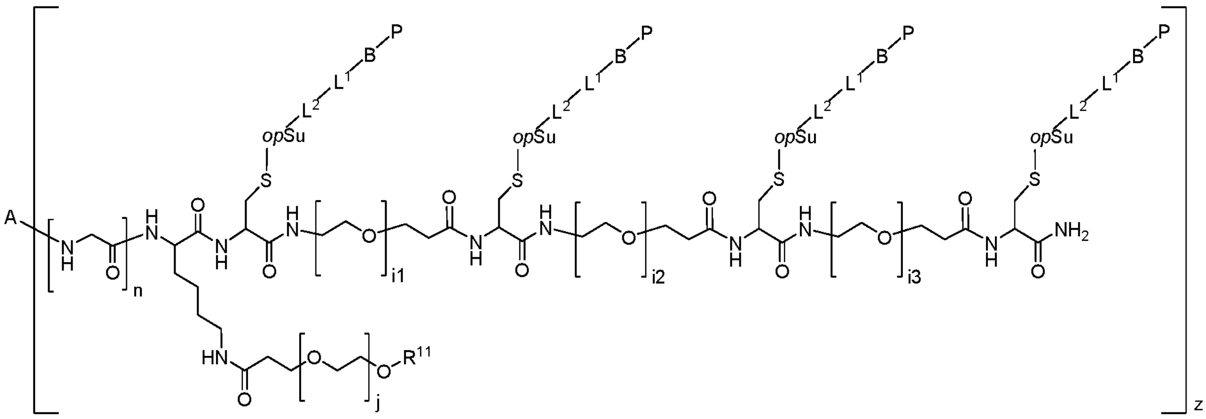

(conjugate LC302-4).

In an embodiment, z is 1-4; in an embodiment, z is 2 or 4; in an embodiment, z is 2; in an ambodiment, in the conjugates LC301-1, LC301-2, and LC302-1, z in 2 or 4. In an embodiment, in the conjugates LC301-3, LC301-4, LC301-5, LC302-2, LC302-3, and LC302-4, z is 2.

In an embodiment, z is 1-4; in an embodiment, z is 2 or 4; in an embodiment, z is 2; in an embodiment, in the conjugates LC301-1, LC301-2, and LC302-1, z is 2 or 4. In an embodiment, in the conjugates LC301-3, LC301-4, LC301-5, LC302-2, LC302-3, and LC302-4, z is 2.

In an embodiment, B in the compound of formula (I) is a terminal group $R^{10}$, and the cleavable sequence 1 in $L^1$ is linked to the payload to form the compound of formula (II), wherein B is not present in the molecule resulting from the linking of the cleavable sequence 1 to the payload. In this case, it can also be understood as follows: M is LKa-$L^2$-$L^1$-B-P and B is absent. In this case, M may also be denoted as $LK_A$-$L^2$-$L^1$-P. In an embodiment, n is 3, $L^2$ is $-(CH_2)_p-(CH_2)_2(CO)-$, p is 3, $L^1$ is GGFG, B is $-NH-CH_2-U-$, or is absent, or is $-NH-CH_2-U-(CH_2)_g-(CO)-$, U is O, and g is 1. In an embodiment, the conjugate LC301-1 has the following structure:

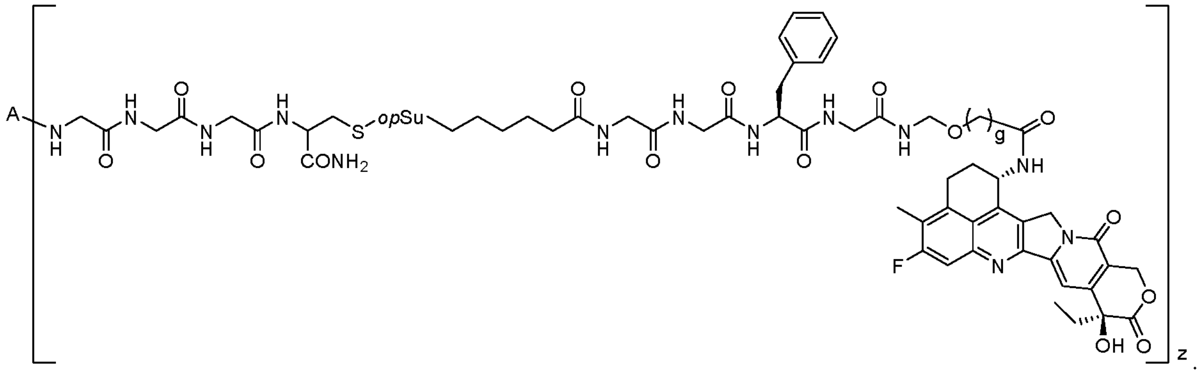

In an embodiment, the conjugate LC301-2 has the following structure:

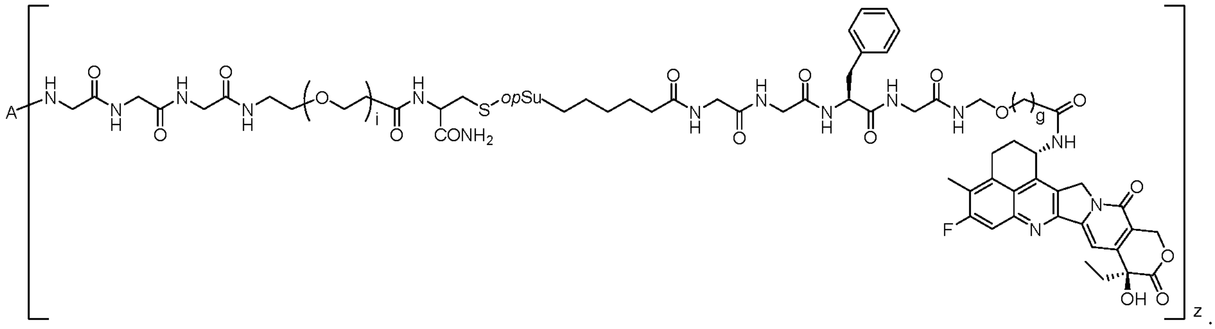

In an embodiment, the conjugate LC301-3 has the following structure:

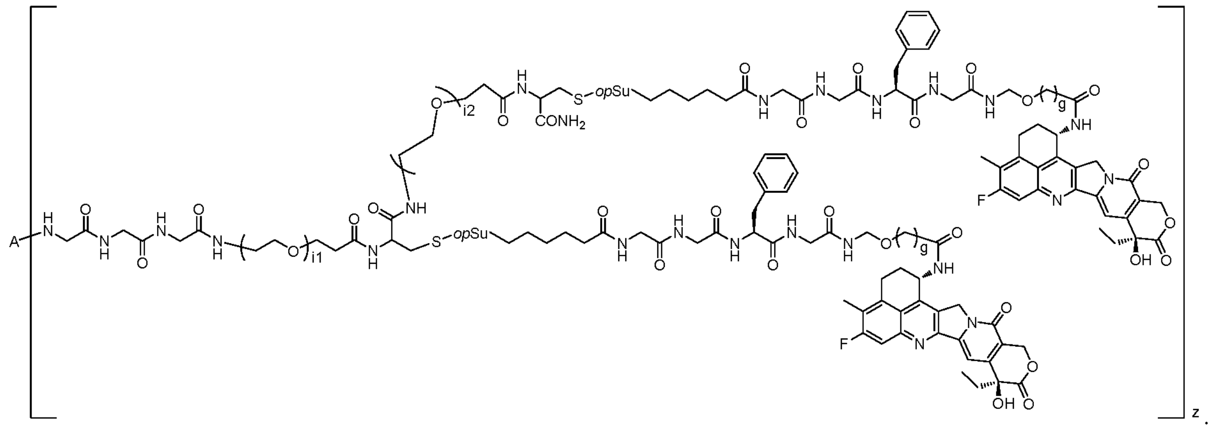

In an embodiment, the conjugate LC301-4 has the following structure:

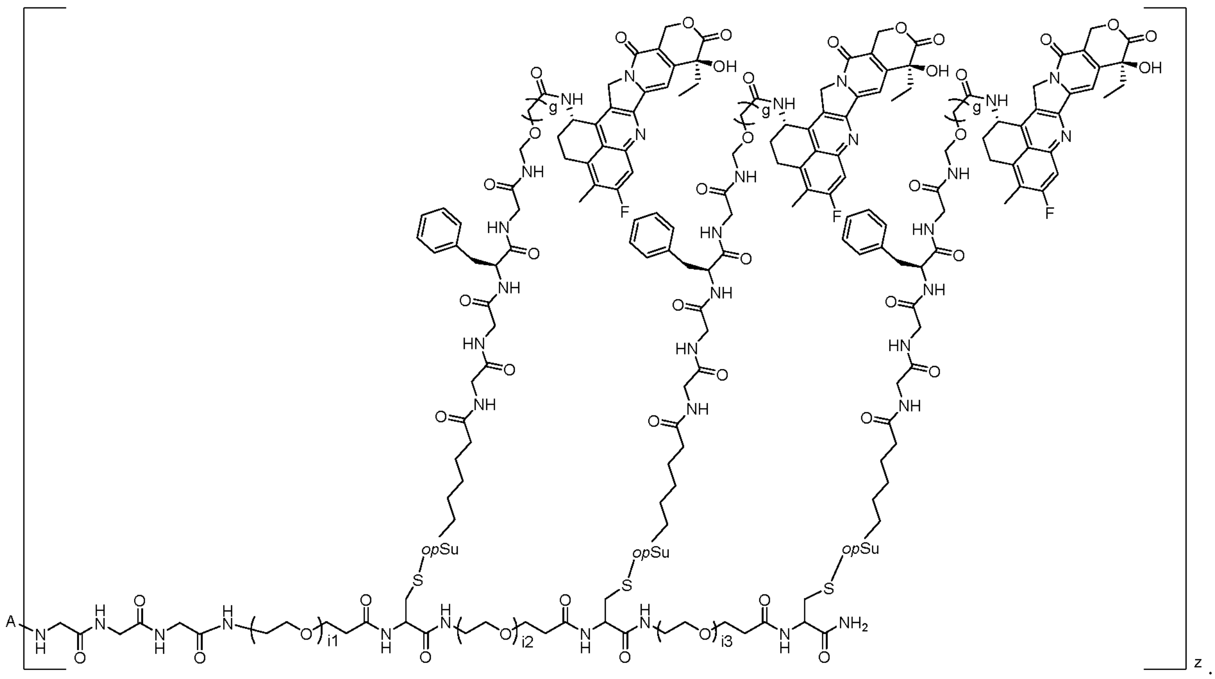

In an embodiment, the conjugate LC301-5 has the following structure:

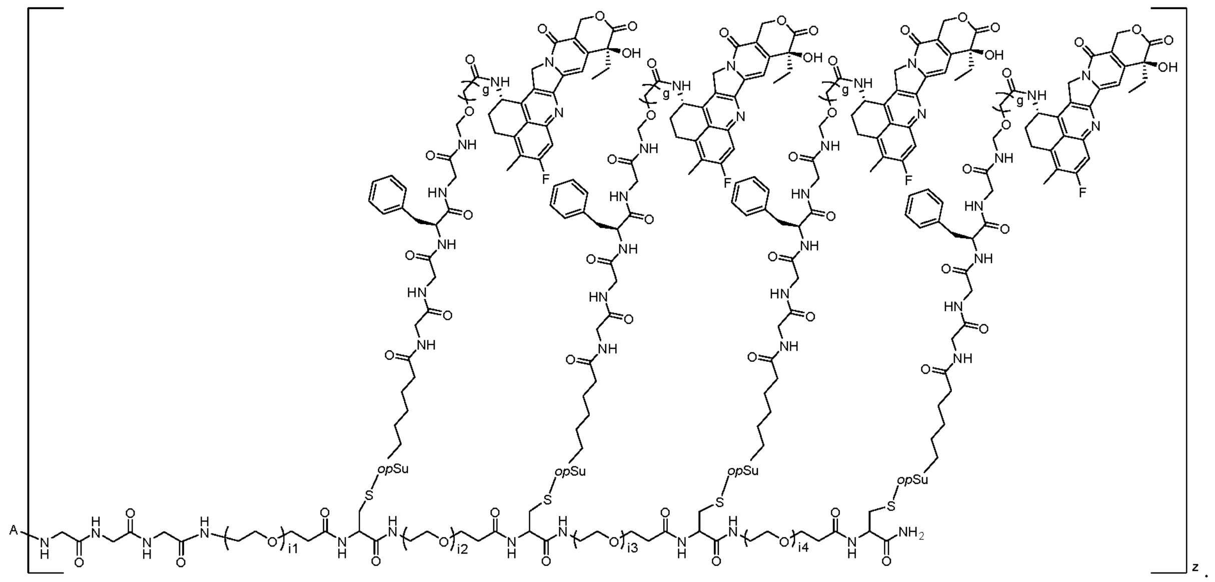

In an embodiment, the conjugate LC302-1 has the following structure:

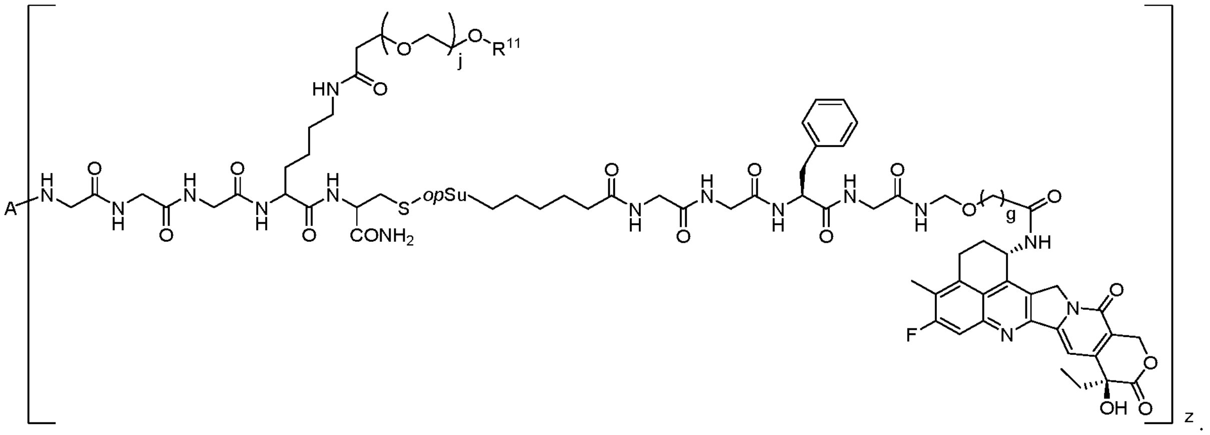

In an embodiment, the conjugate LC302-2 has the following structure:

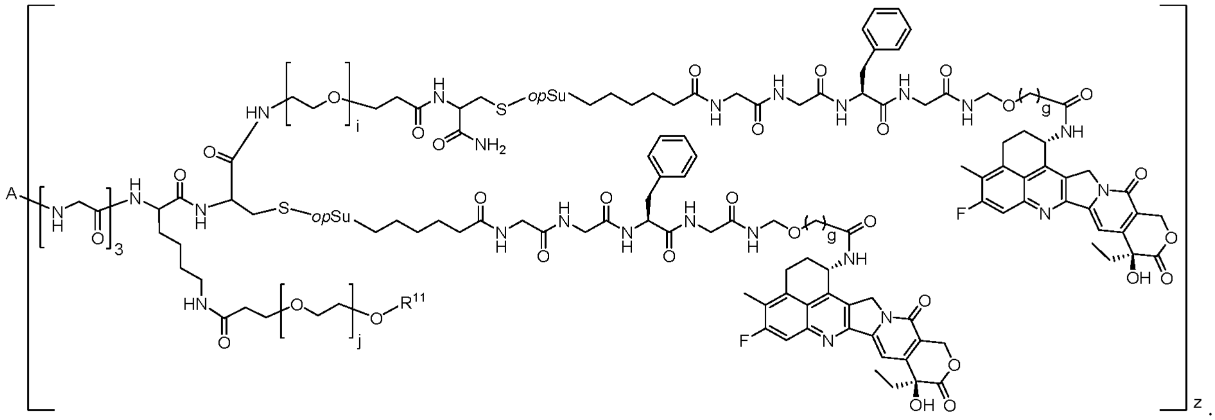

In an embodiment, the conjugate LC302-3 has the following structure:

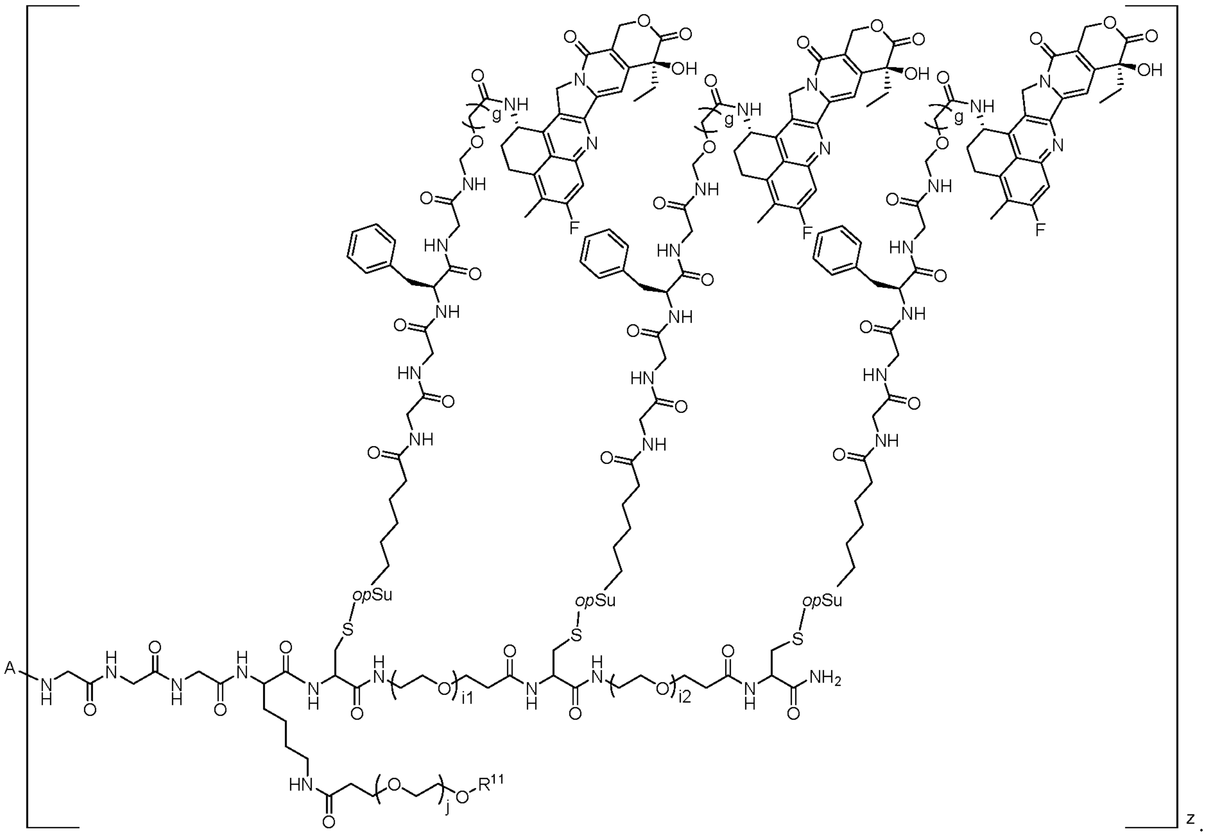

In an embodiment, the conjugate LC302-4 has the following structure:

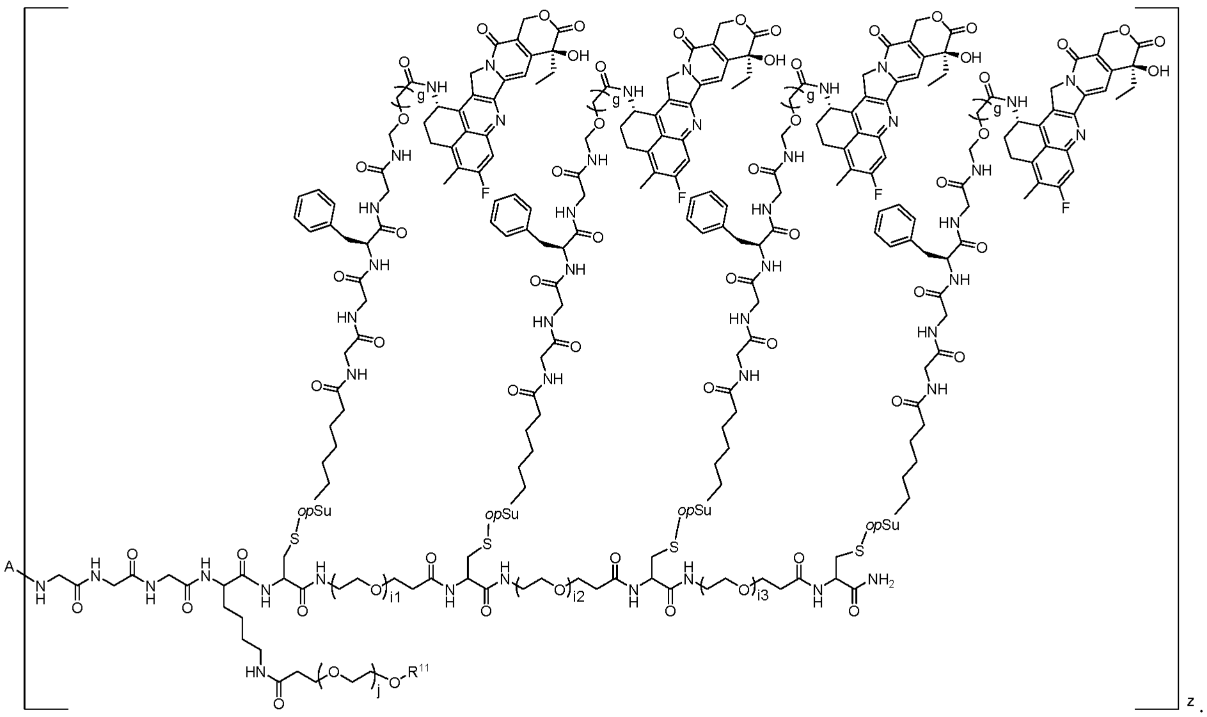

In an embodiment, i is 4, g is 1, and $R^{11}$ is methyl.

In an embodiment, the conjugate LC301-2 is as shown below (conjugate LC301-2-1):

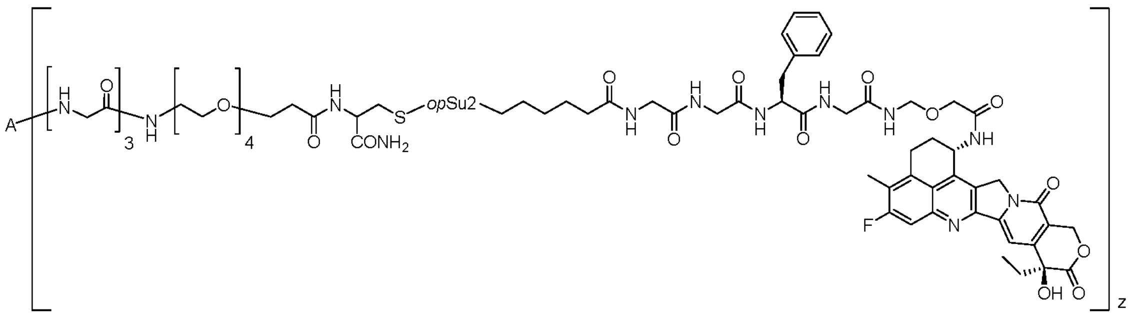

(Conjugate LC301-2-1).

In an embodiment, i is 4, j is 8, g is 1, and $R^{11}$ is methyl. In an embodiment, the conjugate LC302-2 is as shown below (conjugate LC302-2-1):

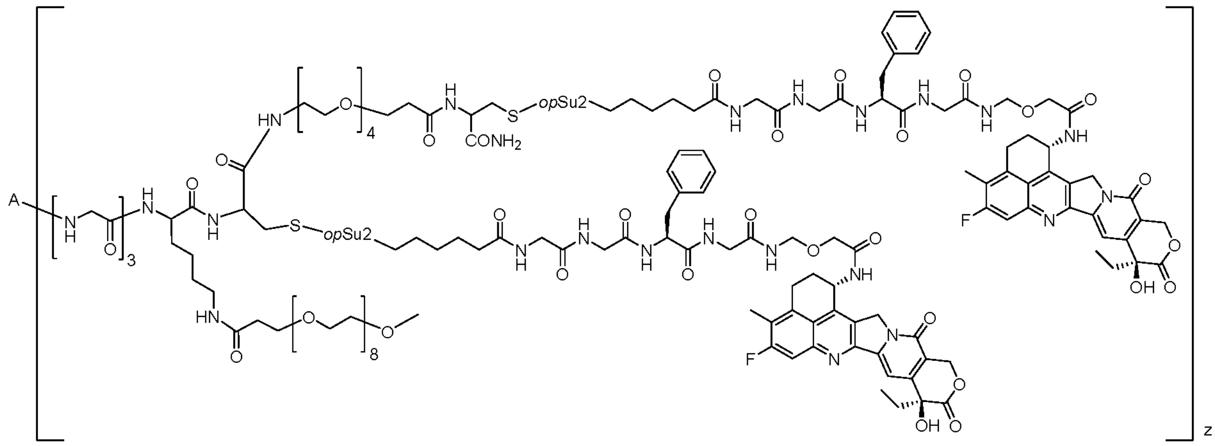

(conjugate LC302-2-1).

In an embodiment, i is 4, j is 12, g is 1, and $R^{11}$ is methyl. In an embodiment, the conjugate LC302-2 is as shown below (conjugate LC302-2-4):

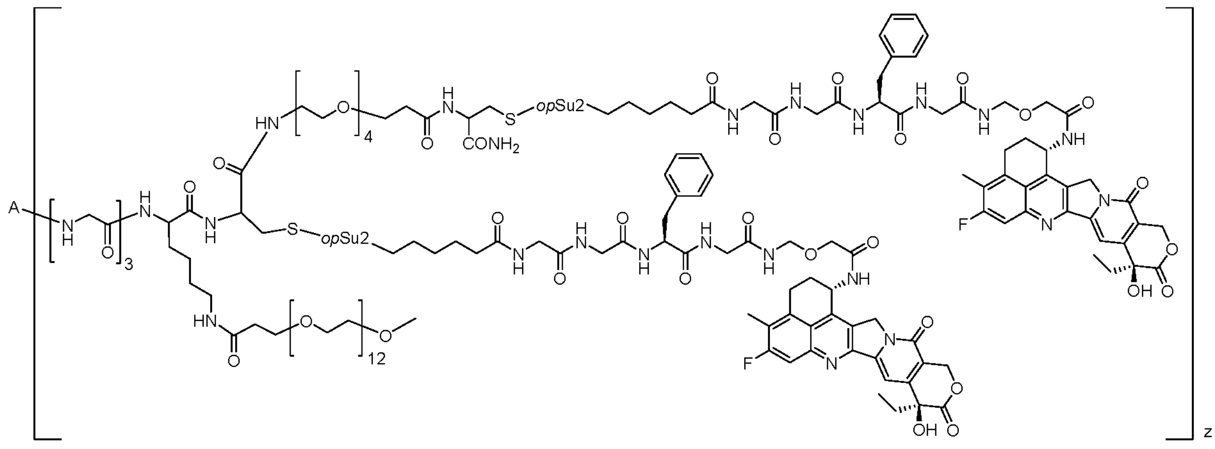

(conjugate LC302-2-4).

### Preparation of Conjugates

The conjugates of the present disclosure may be prepared by any method known in the art. In some embodiments, the conjugates are prepared by site-specific conjugation of a targeting molecule and a payload-bearing compound of formula (I) under catalysis of a ligase, wherein the targeting molecule is modified by a ligase recognition sequence. The method comprises step A and step B.

Step (A). Preparation of linking unit-payload intermediate

In a preferred embodiment, B in the compound of formula (I) is covalently linked via a reactive group to a payload comprising another reactive group.

The linking unit-payload intermediate prepared by using the compound of formula (I) of the present disclosure features definite structure, definite composition, and high purity, such that less or no other impurities are introduced when the intermediate is subjected to coupling reaction with an antibody. When such intermediates are used for site-specific conjugation to modified antibodies containing a ligase recognition sequence under catalysis of a ligase, homogeneous ADCs with highly controllable quality are obtained.

Step B. Linking of targeting molecule to payload-bearing compound of formula (I)

The targeting molecule of the present disclosure can be conjugated to the payload-bearing compound of formula (I) (i.e., the compound of formula (II)) by any method known in the art.

The targeting molecule and the payload-bearing compound of formula (I) are linked to each other by a ligase-specific recognition sequence of the substrate. The recognition sequence depends on the particular ligase used. In an embodiment, the targeting molecule is an antibody that has a recognition sequence-based terminal modification introduced at the C-terminus of the light chain and/or heavy chain, and the targeting molecule is conjugated to the compound of formula (II) under catalysis of a wild-type ligase, an optimized engineered ligase, or any combination thereof, as well as under suitable catalytic reaction conditions.

In a specific embodiment, the ligase is Sortase A, and the conjugation reaction can be represented by the following scheme:

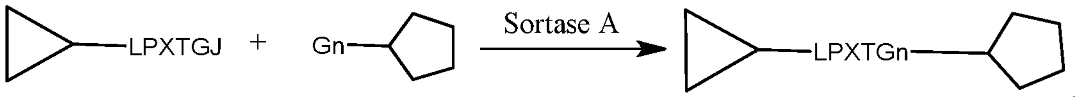


The triangle represents a part of the antibody, and the pentagon represents a part of the compound of formula (II). n, X, and J are each as defined above. When conjugated to the corresponding recognition sequence $G_n$ for receptor substrate, the peptide bond upstream of glycine in LPXTGJ sequence is cleaved by Sortase A, and the generated intermediate is linked to the free N-terminus of $G_n$ to generate a new peptide bond. The resulting amino acid sequence is $LPXTG_n$. The sequences $G_n$ and LPXTGJ are as defined above.

**Metabolism of Conjugates in Physiological Environment**

When part or all of the linker in the tumor cell is cleaved, the anti-tumor compound is partially released to exhibit the anti-tumor effect of the anti-tumor compound. Since the linker is cleaved at the site of linking to the drug, the anti-tumor compound is released in its inherent structure to exhibit its inherent anti-tumor effect.

In an embodiment, the cleavable sequence 1 (e.g., GGFG) may be cleaved by a lysosomal enzyme (e.g., cathepsin B and/or cathepsin L).

In an embodiment, Sp1 comprises a self-immolative spacer. In an embodiment, Sp1 comprises PABC, acetal, or heteroacetal. In an embodiment, $L^1$ is GGFG. In an embodiment, the linker comprises - GGFG-NH-$CH_2$-O-. In an embodiment, -GGFG-NH-$CH_2$-O- represents a combination of a restriction enzyme site and a self-immolative spacer that will cleave and release the molecule of interest (e.g., drug) in the cell.

**Table of Specific Conjugates**

In an embodiment, the payload is a cytotoxin or a fragment thereof. In an embodiment, the antibody is modified trastuzumab, preferably Ab0001-$LCCT_L$-HC (light chain: SEQ ID NO: 1; heavy chain: SEQ ID NO: 2) or Ab0001-$LCCT_L$-$HCCT_L$ (light chain: SEQ ID NO: 3; heavy chain: SEQ ID NO: 4). The sequences of Ab0001-$LCCT_L$-HC and Ab0001-$LCCT_L$-$HCCT_L$ are all based on the amino acid sequence of Ab0001 (trastuzumab), with GALPETGG introduced at the C-terminus of the light chain (Ab0001-$LCCT_L$-HC) or GALPETGG introduced at the C-termini of the light and heavy chains (Ab0001-$LCCT_L$-$HCCT_L$), wherein LPETGG is the recognition sequence for ligase donor substrate, and GA is the spacer sequence. In Ab0001-$LCCT_L$-$HCCT_L$, the C-terminal lysine of the Ab0001 heavy chain may be retained, as shown in SEQ ID NO: 4, or may be deleted prior to the introduction of GALPETGG (resulting sequence not shown in sequence listing).

In a preferred embodiment, in the conjugates of the present disclosure, the payload (represented by P in formula (III)) is a compound of formula (i) or compounds 1-14, preferably compound 10. In a preferred embodiment, the linker-payload in the conjugates of the present disclosure (the moiety except for A in formula (III)) has a structure selected from LB301-1-1, LB301-2-1, LB301-3-1, LB301-4-1, LB301-5-1, LB302-1-1, LB302-1-4, LB302-2-1, LB302-2-4, LB302-3-1, LB302-3-4, LB302-4-1, and LB302-4-4, as described above. In a preferred embodiment, in the conjugates of the present disclosure, the targeting molecule (represented by A in formula (III)) is selected from Ab0001-LCCT$_L$-HC and Ab001-LCCT$_L$-HCCT$_L$.

In a more preferred embodiment, the conjugates of the present disclosure are shown in the table below. (Nomenclature of ADCs: the number in the parenthesis represents the number of payload (drug) molecules intended to be linked to the antibody).

| ADC | Structural formula of linker-payload | Payload | A (targeting molecule) |
|---|---|---|---|
| LC301-1-1(2) | LB301-1-1 | Compound 10 | Ab0001-LCCT$_L$-HC |
| LC301-1-1(4) | LB301-1-1 | Compound 10 | Ab0001-LCCT$_L$-HCCT$_L$ |
| LC301-2-1(2) | LB301-2-1 | Compound 10 | Ab0001-LCCT$_L$-HC |
| LC301-2-1(4) | LB301-2-1 | Compound 10 | Ab0001-LCCT$_L$-HCCT$_L$ |
| LC301-3-1(4) | LB301-3-1 | Compound 10 | Ab0001-LCCT$_L$-HC |
| LC301-4-1(6) | LB301-4-1 | Compound 10 | Ab0001-LCCT$_L$-HC |
| LC301-5-1(8) | LB301-5-1 | Compound 10 | Ab0001-LCCT$_L$-HC |
| LC302-1-1(2) | LB302-1-1 | Compound 10 | Ab0001-LCCT$_L$-HC |
| LC302-1-1(4) | LB302-1-1 | Compound 10 | Ab0001-LCCT$_L$-HCCT$_L$ |
| LC302-1-4(2) | LB302-1-4 | Compound 10 | Ab0001-LCCT$_L$-HC |
| LC302-1-4(4) | LB302-1-4 | Compound 10 | Ab0001-LCCT$_L$-HCCT$_L$ |
| LC302-2-1(4) | LB302-2-1 | Compound 10 | Ab0001-LCCT$_L$-HC |
| LC302-2-4(4) | LB302-2-4 | Compound 10 | Ab0001-LCCT$_L$-HC |
| LC302-3-1(6) | LB302-3-1 | Compound 10 | Ab0001-LCCT$_L$-HC |
| LC302-3-4(6) | LB302-3-4 | Compound 10 | Ab0001-LCCT$_L$-HC |
| LC302-4-1(8) | LB302-4-1 | Compound 10 | Ab0001-LCCT$_L$-HC |
| LC302-4-4(8) | LB302-4-4 | Compound 10 | Ab0001-LCCT$_L$-HC |

Pharmaceutical Composition and Pharmaceutical Formulation

The pharmaceutical composition described herein comprises an active ingredient, a buffering agent, a stabilizer, and optionally a surfactant, wherein the active ingredient comprises the conjugate of formula (III) described above. At least one other pharmaceutically acceptable carrier, apart from those described above, may be included according to actual needs and without hindering the technical effects of the present disclosure.

To achieve the effects of improving storage stability and reducing degradation of the active ingredient, preferably, the buffer is selected from an acetic acid buffer, a histidine buffer, a citric acid buffer, and a phosphoric acid buffer; more preferably, the buffer is an acetic acid buffer or a histidine buffer, and further more preferably a histidine buffer. The molarity of the buffer may be 10-50 mM, e.g., about 15 mM, 16 mM, 18 mM, 19 mM, 20 mM, 21 mM, 23 mM, 24 mM, or 25 mM.

The stabilizer used in the formulation of the present disclosure may include saccharide. To achieve the effects of improving storage stability and reducing degradation of the active ingredient, preferably, the saccharide is selected from sucrose and trehalose (or trehalose dihydrate); more preferably, the saccharide is sucrose. The concentration of the saccharide may be 1-20% (w:v), e.g., about 6%, 7%, 8%, 8.1%, 9%, 10%, or 12% (w:v).

To achieve the effects of improving storage stability and reducing degradation of the active ingredient, preferably, the pH of the pharmaceutical composition is weakly acidic to neutral, e.g., 5.0-6.5, preferably 5.2-6.0, and more preferably about 5.5.

To reduce the surface tension of the solution, increase the solubility of the protein, and simultaneously inhibit the aggregation of protein molecules under mechanical stress, the pharmaceutical composition described herein may further comprise a surfactant. The surfactant may be selected by those skilled in the art according to actual needs. The surfactant may be a polysorbate (tween), such as polysorbate 20 or polysorbate 80. The concentration of the surfactant may be 0.01-10 mg/mL, such as 0.1-1 mg/mL, e.g., about 0.2 mg/mL, 0.4 mg/mL, 0.6 mg/mL, 0.8 mg/mL, or 1 mg/mL.

The pharmaceutical composition of the present disclosure may be administered in any manner as long as it achieves the effect of preventing, alleviating, or treating the symptoms of a human being or an animal. For example, various suitable dosage forms may be prepared according to the administration route, particularly injections such as a lyophilized powder injection, an injection, or sterile injection powder. The pharmaceutical composition of the present disclosure may contain water. Those skilled in the art will judge, based on their general knowledge, that in some dosage forms (e.g., an injection), the pharmaceutical composition of the present disclosure contains water, while in other dosage forms (e.g., a lyophilized powder injection), the pharmaceutical composition is stored in an anhydrous form during storage, although it is in an aqueous form during preparation and use.

The term "pharmaceutically acceptable" means that a substance, when in contact with tissues of the patient within the scope of sound medical judgment, results in no undue toxicity, irritation, allergic reaction, or the like, has a reasonable advantage-disadvantage ratio, and is effective for the intended use.

The term pharmaceutically acceptable carrier refers to those carrier materials that are pharmaceutically acceptable and do not interfere with the biological activity and performance of the conjugate. Examples of aqueous carriers include, but are not limited to, buffered saline and the like. Pharmaceutically acceptable carriers also include carrier substances that allow the composition to approximate physiological conditions, such as pH adjusters and buffering agents, and toxicity adjusters, sodium acetate, sodium chloride, potassium chloride, calcium chloride, sodium lactate, and the like.

In an embodiment, the drug/antibody ratio (DAR) of the pharmaceutical composition of the present disclosure is an integer or non-integer from 1 to 20, for example from about 1 to about 10, from about 1 to about 8, from about 1 to about 6, from about 1 to about 4, from about 1 to about 3, from about 1 to about 2.5, or from about 1 to about 2. In a particular embodiment, the DAR of the conjugate of the present disclosure is about 2, about 4, about 6, or about 8.

Treatment Method and Use

The pharmaceutical composition of the present disclosure can be used for treating a tumor and/or an autoimmune disease. Tumors susceptible to conjugate treatment include those characterized by specific tumor-associated antigens or cell surface receptors, and these tumor cells can be recognized by the targeting molecule in the conjugate and thereby can be killed by the payload/cytotoxin in the conjugate.

Accordingly, in yet another aspect, the present disclosure further provides use of the pharmaceutical composition of the present disclosure in the manufacture of a medicament for treating, ameliorating, or alleviating a disease, a disorder, or a condition selected from a tumor or an autoimmune disease.

In another aspect, the present disclosure provides the pharmaceutical composition of the present disclosure for use in treating a tumor or an autoimmune disease.

In a further aspect, the present disclosure provides a method for treating a tumor or an autoimmune disease, and the method comprises administering to an individual in need thereof an effective amount of the pharmaceutical composition of the present disclosure.

In a preferred embodiment, the conjugate provided by the present disclosure and formed by linking an anti-human HER2 antibody to a small molecule cytotoxin can specifically bind to HER2 on the surface of tumor cells and thus selectively kill tumor cells expressing HER2. In another preferred embodiment, the present disclosure provides use of the pharmaceutical composition of the present disclosure in the manufacture of a medicament for treating, ameliorating, or alleviating a disease, a disorder, or a condition selected from HER2-positive tumors. In a more preferred embodiment, the disease, the disorder, or the condition is selected from the group consisting of breast cancer, gastric cancer, lung cancer, ovarian cancer, urothelial cancer, and the like.

The content of the active ingredient (i.e., conjugate) in the pharmaceutical composition administered to a subject can be adjusted to a considerable extent. The amount thereof may vary depending on the particular administration route and the needs of the subject and may be determined based on the judgment of a healthcare professional.

**Beneficial Effects**

The antibody-drug conjugates of the present disclosure use a specially designed linker-payload, are more stable, and can achieve a good therapeutic effect at a relatively low DAR, thus reducing side effects and improving therapeutic index.

The present disclosure uses a linking unit with a unique structure and uses a ligase to catalyze the conjugation of a targeting molecule to a payload. The conjugates of the present disclosure feature good homogeneity, high activity, and high selectivity. In addition, the toxicity of the linking unit-payload intermediate is far lower than that of a free payload, such that the manufacturing process of the drug is less harmful, and the industrial production is facilitated.

The conjugates of the present disclosure achieve at least one of the following technical effects:

(1) High inhibitory activity for target cells or strong killing effect for target cells.

(2) Excellent physicochemical properties (e.g., solubility and physical and/or chemical stability).

(3) Excellent pharmacokinetic properties (e.g., good stability in plasma and suitable half-life and duration of action).

(4) Excellent safety (lower toxicity and/or less side effects for non-target normal cells or tissues, and relatively wide therapeutic window), and the like.

(5) Being highly modularized in design and thereby enabling assembly of multiple drugs in a simple way.

The formulation of the present disclosure is stable and can realize long-term stable storage of drugs.

## Examples

Preparation Examples

To more clearly illustrate the objects and technical solutions of the present disclosure, the present disclosure will be further described with reference to specific examples. It should be understood that these examples are merely intended to illustrate the present disclosure rather than limit the scope of the present disclosure. Specific experimental methods not mentioned in the following examples are performed according to the conventional experimental methods.

### Instruments, materials, and reagents

Unless otherwise stated, the instruments and reagents used in the examples are commercially available. The reagents can be used directly without further purification.

MS: Thermo Fisher Q Exactive Plus, Water2795-Quattro micro triple quadrupole mass spectrometer

HPLC: Waters 2695, Agilent 1100, Agilent 1200

Semi-preparative HPLC: Lisure HP plus 50D

Flow cytometer: CytoFLEX S

HIC-HPLC: Butyl-HIC; mobile phase A: 25 mM PB, 2 M $(NH_4)_2SO_4$, pH 7.0; mobile phase B: 25 mM PB, pH 7.0; flow rate: 0.8 mL/min; acquisition time: 25 min; injection volume: 20 μg; column temperature: 25 °C; detection wavelength: 280 nm; sample box temperature: 8 °C.

SEC-HPLC: chromatographic column: TSK-gel G3000 SWXL, TOSOH 7.8 mm ID×300 mm, 5 μm; mobile phase: 0.2 M $KH_2PO_4$, 0.25 M KCl, pH 6.2; flow rate: 0.5 mL/min; acquisition time: 30 min; injection volume: 50 μg; column temperature: 25 °C; detection wavelength: 280 nm; sample tray temperature: 8 °C.

CHO cells were acquired from Thermo Fisher Scientific; pcDNA3.3 was acquired from Life Technology; HEK293F was acquired from Prejin; PEIMAX transfection reagent was acquired from Polyscience; MabSelect Sure ProA was acquired from GE; Capto S ImpAct was acquired from GE; Rink-amide-MBHA-resin and 2-chlorotrityl chloride resin were acquired from Nankai synthesis; HCC1954 was acquired from ATCC CAT# CRL-2338; SK-BR-3 was acquired from ATCC CAT# HTB-30; BT-474 was acquired from ATCC CAT# HTB-20; NCI-N87 (ATCC Cat# CRL-5822); MCF7 was acquired from ATCC CAT# HTB-22; MDA-MB-231 was acquired from ATCC CAT# HTB-26; MDA-MB-468 was acquired from ATCC CAT# HTB-132; CFPAC-1 was acquired from ATCC CAT# CRL-1918; NCI-H2110 was acquired from ATCC CAT# CRL-5924; JIMT-1 was acquired from Wuxi Apptech; Capan-1 was acquired from ATCC CAT# CRL-1573; the antibody trastuzumab was prepared according to the known sequence; optimized recombinant enzyme Sortase A derived from *Staphylococcus aureus* was prepared in *E. coli.*

Example 1: Construction of Expression Vector for Antibody, and Expression, Purification and Identification of Antibody

1.1 Production of modified anti-human HER2 antibody Ab0001-LCCT$_L$-HC

Expression plasmids for antibody Ab0001-LCCT$_L$-HC (light chain: SEQ ID NO: 1; heavy chain: SEQ ID NO: 2)

were constructed as follows. Sequence of antibody Ab0001-LCCT$_L$-HC: based on the amino acid sequence of trastuzumab, GALPETGG was introduced at the C terminus of the light chain, wherein LPETGG was a recognition sequence for ligase donor substrate, and GA was a spacer sequence. CHO cells were transfected with the plasmids. Cell populations were established and screened to obtain a high-expression cell population, which was cultured according to the culture process of trastuzumab in a 5-10 L reactor, and the supernatant was collected.

1.2 Purification of antibody Ab0001-LCCT$_L$-HC

Ab0001-LCCT$_L$-HC was purified using a standard process in which the MabSelect affinity chromatography and Sepharose S cation exchange chromatography were combined, and the purified product was dissolved in the drug buffer of the innovator drug, trastuzumab (5 mM histidine-HCl, 2% trehalose, 0.009% polysorbate 20, pH 6.0) and then frozen in small aliquots.

1.3 Quality control of antibody Ab0001-LCCT$_L$-HC

The purity of the antibody Ab0001-LCCT$_L$-HC purified by SDS-PAGE described above was 98.5%; SEC-HPLC detection showed that the content of the macromolecular polymer in the sample was less than 0.4%; the content of endotoxin was less than 0.098 EU/mg.

1.4 Preparation of other modified anti-human antibodies

According to a similar method, a terminal modification based on the ligase recognition sequence was introduced at the C-termini of the light chain and/or heavy chain of trastuzumab, thus obtaining modified antibodies.

Modified anti-human HER2 antibodies based on Ab0001 (trastuzumab) are listed in Table 1. LPETGG in the terminal modification sequence is a recognition sequence for ligase donor substrate, and GA is a spacer sequence.

Table 1. Modified anti-human HER2 antibodies

| | Sequence | Sequence introduced at terminus |
|---|---|---|
| Light chain of Ab0001-LCCT$_L$-HC | SEQ ID NO:1 | GALPETGG |
| Heavy chain of Ab0001-LCCT$_L$-HC | SEQ ID NO:2 | -* |
| Light chain of Ab0001-LCCT$_L$-HCCT$_L$ | SEQ ID NO:3 | GALPETGG |
| Heavy chain of Ab0001-LCCT$_L$-HCCT$_L$ | SEQ ID NO:4 | GALPETGG |
| *: "-" indicates no terminal modification | | |

Example 2: Preparation of Intermediates

2.1 Preparation of intermediate Mc-GGFG-Dxd(RF1184)

The intermediate Mc-GGFG-Dxd (RF1184) is commercially available or can be prepared according to the procedure described in EP2907824. This compound is used to prepare a linker-payload intermediate (a compound of formula (IV)) and also to directly link an (optionally modified) antibody to prepare a reference ADC.

2.2 Preparation of linker-payload intermediate LB301-2-1

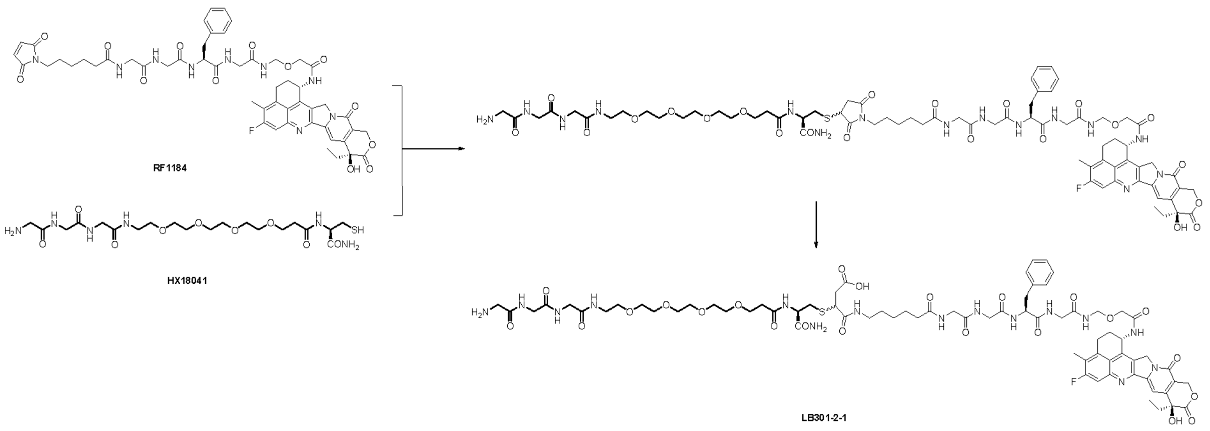

RF1184

HX18041

LB301-2-1

Step 1: Preparation of linker HX18041

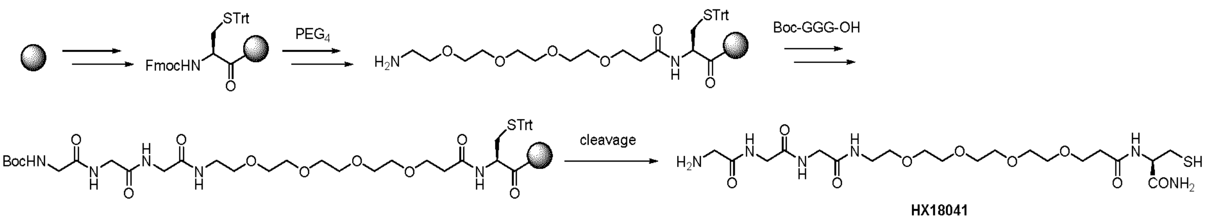


HX18041

HX18041 was synthesized by a conventional solid phase peptide synthesis method using the Rink-amide-MBHA-resin. Fmoc was used to protect the amino acids in the linking unit. The conjugation reagent was selected from HOBT, HOAt/DIC, DCC, EDCI, and HATU. After synthesis, the resin was cleaved using trifluoroacetic acid. The product was purified by HPLC, lyophilized, and stored for later use. Theoretical molecular weight: 538.24, found: $[M+H]^+$ = 539.2.

Step 2: Preparation of linker-payload intermediate LB301-2-1

HX18041 and the intermediate MC-GGFG-Dxd (molar ratio: ~1.2:1) were weighed out, each dissolved in water and DMF, and then fully mixed to obtain a mixture, followed by reacting at 0-40 °C for 0.5-20 h. After the reaction was completed, a proper amount of Tris base solution or another solution for promoting ring-opening reaction was added into the reaction mixture directly, and the resulting mixture was reacted at 0-40 °C for 0.2-20 h. After the reaction was completed, the product was purified by semi-preparative/preparative HPLC and lyophilized to obtain the linker-payload LB301-2-1. Theoretical mass: 1589.65, found: $[(M+2H)/2]^+$ = 796.6.

2.3 Preparation of linker-payload intermediate LB302-2-1

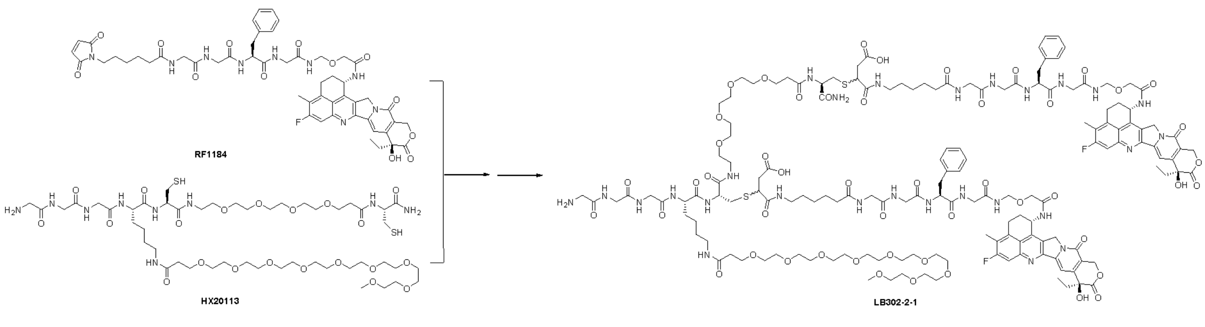

RF1184

HX20113

LB302-2-1

Step 1: Preparation of linker HX20113

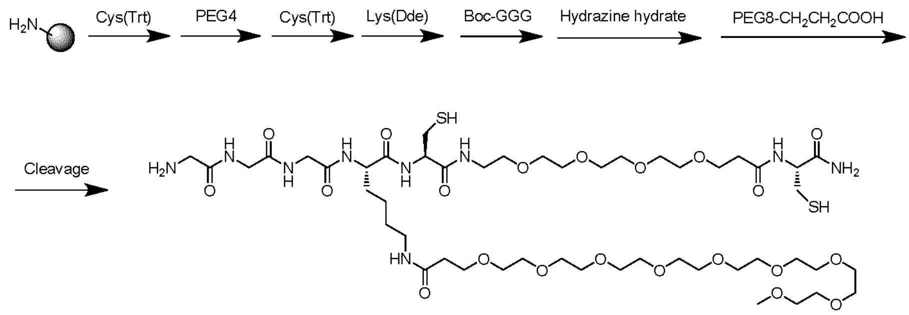


HX20113

HX20113 was synthesized by a conventional solid phase peptide synthesis method using the Rink-amide-MBHA-resin. Fmoc was used to protect the amino acids in the linking unit. The conjugation reagent was selected from HOBT, HOAt/DIC, DCC, EDCI, and HATU. After synthesis, the resin was cleaved using trifluoroacetic acid. The product was purified by HPLC, lyophilized, and stored for later use. Theoretical mass: 1207.59, found: $[M-H]^-$ = 1206.7.

Step 2: Preparation of linker-payload intermediate LB302-2-1

HX20113 and the intermediate MC-GGFG-Dxd (molar ratio: ~1:2) were weighed out, each dissolved in water and DMF, and then fully mixed to obtain a mixture, followed by reacting at 0-40 °C for 0.5-30 h. After the reaction was completed, a proper amount of Tris base solution or another solution for promoting ring-opening reaction was added into the reaction mixture directly, and the resulting mixture was reacted at 0-40 °C for 0.2-20 h. After the reaction was completed, the product was purified by semi-preparative/preparative HPLC and lyophilized to obtain the linker-payload intermediate LB302-2-1. Theoretical mass: 3310.41, found: $[(M+3H)/3]^+$ = 1104.5.

2.4 Preparation of linker-payload intermediate LB302-2-4

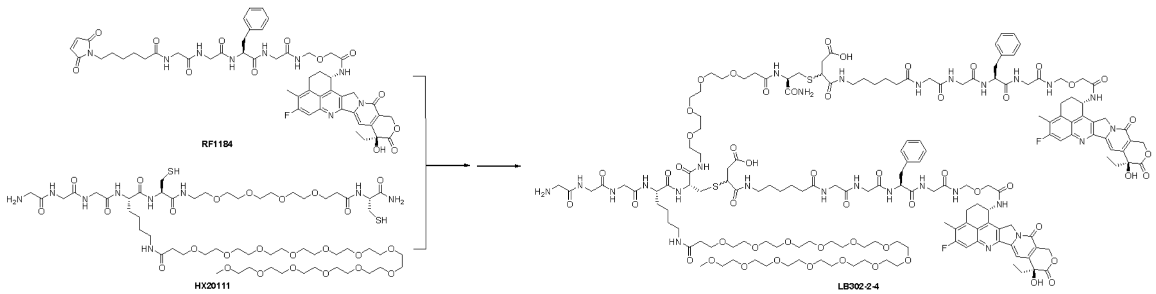


Step 1: Preparation of linker HX20111

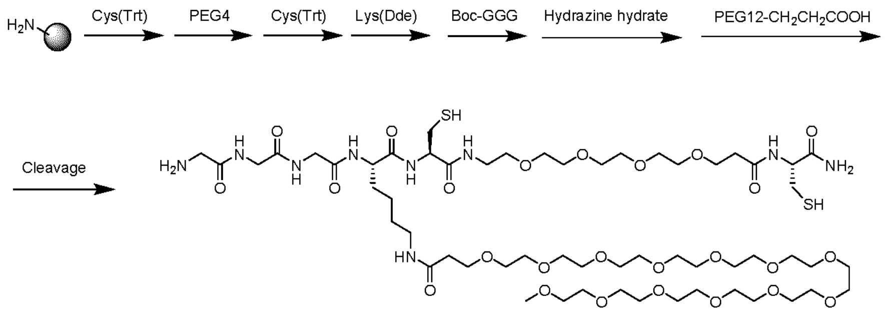


HX20111

HX20111 was synthesized by a conventional solid phase peptide synthesis method using the Rink-amide-MBHA-resin. Fmoc was used to protect the amino acids in the linking unit. The conjugation reagent was selected from HOBT, HOAt/DIC, DCC, EDCI, and HATU. After synthesis, the resin was cleaved using trifluoroacetic acid. The product was purified by HPLC, lyophilized, and stored for later use. Theoretical mass: 1383.70, found: $[M-H]^-$ = 1382.6.

Step 2: Preparation of linker-payload intermediate LB302-2-4

HX20111 and the intermediate MC-GGFG-Dxd (molar ratio: ~1:2) were weighed out, each dissolved in water and DMF, and then fully mixed to obtain a mixture, followed by reacting at 0-40 °C for 0.5-30 h. After the reaction was completed, a proper amount of Tris base solution or another solution for promoting ring-opening reaction was added into the reaction mixture directly, and the resulting mixture was reacted at 0-40 °C for 0.2-20 h. After the reaction was completed, the product was purified by semi-preparative/preparative HPLC and lyophilized to obtain the linker-payload intermediate LB302-2-4. Theoretical mass: 3486.52, found: $[(M+3H)/3]^+$ = 1163.3.

Example 3. Preparation of Antibody-Conjugated Drugs

3.1 The linker-payload intermediates were each site-specifically conjugated to the antibody by a ligase to form ADCs. Methods for conjugation reactions can be found in WO2015165413A1. The resulting ADCs are shown in Table 2 below:

Table 2. Generated ADCs

| Name of ADC | Linker-payload | Antibody |
|---|---|---|
| LC301-2-1(2) | LB301-2-1 | Ab0001-LCCT$_L$-HC |
| LC302-2-1(4) | LB302-2-1 | Ab0001-LCCT$_L$-HC |
| LC302-2-4(4) | LB302-2-4 | Ab0001-LCCT$_L$-HC |
| LC301-2-1(4) | LB301-2-1 | Ab0001-LCCT$_L$-HCCT$_L$ |

3.2 Reference ADCs LC1184(8) and LC1184(4) were prepared by directly linking the intermediate RF1184 to an (optionally modified) antibody (Cys conjugation, i.e., conjugation via a linkage formed by the maleimide structure and the sulfhydryl group of cysteine). Methods for conjugation reactions are known in the art. For LC1184(8), eight RF1184 were introduced via reduced inter-chain cysteine. For LC1184(4), four RF1184 were introduced via reduced inter-chain cysteine.

Example 4: Effect of HER2-Targeting Conjugates on Cell Proliferation

Cytotoxicity assays were performed using cancer cells SK-BR-3 (FIG. 1) and NCI-N87 (FIG. 2) with high expression of HER2, cancer cells CFPAC-1 (FIG. 3) and NCI-H2110 (FIG. 4) with low expression of HER2, and HER2-negative cell line MDA-MB-468 (FIG. 5) to analyze the effect of the conjugates on tumor cell proliferation. The test samples included conjugates LC302-2-1(4) and LC302-2-4(4) and small molecule compound 10 (the structure is as described above). Briefly, 3000-5000 cells were plated in a 96-well plate, and the cells were able to adhere after overnight growth. Cells were treated with the specified drugs at different concentrations for 120 h. The cell viability was examined by CellTiter-Glo® Luminescent Cell Viability Assay, and the percent cell viability was calculated.

In SK-BR-3 and NCI-N87 with high expression of HER2, conjugates LC302-2-1(4) and LC302-2-4(4) showed similar efficacy. The $IC_{50}$ values of LC302-2-1(4) and LC302-2-4(4) were lower than that of the small molecule payload

Dxd. In cells with low expression of HER2 and HER2-negative cells, conjugates LC302-2-1(4) and LC302-2-4(4) showed minimal efficacy. The specific results are shown in Table 3.

Table 3. Test results for effect of HER2-targeting conjugates on cell proliferation

| Sample | $IC_{50}$ (nM) | | | | |
|---|---|---|---|---|---|
| | SK-BR-3 | N87 | CFPAC-1 | H2110 | MDA-MB-468 |
| LC302-2-1(4) | 0.1926 | 0.6353 | N/A | N/A | N/A |
| LC302-2-4(4) | 0.1985 | 0.6353 | N/A | N/A | N/A |
| Compound 10 | 1.472 | 4.682 | - | - | - |
| N/A indicates a weak inhibitory effect, and - indicates not tested. | | | | | |

Example *5: In Vivo* Evaluation of Conjugates

For the *in vivo* anti-tumor efficacy study, SCID Beige mice were inoculated subcutaneously in the right flank with $5\times10^6$ JIMT-1 human breast cancer cells (HER2 medium). After 7 days, when the average tumor volume reached 142 $mm^3$, the tumor-bearing mice were grouped and intravenously given LC1184(8) and five other different conjugates at 5 mg/kg. Tumor volume was measured twice weekly with a caliper. LC1184(8) showed better efficacy than LC1184(4), indicating that higher DAR will result in better efficacy when the same payload is used. The efficacy of LC302-2-1(4) and LC302-2-4(4) was better than that of LC1184(8). LC301-2-1(4) showed comparable efficacy to LC1184(8), while LC301-2-1(2) showed comparable efficacy to LC1184(4) (FIG. 6). The conjugates of the present disclosure achieve good efficacy at a relatively low DAR.

BALB/c nude mice were inoculated subcutaneously in the right flank with $5\times10^6$ Capan-1 human pancreatic cancer cells (with low expression of HER2) to prepare a xenograft tumor model. After 8 days, when the tumor volume averaged 178 $mm^3$, the tumor-bearing mice were intravenously administrated LC1184(8) and five other different conjugates at 5 mg/kg. Tumor volume was measured twice weekly with a caliper. The efficacy of LC1184(8) was better than that of LC1184(4). LC302-2-1(4), LC302-2-4(4), and LC301-2-1(4) showed comparable efficacy to LC1184(8), while LC301-2-1(2) showed comparable efficacy to LC1184(4) (FIG. 7).

Example 6: *Ex Vivo* Serum Stability of Conjugates

For the *ex vivo* serum stability study, conjugates LC302-2-1(4), LC302-2-4(4), and LC1184(8) were each inoculated into mixed human serum at 37 °C. At 0 h, 24 h, 48 h, and 96 h, the conjugate was captured with an antigen, deglycosylated with glycosidase, and then dissociated with acid. The supernatant was collected and centrifuged, and then detected by high resolution LC-MS to determine the DAR.

LC302-2-1(4) and LC302-2-4(4) were very stable after 96 h of incubation in serum. After 96 h of incubation, there was no significant reduction in DAR for LC302-2-1(4) and LC302-2-4(4). However, the DAR of LC1184(8) decreased from 7.8 to 2.6 after 96 h of incubation (FIG. 8). The stability of the linker indicates that more payloads are delivered to the targeted tumor and the release of off-target free payload is reduced, thereby increasing the therapeutic index.

Example 7: Formulation Study

1. Detection method

1.1. Appearance and visible particle (visual method)

Appearance was determined by visual inspection. The illumination intensity of the clarity detector was ensured to be kept between 1000 lx and 1500 lx. The sample was kept at the same level as the eye, and gently shaken or inverted to prevent the generation of bubbles. Visual inspection was performed in front of a black background and a white background. The results were recorded in terms of the two aspects of appearance and visible particle.

1.2. Protein concentration (UV method)

The protein concentration was detected using a UV-visible spectrophotometer (dual wavelength method). The cuvette was washed with ultrapure water three times, then 400 μL of ultrapure water was added to the cuvette, and then the "measure" button was clicked; the calibration was performed by taking the ultrapure water as the blank control. After the

test sample was well mixed, the mixture was diluted with water for injection (not more than 10-fold dilution each time) to a final concentration of about 0.5 mg/mL. For each sample, three duplicate samples were required to be prepared and each well mixed for later use. The absorbance of the diluted sample at 280 nm and 360 nm (A280 and A360) was measured with a UV spectrophotometer. The measurements for the three duplicate samples were recorded as $A280_1$, $A360_1$, $A280_2$, $A360_2$, $A280_3$, and $A360_3$. A280 and A360 were calculated for each test sample according to the following formulas.

$$A280 = (A280_1 + A280_2 + A280_3)/3;\ A360 = (A360_1 + A360_2 + A360_3)/3$$

The $A_{280}$ and $A_{360}$ obtained by the above calculation were substituted into the following formula (1) and formula (2) to calculate $C_{mAb}$, and the $C_{mAb}$ was substituted into formula (3) to obtain the protein concentration C of the test sample;

$$A_{280} = \varepsilon_{mAb\text{-}280}*L*C_{mAb} + \varepsilon_{Linker\text{-}Drug\text{-}280}*L*C_{Linker\text{-}Drug} \quad (1)$$

$$A_{360} = \varepsilon_{Linker\text{-}Drug\text{-}360}*L*C_{linker\text{-}Drug} \quad (2)$$

$$C = C_{mAb}*N \quad (3)$$

where the extinction coefficient of the naked antibody at 280 nm was 1.47 $(g/L)^{-1}cm^{-1}$, the extinction coefficient of the Linker-Drug at 280 nm was 2.39 $(g/L)^{-1}cm^{-1}$, and the extinction coefficient at 360 nm was 8.73 $(g/L)^{-1}cm^{-1}$. N is the dilution factor.

1.3. Purity (SEC-HPLC)

Sample purity was detected using size-exclusion high-performance liquid chromatography (SEC-HPLC). The chromatographic column used was TSKgel G3000SWXL gel chromatographic column (7.8×300 mm); the mobile phase was 0.2 mol/L potassium dihydrogen phosphate-0.25 mol/L potassium chloride solution, and the pH was 6.2; the test sample was diluted with ultrapure water to about 2 mg/mL to prepare the test solution, and 50 μL of the test solution was injected into a high-performance liquid chromatograph. The column temperature was 25 °C, the flow rate was 0.5 mL/min, the detection wavelength was 280 nm, the injector temperature was 4 °C, and the analysis time was 30 min. The relative percentages of aggregate, monomer, and fragment components were calculated using the area normalization method.

1.4. Charge isomer (CEX-HPLC)

The charge heterogeneity of samples was detected by cation-exchange high-performance liquid chromatography (CEX-HPLC). The chromatographic column was a ProPac WCX-10 (4×250 mm) cation-exchange chromatographic column. The composition of mobile phase was as follows: A: 100% water, B: 200 mM NaCl, C: 100 mM sodium dihydrogen phosphate, D: 100 mM disodium hydrogen phosphate. The test sample was diluted with ultrapure water to about 2 mg/mL to prepare the test solution. 15 μL of the test solution was injected into the liquid chromatograph, and then the gradient elution was performed as shown in the table below. The column temperature was 45 °C, the flow rate was 0.8 mL/min, and the detection wavelength was 214 nm. The relative percentages of the acidic component, the main peak, and the basic component were calculated using the area normalization method.

1.5. Purity (reduced CE-SDS)

The sample purity was detected by using the reduced CE-SDS method. The test sample was diluted with a sample buffer solution (1% SDS-0.04 mmol/L sodium dihydrogen phosphate, pH 6.5) to a 1.0 mg/mL solution to prepare the test solution. 95 μL of the test solution and 5 μL of β-mercaptoethanol were well mixed by vortex mixing. The mixture was heated in a 70 °C water bath for 15 min, then cooled to room temperature, and then centrifuged for 5 min at 12000 rpm before sample loading. Separation was performed using an uncoated capillary (AB Sciex, capillary inner diameter: 50 μm, length: 30.2 cm, effective separation length: 20 cm). The sample injection voltage was -5 KV, the sample injection time was 20 s, the separation voltage was -15 KV, the analysis time was 35 min, the column temperature was 25 °C, and sample chamber temperature was 15 °C. The purities of LC, LC-Drug, and HC were calculated using the area normalization method, and the sum of the purities of the three was the sample purity.

1.6. DAR value (HIC)

The DAR value was detected by using hydrophobic interaction chromatography (HIC method). The chromatographic column used was MabPac HIC-20 (4.6 × 100 mm, 5 μm particle size). The composition of mobile phase A was 50 mM PB + 1 M sodium sulfate, pH 7.0±0.05; the composition of mobile phase B was 50 mM PB (phosphate buffer), pH 7.0 ±0.05. The test sample was diluted with pure water to about 5 mg/mL to prepare the test solution, and 10 μL of the test solution was injected into the liquid chromatograph. The chromatographic parameters were set as follows: column temperature 30 °C; sample chamber temperature 5±3 °C; flow rate 1.0 mL/min; injection volume 10 μL; detection wavelength 280 nm; elution time 30 min; the elution gradient is shown in the table below. The percentages of the DAR0, DAR2, and DAR4 peaks of samples were calculated by the area normalization method; DAR value = DAR2% × 2 + DAR4% × 4.

1.7. Free drug (RP-HPLC)

The free drug was detected by reverse-phase high-performance liquid chromatography (RP-HPLC). The chromatographic column used was Agilent Poroshell 120 EC-C18 (4.6 × 100 mm, 2.7 μm particle size). The mobile phase A was 0.1% TFA (trifluoroacetic acid) in water, and the mobile phase B was 0.1% TFA in acetonitrile. 100 μL of the test sample and 200 μL of acetone were well mixed. The resulting mixture was centrifuged at 13000 rpm for 5 min, and the supernatant was collected as the test solution. A proper amount of small molecule sample was prepared into 0.133-1.33 μg/mL reference solution. 7 μL of the test solution was injected into the liquid chromatograph, and the reference solution was analyzed using the same procedure. The flow rate was 1.0 mL/min, the column temperature was 40 °C, the detection wavelength was 261 nm, and the injector temperature was 4 °C. Gradient elution was performed as shown in the table below. The content of free small molecules in the sample was calculated using the external standard method.

1.8. Binding Activity (ELISA)

The binding activity was determined using enzyme-linked immunosorbent assay (ELISA). A microplate was coated with the antigen (Human Her2 protein), and the antigen was incubated overnight at 2-8 °C and then blocked with a blocking solution. The plate was washed with a washing solution, then a reference solution and test solutions were added separately, and the mixtures were each incubated at 25 °C for 1 h with shaking; the plate was washed with a washing solution, then a horseradish peroxidase-labeled mouse anti-human IgG-Fc antibody solution was added to the microplate, and the resulting mixtures were each incubated at 25 °C for 1 h with shaking; the plate was washed with a washing solution, and then TMB was added, followed by color development in dark for 10 min; the reaction was terminated with a stop solution, and then the absorbance at wavelength 450 nm was measured. $EC_{50}$ of the reference substance and the test sample was obtained by fitting with a four-parameter logistic regression (4PL) model. The relative activity of the test sample was calculated by using reference substance $EC_{50}$/test sample $EC_{50}$.

2. Content of study

2.1 Procedures

2.1.1 Preparation of displacement buffer

2 L of each of the respective displacement buffers were prepared according to Table 4. After the dialysis bag was soaked in the buffer, one end of the dialysis bag was sealed with a dialysis clamp. The LC302-2-4(4) sample (Lot: 20210409) was carefully transferred to the dialysis bag, and then the opening of the bag was clamped shut. The dialysis bag was then placed in a beaker containing the buffer and left to stand at room temperature for dialysis. After 4 h of dialysis, the dialysis bag was transferred to a fresh dialysis buffer and subjected to dialysis overnight at 2-8 °C, and the dialysis overnight at 2-8 °C was repeated twice. During each displacement of dialysis buffer, the ratio of the volume of the displaced liquid to that of the displacement buffer was controlled to be not higher than 1: 100. After dialysis was completed, the protein concentration was determined and diluted to a target concentration of 20 mg/mL. Note: Trehalose in the Examples was trehalose crystal (trehalose dihydrate).

Table 4. Formulation composition

| No. | Buffer system | pH | Auxiliary material | Surfactant | LC302-2-4(4) |
|---|---|---|---|---|---|
| FD-01 | 20 mM acetic acid buffer | 5.0 | 9% (w:v) sucrose | N/A | 20 mg/mL |

(continued)

| No. | Buffer system | pH | Auxiliary material | Surfactant | LC302-2-4(4) |
|---|---|---|---|---|---|
| FD-02 | 20 mM histidine buffer | 5.5 | 9% (w:v) sucrose | | |
| FD-03 | 20 mM histidine buffer | 6.0 | 9% (w:v) sucrose | | |
| FD-04 | 20 mM citric acid buffer | 6.5 | 9% (w:v) sucrose | | |
| FD-05 | 20 m phosphoric acid buffer | 7.0 | 9% (w:v) sucrose | | |
| FD-06 | 20 mM histidine buffer | 5.5 | 9% (w:v) trehalose | 0.2 mg/mL polysorbate 20 (PS20) | |
| FD-07 | 20 mM histidine buffer | 5.5 | 9% (w:v) sucrose | 0.2 mg/mL polysorbate 20 (PS20) | |
| FD-08 | 20 mM histidine buffer | 5.5 | 9% (w:v) sucrose | 0.2 mg/mL polysorbate 80 (PS80) | |

2.1.2 Preparation of 20 mg/mL polysorbate 20/80 stock solution

Before sterilization and filtration, a fresh polysorbate stock solution was prepared. 2% (20 mg/mL) polysorbate 20 or polysorbate 80 stock solution with a corresponding volume was added according to the design scheme of the formulation and the volume of the stock solution.

2.1.3 Sterilization, filtration, and filling

The solution was aseptically filled into 2R vials at 0.5 mL/vial on a super-clean bench, with 25 vials per group. The vials were plugged, capped, and labeled, and then stored and tested for stability.

2.2 Experimental results

**(1) Appearance and visible particles**

According to the results in Table 5, no obvious visible particles were observed at T0 for all the samples, all of which were colorless and clear liquids. For the formulation FD-01, a small amount of fine visible particles was generated after storage at -20 °C and 5 freeze-thaw cycles; for the formulation FD-04, a small amount of fine visible particles was generated after storage at -20 °C for 4 weeks; for the formulation FD-05, a small amount of fine visible particles was generated after storage at -20 °C and 5 freeze-thaw cycles or storage at a high temperature of 40 °C for 7 days; for the formulations FD-02, 03, 06, 07 and 08, no visible particles were generated under all storage conditions.

Table 5. Detection results for appearance and visible particles

| Sample No. | 2-8°C | | | -20°C | | 25°C | | 40 °C (7 days) | Shaking (7 days) | 5 freeze-thaw cycles (F/T5C) |
|---|---|---|---|---|---|---|---|---|---|---|
| | 0 weeks | 4 weeks | 12 weeks | 4 weeks | 12 weeks | 4 weeks | 12 weeks | | | |
| FD-01 | FVP | FVP | FVP | 1 | 1 | FVP | FVP | FVP | FVP | 1 |
| FD-02 | FVP | FVP | FVP | FVP | FVP | FVP | FVP | FVP | FVP | FVP |
| FD-03 | FVP | FVP | FVP | FVP | FVP | FVP | FVP | FVP | FVP | FVP |
| FD-04 | FVP | FVP | FVP | 1 | FVP | FVP | FVP | FVP | FVP | FVP |
| FD-05 | FVP | FVP | FVP | 1 | FVP | FVP | FVP | 1 | FVP | 1 |
| FD-06 | FVP | FVP | FVP | FVP | FVP | FVP | FVP | FVP | FVP | FVP |
| FD-07 | FVP | FVP | FVP | FVP | FVP | FVP | FVP | FVP | FVP | FVP |

(continued)

| Sample No. | 2-8°C | | | -20°C | | 25°C | | 40 °C (7 days) | Shaking (7 days) | 5 freeze-thaw cycles (F/T5C) |
|---|---|---|---|---|---|---|---|---|---|---|
| | 0 weeks | 4 weeks | 12 weeks | 4 weeks | 12 weeks | 4 weeks | 12 weeks | | | |
| FD-08 | FVP | FVP | FVP | FVP | FVP | FVP | FVP | FVP | FVP | FVP |
| Note: FVP: colorless, clear liquid without visible particle; 1: has fine particles generated | | | | | | | | | | |

**(2) SEC-HPLC results for purity**

According to the results of SEC aggregate content in Table 6, under accelerated and high temperature conditions, the formulations FD-04 and 05 showed a small increase in the ratio of the aggregates, and the formulations FD-01, 03, 06, 07 and 08 showed no significant increase in the ratio of the aggregates; after storage at 2-8 °C and -20 °C for 12 weeks, no formulations showed significant increase in aggregates.

Table 6. SEC-HPLC detection results for aggregate content (%)

| Sample No. | 2-8°C | | | -20°C | | 25°C | | 40°C | Shaking | 5 freeze-thaw cycles |
|---|---|---|---|---|---|---|---|---|---|---|
| | 0 weeks | 4 weeks | 12 weeks | 4 weeks | 12 weeks | 4 weeks | 12 weeks | 7 days | 7 days | |
| FD-01 | 4.83 | 5.02 | 5.05 | 5.00 | 5.07 | 5.07 | 5.58 | 5.18 | 4.93 | 4.89 |
| FD-02 | 4.92 | 5.10 | 5.11 | 5.04 | 5.12 | 5.06 | 5.41 | 5.17 | 4.97 | 4.93 |
| FD-03 | 4.99 | 5.08 | 5.17 | 5.07 | 5.18 | 5.14 | 5.49 | 5.25 | 5.05 | 4.99 |
| FD-04 | 5.20 | 5.40 | 5.44 | 5.38 | 5.45 | 5.61 | 6.13 | 5.88 | 5.37 | 5.42 |
| FD-05 | 5.68 | 5.87 | 5.99 | 5.76 | 5.87 | 6.32 | 7.67 | 7.08 | 5.87 | 5.97 |
| FD-06 | 4.95 | 5.08 | 5.10 | 5.05 | 5.12 | 5.18 | 5.47 | 5.13 | 5.00 | 4.94 |
| FD-07 | 4.91 | 5.02 | 5.08 | 5.00 | 5.11 | 4.85 | 5.38 | 5.14 | 4.94 | 4.93 |
| FD-08 | 4.92 | 5.03 | 5.11 | 5.00 | 5.12 | 5.18 | 5.39 | 5.12 | 4.97 | 4.92 |

**(3) Charge isomer (CEX-HPLC)**

According to the CEX-HPLC purity results in Tables 7-9, after storage under accelerated and high temperature conditions, the proportions of acidic component of the formulations FD-03, 04, and 05 were increased to different degrees, and the the proportions of acidic component of the formulations FD-01, 02 and FD-06, 07 and 08 were not significantly changed. The proportions of basic component of the formulations FD-01, 02 and 03 and formulations FD-06, 07 and 08 all increased significantly, the proportion of basic component of the formulation FD-04 was not significantly changed, and the proportion of basic component of the formulation FD-05 was slightly reduced. All samples showed decrease in the main peak purity, with the five formulations FD-01, 02, 06, 07 and 08 showing a slower decrease in main peak purity and the formulation FD-05 showing a relatively larger decrease. After storage at 2-8 °C and -20 °C for 12 weeks, no sample showed significant decrease in purity.

Table 7. CEX-HPLC detection results for acidic peak (%)

| Sample No. | 2-8°C | | | -20°C | | 25°C | | 40°C | Shaking | 5 freeze-thaw cycles |
|---|---|---|---|---|---|---|---|---|---|---|
| | 0 weeks | 4 weeks | 12 weeks | 4 weeks | 12 weeks | 4 weeks | 12 weeks | 7 days | 7 days | |
| FD-01 | 29.35 | 29.09 | 28.84 | 29.49 | 30.01 | 27.19 | 32.32 | 27.02 | 29.17 | 29.20 |
| FD-02 | 31.55 | 31.65 | 31.37 | 31.87 | 32.16 | 29.15 | 29.49 | 27.36 | 31.62 | 31.69 |
| FD-03 | 36.00 | 36.68 | 37.29 | 36.21 | 37.03 | 37.73 | 42.67 | 35.74 | 36.99 | 36.72 |

(continued)

| Sample No. | 2-8°C | | | -20°C | | 25°C | | 40°C | Shaking | 5 freeze-thaw cycles |
|---|---|---|---|---|---|---|---|---|---|---|
| | 0 weeks | 4 weeks | 12 weeks | 4 weeks | 12 weeks | 4 weeks | 12 weeks | 7 days | 7 days | |
| FD-04 | 40.34 | 41.62 | 41.64 | 39.68 | 40.17 | 44.20 | 48.92 | 47.95 | 41.70 | 42.19 |
| FD-05 | 55.19 | 56.60 | 59.41 | 49.58 | 51.90 | 66.51 | 74.58 | 71.19 | 57.69 | 56.95 |
| FD-06 | 32.37 | 32.21 | 32.10 | 32.18 | 33.18 | 30.02 | 30.82 | 28.97 | 32.45 | 32.51 |
| FD-07 | 32.40 | 32.26 | 31.81 | 32.25 | 32.68 | 29.63 | 30.14 | 28.18 | 32.25 | 32.38 |
| FD-08 | 32.25 | 32.12 | 32.20 | 32.38 | 32.92 | 29.64 | 30.41 | 28.46 | 32.30 | 32.39 |

Table 8. CEX-HPLC detection results for main peak (%)

| Sample No. | 2-8°C | | | -20°C | | 25°C | | 40°C | Shaking | 5 freeze-thaw cycles |
|---|---|---|---|---|---|---|---|---|---|---|
| | 0 weeks | 4 weeks | 12 weeks | 4 weeks | 12 weeks | 4 weeks | 12 weeks | 7 days | 7 days | |
| FD-01 | 36.99 | 36.71 | 34.48 | 37.57 | 36.78 | 29.53 | 21.63 | 26.01 | 35.84 | 35.77 |
| FD-02 | 35.11 | 34.84 | 34.27 | 35.17 | 35.21 | 29.38 | 22.37 | 26.30 | 34.46 | 34.42 |
| FD-03 | 31.48 | 31.22 | 29.97 | 31.75 | 31.03 | 26.11 | 19.45 | 24.15 | 30.38 | 30.53 |
| FD-04 | 27.68 | 27.05 | 26.94 | 28.85 | 28.60 | 22.77 | 17.57 | 19.92 | 26.45 | 26.02 |
| FD-05 | 17.74 | 17.18 | 15.67 | 21.90 | 20.48 | 12.51 | 7.91 | 10.24 | 16.52 | 16.89 |
| FD-06 | 34.64 | 34.50 | 34.12 | 35.27 | 34.65 | 29.45 | 22.41 | 26.29 | 33.82 | 33.78 |
| FD-07 | 34.74 | 34.43 | 34.19 | 35.26 | 35.02 | 29.43 | 22.55 | 26.15 | 34.08 | 33.82 |
| FD-08 | 34.72 | 34.49 | 33.82 | 34.99 | 34.70 | 29.41 | 22.23 | 26.04 | 34.19 | 33.94 |

Table 9. CEX-HPLC detection results for basic peak (%)

| Sample No. | 2-8°C | | | -20°C | | 25°C | | 40°C | Shaking | 5 freeze-thaw cycles |
|---|---|---|---|---|---|---|---|---|---|---|
| | 0 weeks | 4 weeks | 12 weeks | 4 weeks | 12 weeks | 4 weeks | 12 weeks | 7 days | 7 days | |
| FD-01 | 33.67 | 34.20 | 36.68 | 32.94 | 33.21 | 43.29 | 46.06 | 46.97 | 34.99 | 35.04 |
| FD-02 | 33.34 | 33.50 | 34.36 | 32.96 | 32.64 | 41.47 | 48.13 | 46.34 | 33.93 | 33.89 |
| FD-03 | 32.52 | 32.10 | 32.75 | 32.04 | 31.94 | 36.16 | 37.88 | 40.11 | 32.63 | 32.75 |
| FD-04 | 31.97 | 31.34 | 31.42 | 31.47 | 31.23 | 33.03 | 33.51 | 32.13 | 31.85 | 31.79 |
| FD-05 | 27.08 | 26.22 | 24.92 | 28.52 | 27.62 | 20.98 | 17.51 | 18.57 | 25.79 | 26.17 |
| FD-06 | 32.99 | 33.29 | 33.78 | 32.55 | 32.17 | 40.53 | 46.77 | 44.74 | 33.73 | 33.71 |
| FD-07 | 32.86 | 33.30 | 34.00 | 32.49 | 32.30 | 40.95 | 47.31 | 45.67 | 33.67 | 33.80 |
| FD-08 | 33.03 | 33.39 | 33.98 | 32.63 | 32.38 | 40.95 | 47.36 | 45.50 | 33.51 | 33.67 |

**(4) Reduced CE-SDS purity**

According to the results of reduced CE-SDS purity in Table 10, the purity of formulations FD-04 and 05 was reduced under accelerated and high temperature conditions, and the purity of formulation FD-08 was also reduced to some extent after storage for 7 days at high temperature, and the difference between the other groups was small. For all the formulations, after storage for 12 weeks at 2-8 °C and -20 °C, the purity of the samples did not change significantly.

Table 10. Reduced CE-SDS detection results for purity (%)

| Sample No. | T0 | 40°C | 2-8°C | -20°C | 25°C |
|---|---|---|---|---|---|
| | | 7 days | 12 weeks | 12 weeks | 12 weeks |
| FD-01 | 97.37 | 96.83 | 97.45 | 97.54 | 97.04 |
| FD-02 | 97.63 | 96.98 | 97.56 | 97.71 | 97.05 |
| FD-03 | 97.25 | 96.80 | 97.42 | 97.59 | 96.92 |
| FD-04 | 96.88 | 95.35 | 97.35 | 97.43 | 96.08 |
| FD-05 | 97.02 | 94.37 | 97.36 | 96.88 | 94.42 |
| FD-06 | 97.26 | 96.97 | 97.49 | 97.44 | 96.74 |
| FD-07 | 97.31 | 96.70 | 97.60 | 97.51 | 96.79 |
| FD-08 | 97.43 | 96.15 | 97.47 | 97.64 | 97.07 |

**(5) DAR value**

According to Table 11, the DAR values of different formulations did not change significantly under different examination conditions and time points.

Table 11. Detection results for DAR values

| Sample No. | 2-8°C (W) | | | -20°C(W) | | 25°C (W) | | 40°C (D) |
|---|---|---|---|---|---|---|---|---|
| | 0 | 4 | 12 | 4 | 12 | 4 | 12 | 7 |
| FD-01 | 3.2 | 3.2 | 3.1 | 3.2 | 3.1 | 3.2 | 3.2 | 3.2 |
| FD-02 | 3.2 | 3.2 | 3.1 | 3.2 | 3.1 | 3.2 | 3.2 | 3.2 |
| FD-03 | 3.2 | 3.2 | 3.2 | 3.2 | 3.2 | 3.2 | 3.2 | 3.2 |
| FD-04 | 3.2 | 3.2 | 3.2 | 3.2 | 3.2 | 3.2 | 3.2 | 3.2 |
| FD-05 | 3.4 | 3.4 | 3.2 | 3.2 | 3.2 | 3.3 | 3.3 | 3.3 |
| FD-06 | 3.2 | 3.2 | 3.2 | 3.2 | 3.2 | 3.2 | 3.2 | 3.3 |
| FD-07 | 3.2 | 3.2 | 3.1 | 3.2 | 3.2 | 3.2 | 3.2 | 3.2 |
| FD-08 | 3.2 | 3.2 | 3.2 | 3.2 | 3.2 | 3.2 | 3.2 | 3.2 |

(6) **Free drug**

Tables 12 and 13 show the detection results for the free drugs under 8 different examination conditions and time points. The results showed that the shedding of Dxd was promoted when the pH of formulation was relatively low (e.g., 5.5 and below). When the pH was relatively high (e.g., 6.5-7.0), the shedding of LB302-2-4 was accelerated. Under the conditions of 2-8 °C and -20 °C and moderate pH (such as 5.5), the shedding of free drugs was not remarkable whether a surfactant was added or not, such that strict quality control requirements were satisfied.

Table 12. Detection results for free drug (LB302-2-4)

| Free drug (LB302-2-2) (ppm) | | | | | | | | |
|---|---|---|---|---|---|---|---|---|
| Sample No. | 2-8°C (W) | | | -20°C (W) | | 25°C (W) | | 40°C (D) |
| | 0 | 4 | 12 | 4 | 12 | 4 | 12 | 7 |
| FD-01 | <20 | <20 | <20 | <20 | <20 | 60 | 83 | 61 |
| FD-02 | <20 | 30 | 22 | 20 | <20 | 68 | 90 | 77 |
| FD-03 | <20 | 41 | 28 | <20 | <20 | 68 | 115 | 73 |
| FD-04 | <20 | 54 | 58 | <20 | <20 | 82 | 130 | 131 |

(continued)

| Free drug (LB302-2-2) (ppm) | | | | | | | | |
|---|---|---|---|---|---|---|---|---|
| Sample No. | 2-8°C (W) | | | -20°C (W) | | 25°C (W) | | 40°C (D) |
| | 0 | 4 | 12 | 4 | 12 | 4 | 12 | 7 |
| FD-05 | 27 | 67 | 45 | 38 | 26 | 181 | 222 | 185 |
| FD-06 | 47 | 98 | 92 | 58 | 65 | 160 | 239 | 170 |
| FD-07 | 49 | 99 | 101 | 105 | 69 | 169 | 250 | 202 |
| FD-08 | 46 | 97 | 97 | 70 | 62 | 163 | 232 | 184 |

Table 13. Detection results for free drug (Dxd)

| Sample No. | 2-8°C (W) | | | -20°C (W) | | 25°C (W) | | 40°C (D) |
|---|---|---|---|---|---|---|---|---|
| | 0 | 4 | 12 | 4 | 12 | 4 | 12 | 7 |
| FD-01 | <20 | <20 | <20 | <20 | <20 | <20 | 42 | 36 |
| FD-02 | <20 | <20 | <20 | <20 | <20 | <20 | 41 | 37 |
| FD-03 | <20 | <20 | <20 | <20 | <20 | <20 | 23 | <20 |
| FD-04 | <20 | <20 | <20 | <20 | <20 | <20 | <20 | <20 |
| FD-05 | <20 | <20 | <20 | <20 | <20 | <20 | 25 | <20 |
| FD-06 | <20 | <20 | <20 | <20 | <20 | <20 | 41 | 36 |
| FD-07 | <20 | <20 | <20 | <20 | <20 | <20 | 43 | 39 |
| FD-08 | <20 | <20 | <20 | <20 | <20 | <20 | 41 | 39 |

**(7) Binding activity (ELISA)**

According to the binding activity results in Table 14, no formulations showed reduction in activity after storage for 7 days at high temperature and under accelerated and long term conditions for 12 weeks.

Table 14. Detection results for binding activity

| Binding Activity (%) | | | | | |
|---|---|---|---|---|---|
| Sample No. | T0 | 40°C | 2-8°C | -20°C | 25°C |
| | | 7d | 12W | 12W | 12W |
| FD-01 | 111 | 115 | 104 | 107 | 119 |
| FD-02 | 103 | 117 | 104 | 106 | 105 |
| FD-03 | 131 | 113 | 103 | 104 | 121 |
| FD-04 | 114 | 122 | 113 | 114 | 129 |
| FD-05 | 106 | 118 | 113 | 119 | 115 |
| FD-06 | 104 | 115 | 105 | 100 | 99 |
| FD-07 | 115 | 117 | 108 | 99 | 114 |
| FD-08 | 114 | 118 | 109 | 105 | 109 |

3. Conclusion

According to the detection results for the visible particles, all the formulations have certain stability, wherein the formulations FD-02, 03, 06, 07 and 08 have better stability. Based on the SEC aggregate detection results and the combined analysis of other assay results (e.g., free drug data), better stability can be achieved when the buffer system is an acetic acid buffer system and a histidine buffer system, with the histidine buffer system being more preferred. From the

CEX results for acid peaks and basic peaks, the main peak purity of the five formulations of FD-01, 02, 06, 07 and 08 is better maintained. From the detection results for free drug, a low pH condition accelerates the shedding of Dxd, and the high pH will cause the shedding of LB302-2-4. However, under a moderate pH (e.g., around 5.5), the shedding of the free drug is inhibited even without the addition of a surfactant.

**Sequence Listing**

SEQ ID No. 1: light chain of Ab0001-LCCT$_L$-HC:

DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGECGALPETGG

SEQ ID No. 2: heavy chain of Ab0001-LCCT$_L$-HC:

EVQLVESGGGLVQPGGSLRLSCAASGFNIKDTYIHWVRQAPGKGLEWVARIYPTNGYTRYAD
SVKGRFTISADTSKNTAYLQMNSLRAEDTAVYYCSRWGGDGFYAMDYWGQGTLVTVSSAS
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK

PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEA
LHNHYTQKSLSLSPGK

SEQ ID No. 3: light chain of Ab0001-LCCT$_L$-HCCT$_L$:

DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHK
VYACEVTHQGLSSPVTKSFNRGECGALPETGG

SEQ ID No. 4: heavy chain of Ab0001-LCCT$_L$-HCCT$_L$:

EVQLVESGGGLVQPGGSLRLSCAASGFNIKDTYIHWVRQAPGKGLEWVARIYPTNGYTRYAD
SVKGRFTISADTSKNTAYLQMNSLRAEDTAVYYCSRWGGDGFYAMDYWGQGTLVTVSSAS
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEA
LHNHYTQKSLSLSPGKGALPETGG

## Claims

1. A pharmaceutical composition, comprising an active ingredient, a buffering agent, a stabilizer, and optionally a surfactant, wherein,

the active ingredient comprises a conjugate having the structure of formula (III):

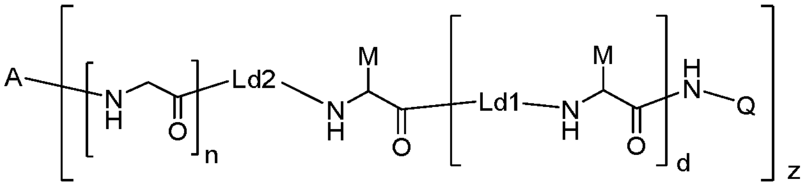 formula (III),

wherein,
n is any integer selected from 2, 3, 4, 5, 6, 7, 8, 9, and 10;
d is 0 or any integer selected from 1, 2, 3, 4, 5, and 6;
each Ld1 and Ld2 is independently a bond, or is selected from -NH-$C_{1-20}$ alkylene-(CO)- and NH-$(PEG)_i$-(CO)-; or is a natural amino acid or oligomeric natural amino acids with a degree of polymerization of 2-10 each independently unsubstituted or substituted with -$(PEG)_j$-$R^{11}$ on the side chain; or is a natural amino acid or oligomeric natural amino acids with a degree of polymerization of 2-10 substituted with -$(PEG)_j$-$R^{11}$ on the side chain, wherein the -$(PEG)_j$-$R^{11}$ has carbonyl linked therebetween;
the -$(PEG)_i$- and -$(PEG)_j$- are each a PEG fragment comprising a specified number of consecutive -(O-$C_2H_4$)- structural units or consecutive -($C_2H_4$-O)- structural units, optionally with additional $C_{1-10}$ alkylene at one terminal;
Q is hydrogen or LKb-P;
M is hydrogen or LKa-LKb-P, provided that Q and M are not both hydrogen;
each LKa is independently selected from

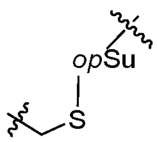, 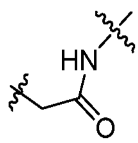, and 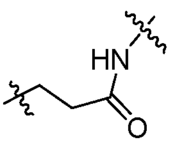;

*opSu* is

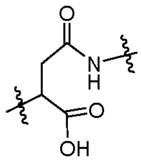, 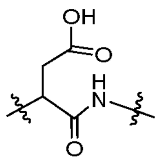,

or a mixture thereof;
each LKb is independently selected from $L^2$-$L^1$-B;
each B is independently absent or is a combination of 1) and 2) below: 1) a self-immolative spacer Sp1; and 2) a bond, or one divalent group, or a combination of two or more divalent groups, wherein the divalent group is selected from: -$CR^1R^2$-, $C_{1-10}$ alkylene, and -(CO)-; or
each B is independently a terminal group $R^{10}$ or a combination of 1), 2), and 3) below: 1) a self-immolative spacer Sp1; 2) a bond, or one divalent group, or a combination of two or more divalent groups, wherein the divalent group is selected from: -$CR^1R^2$-, $C_{1-10}$ alkylene, $C_{4-10}$ cycloalkylene, $C_{4-10}$ heterocyclylene, and -(CO)-; and 3) a terminal group $R^{10}$; $R^{10}$ is hydrogen or a group that can leave when reacting with a group in a payload;
Sp1 is selected from PABC, acetal, heteroacetal, and combinations thereof; preferably, Sp1 is acetal, heteroacetal, or PABC; preferably, the heteroacetal is selected from N,O-heteroacetal; preferably, Sp1 is -O-$CH_2$-U- or -NH-$CH_2$-U-, wherein -O- or -NH- is linked to a cleavable sequence 1, and U is O, S or NH, preferably O or S; preferably, B is -NH-$CH_2$-U-, or is absent, or is -NH-$CH_2$-U-$(CH_2)_g$-(CO)-, wherein U is absent or is O, S or NH, preferably O or S;
$L^1$ is the cleavable sequence 1 comprising an amino acid sequence capable of being cleaved by an enzyme, and

the cleavable sequence 1 comprises 1-10 amino acids; preferably, the cleavable sequence 1 is selected from GLy-GLy-Phe-GLy, Phe-Lys, Val-Cit, Val-Lys, GLy-Phe-Leu-Gly, Ala-Leu-Ala-Leu, Ala-Ala-Ala, and combinations thereof;

$L^2$ is a bond or $C_{2-20}$ alkylene, wherein one or more $-CH_2-$ structures in the alkylene are optionally replaced by the following groups: $-CR^3R^4-$, -O-, -(CO)-, $-S(=O)_2-$, $-NR^5-$, $-N\oplus R^6R^7-$, $C_{4-10}$ cycloalkylene, $C_{4-10}$ heterocyclylene, and phenylene, wherein the cycloalkylene, heterocyclylene, and phenylene are each independently unsubstituted or substituted with at least one substituent selected from halogen, $-C_{1-10}$ alkyl, $-C_{1-10}$ haloalkyl, $-C_{1-10}$ alkylene-NH-$R^8$, and $-C_{1-10}$ alkylene-O-$R^9$;

$R^1$, $R^2$, $R^3$, $R^4$, $R^5$, $R^6$, $R^7$, $R^8$, and $R^9$ are each independently selected from hydrogen, halogen, $-C_{1-10}$ alkyl, $-C_{1-10}$ haloalkyl, and $C_{4-10}$ cycloalkylene;

$R^{11}$ is $C_{1-10}$ alkyl;

each i is independently an integer from 1 to 100, preferably from 1 to 20; preferably, each i is independently an integer from 1 to 12, more preferably from 2 to 8, particularly 4;

each j is independently an integer from 1 to 100, preferably from 1 to 20; preferably, each j is independently an integer from 1 to 12, more preferably from 8 to 12 or from 8 to 13, particularly 8, 9, 12, or 13;

P is the payload linked to the B moiety or the $L^1$ moiety, and the payload is selected from compounds 1-14 below,

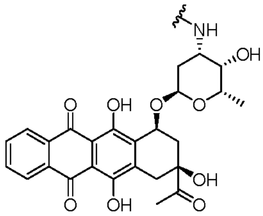

1
(Idarubicin)

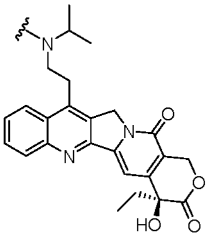

2
(Belotecan)

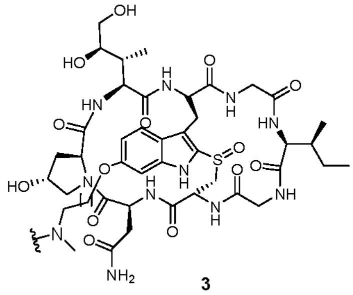

3
(Amanitin)

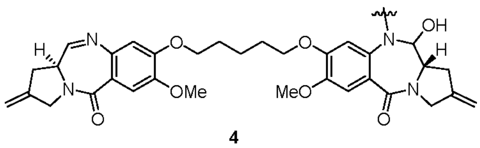

4
(PBD dimer)

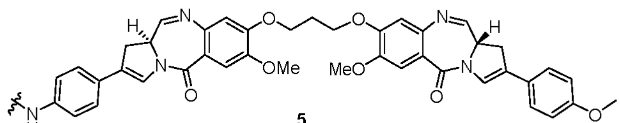

5
(PBD dimer)

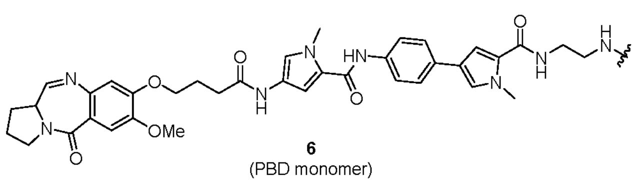

6
(PBD monomer)

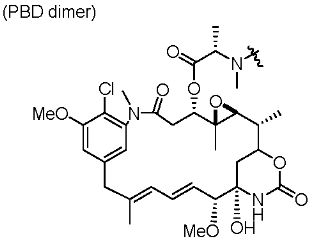

7
(DM)

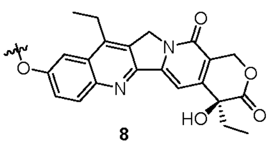

8
(SN-38)

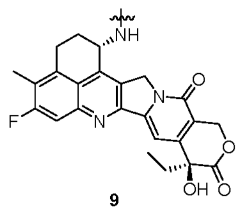

9
(DX8951f)

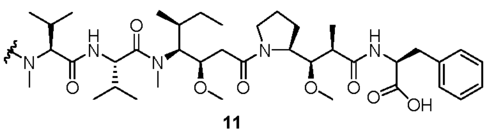

11
(MMAF)

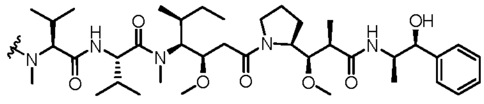

12
(MMAE)

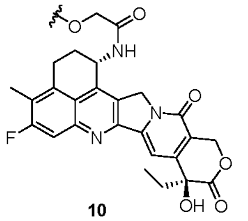

10
(Dxd-(1))

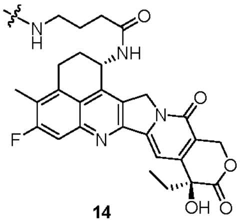

14
(Dxd-(2))

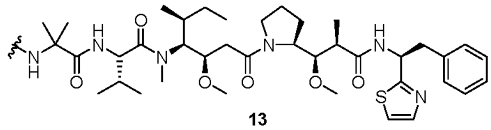

13
(Auristatin 0101)

;

A is a targeting molecule, and the targeting molecule is an antibody or an antigen-binding fragment thereof; preferably, the antibody or the antigen-binding fragment comprises a recognition sequence for ligase donor and is covalently conjugated by a ligase to $(Gly)_n$ in the formula (III); preferably, the antibody or the antigen-binding fragment is covalently conjugated to a -Gly-Gly-Gly- moiety; preferably, heavy and/or light chain termini of the antibody or the antigen-binding fragment are linked to a recognition sequence for ligase donor substrate; preferably, the antibody is an anti-human HER2 antibody; preferably, the C-terminus of a light chain of the anti-HER2 antibody is linked to an amino acid sequence GALPETGG, or the C-termini of both the light chain and heavy chain of the anti-HER2 antibody are linked to the amino acid sequence GALPETGG;
z is an integer from 1 to 20.

**2.** The pharmaceutical composition according to claim 1, wherein, the conjugate has the structure of formula (III-1) below:

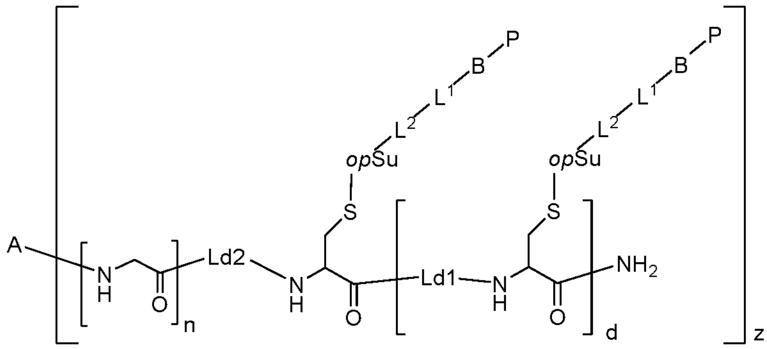
formula (III-1).

**3.** The pharmaceutical composition according to claim 1 or 2, wherein the conjugate has the following structure:

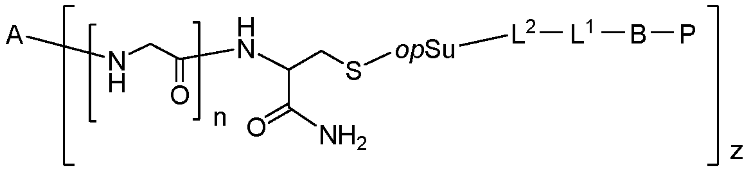
(conjugate LC301-1)

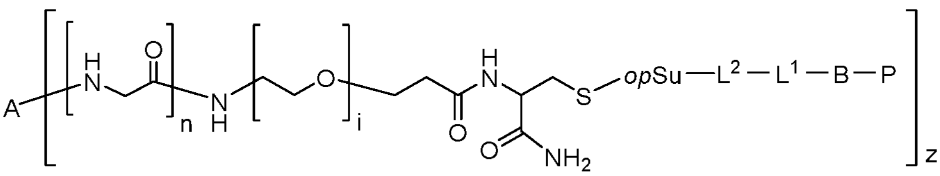

(conjugate LC301-2),

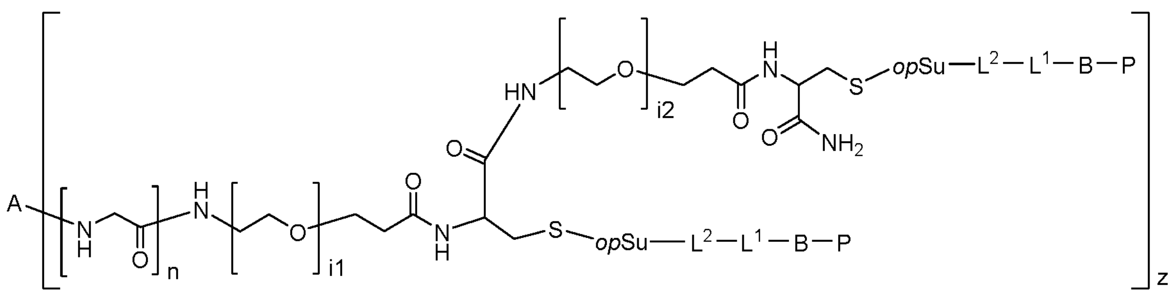

(conjugate LC301-3),

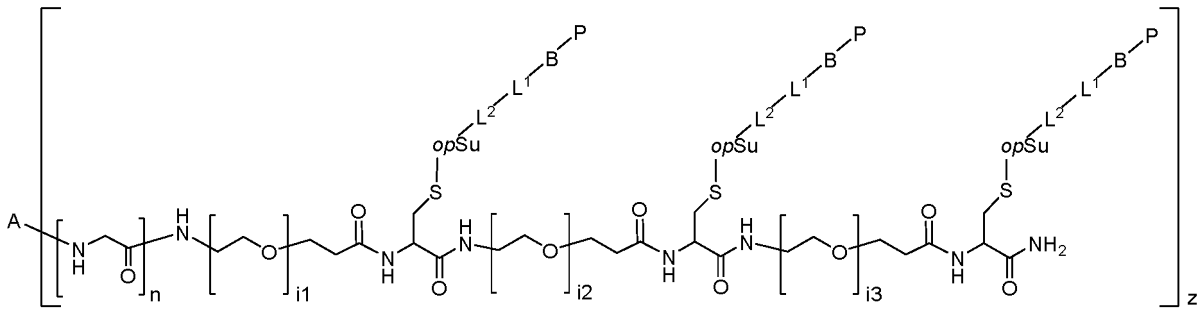

(conjugate LC301-4),

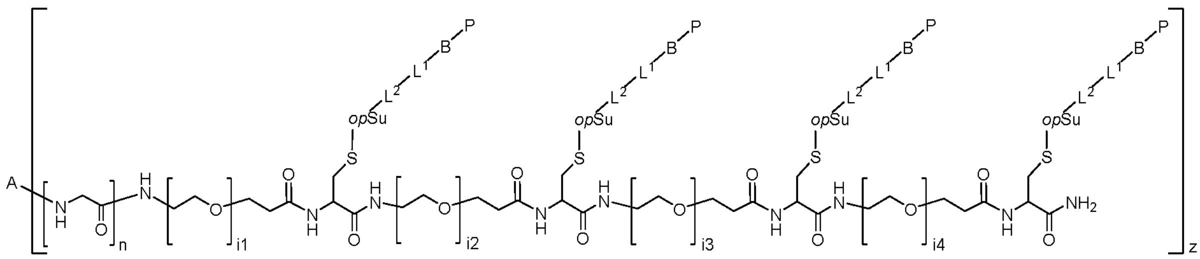

(conjugate LC301-5),

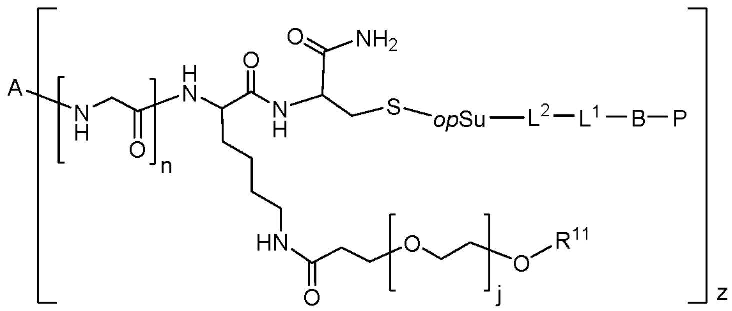

(conjugate LC302-1),

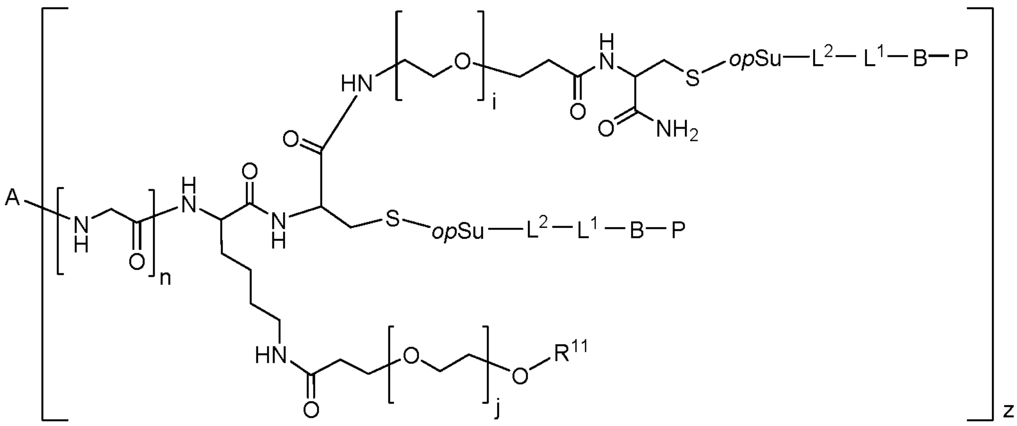

(conjugate LC302-2),

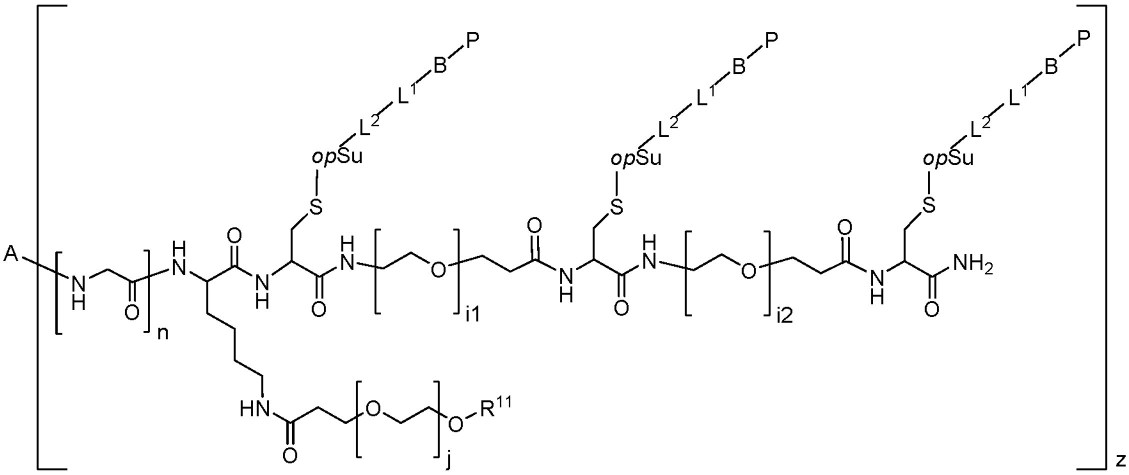

(conjugate LC302-3),

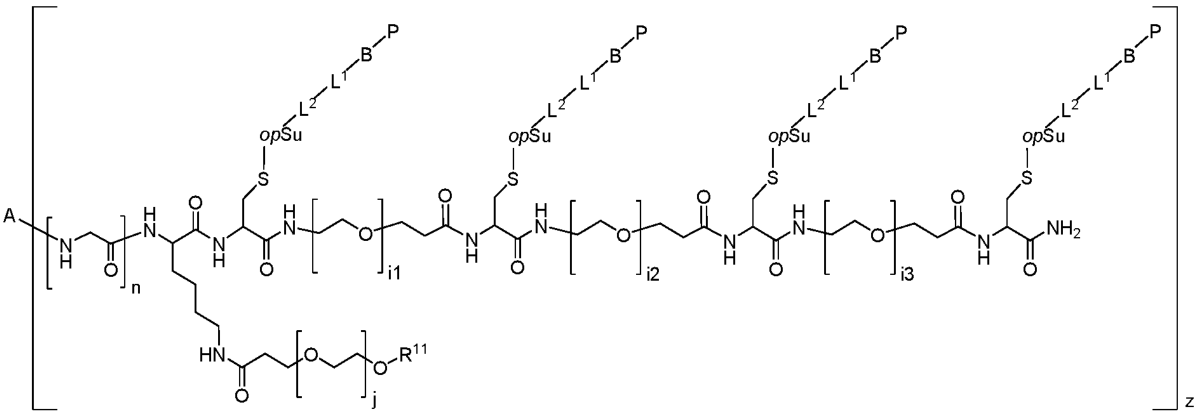

(conjugate LC302-4);

preferably, z is 1-4, preferably 2;
each i, i1, i2, i3, and i4 is independently an integer from 1 to 100, preferably from 1 to 20; preferably, each i, i1, i2, i3, and i4 is independently an integer from 1 to 12, more preferably from 2 to 8, particularly 4;
each j is independently an integer from 1 to 100, preferably from 1 to 20; preferably, each j is independently an integer from 1 to 12, more preferably from 8 to 12 or from 8 to 13, particularly 8, 9, 12, or 13;
preferably, n is 3, $L^2$ is $-(CH_2)_p-(CH_2)_2(CO)-$, p is 3, $L^1$ is GGFG, B is $-NH-CH_2-U-$, or is absent, or is $-NH-CH_2-U-(CH_2)_g-(CO)-$, U is O, and g is 1.

**4.** The pharmaceutical composition according to any one of claims 1-3, wherein the payload is selected from:

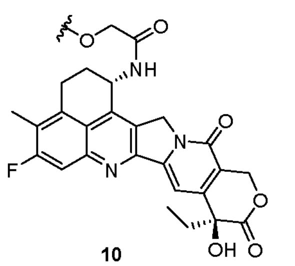

**10**
(Dxd-(1))

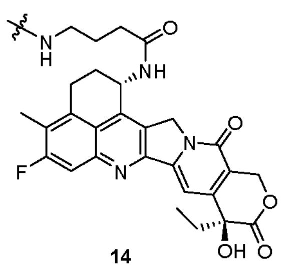

**14**
(Dxd-(2))

.

**5.** The pharmaceutical composition according to any one of claims 1-4, wherein the conjugate is selected from:

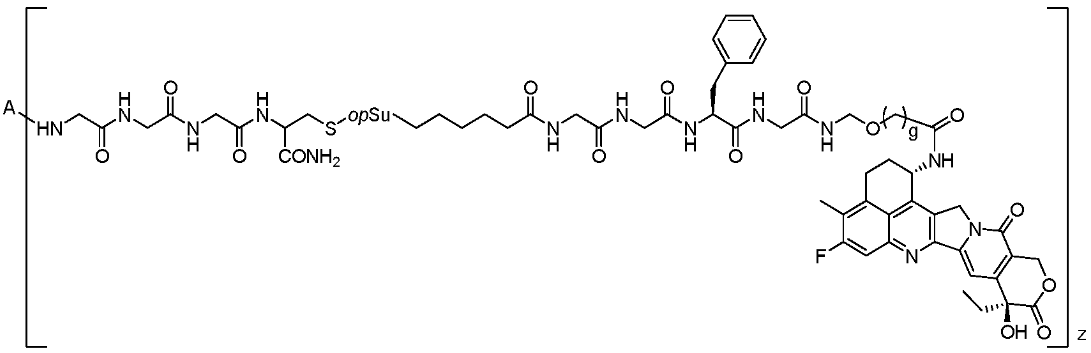

,

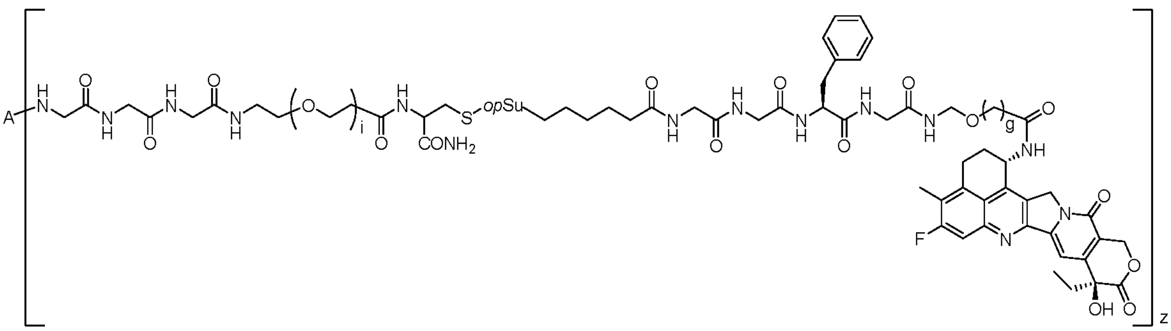

,

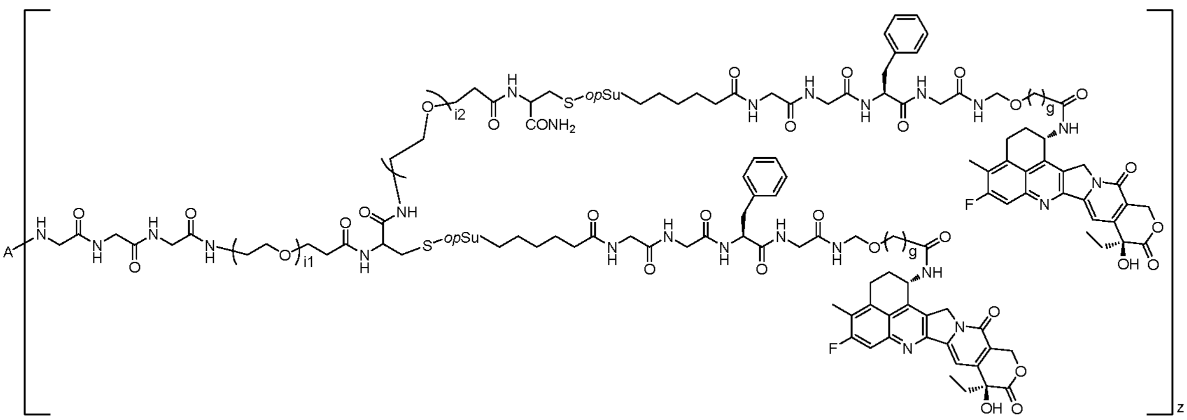

,

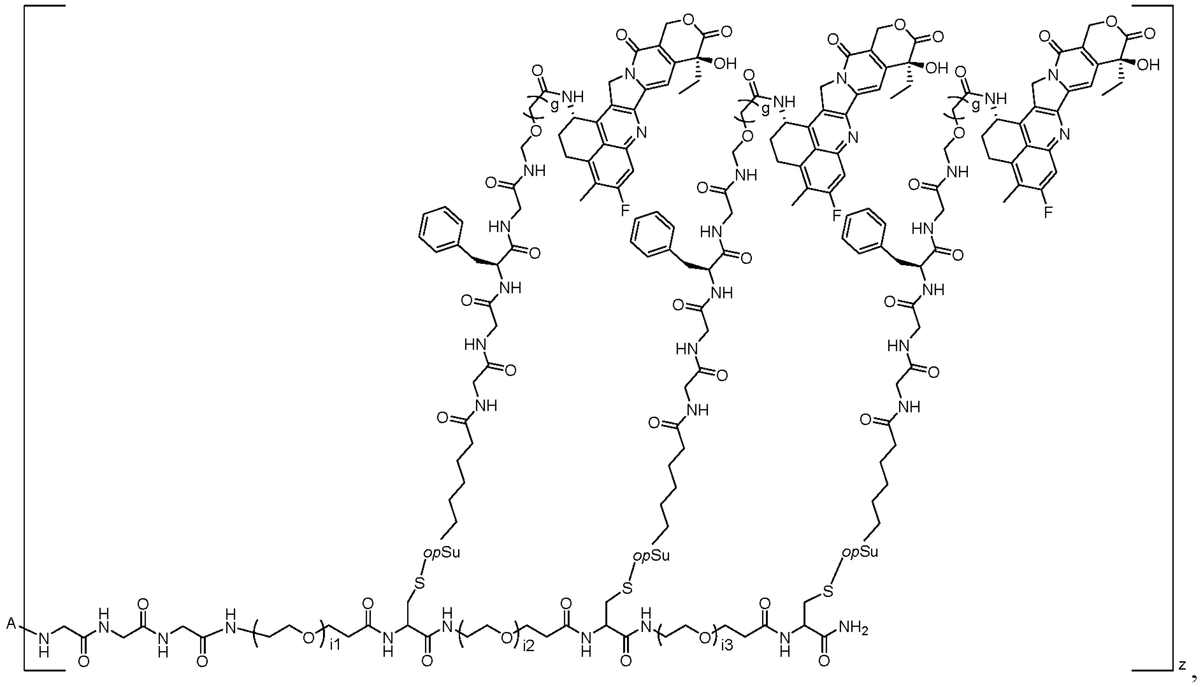

,

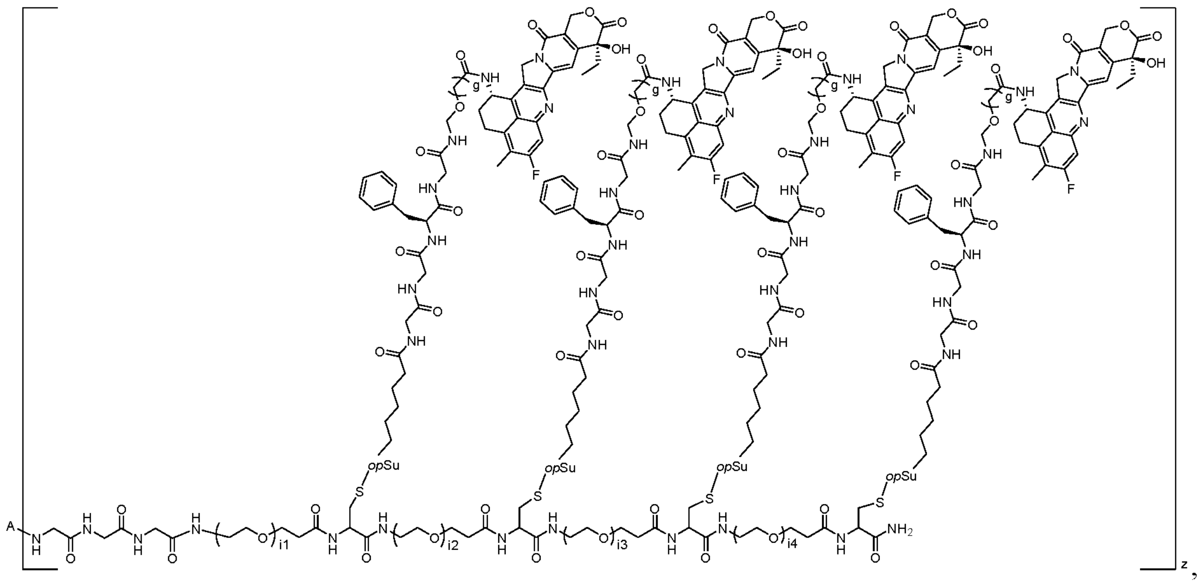

,

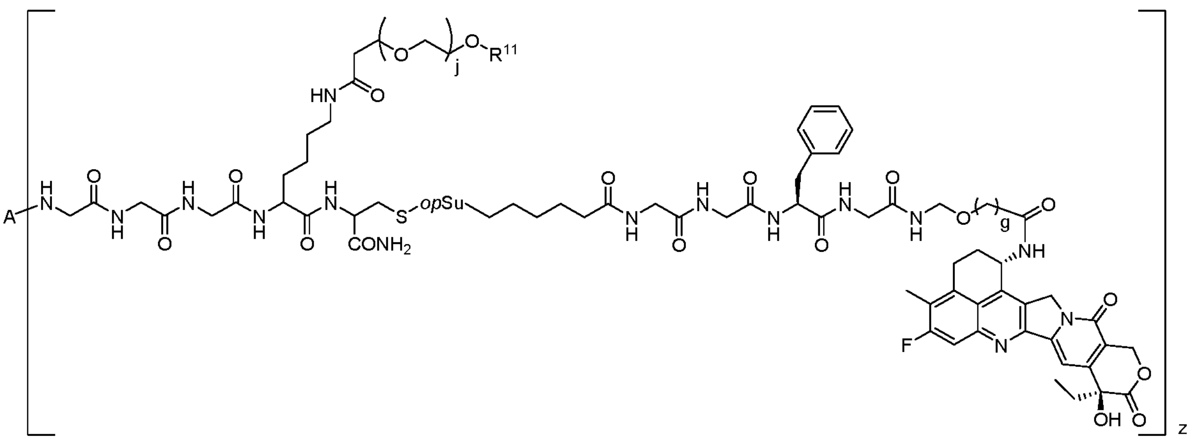

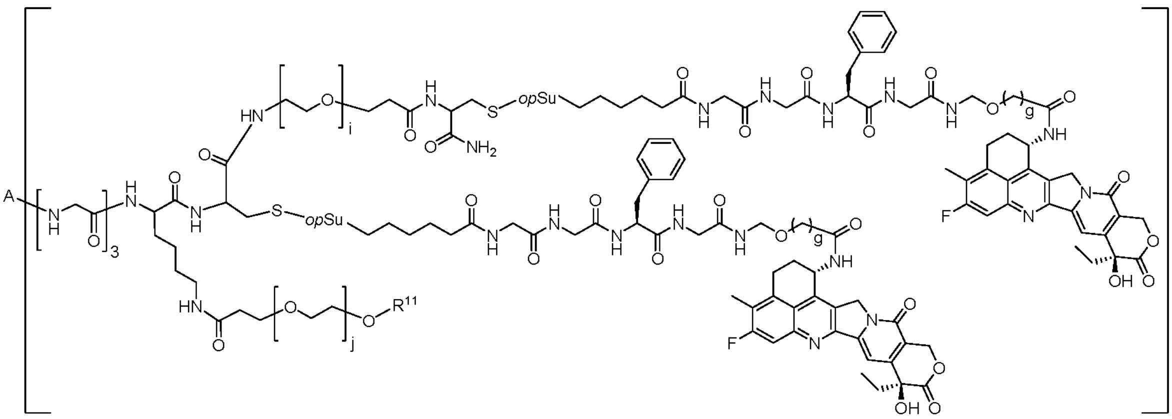

z,

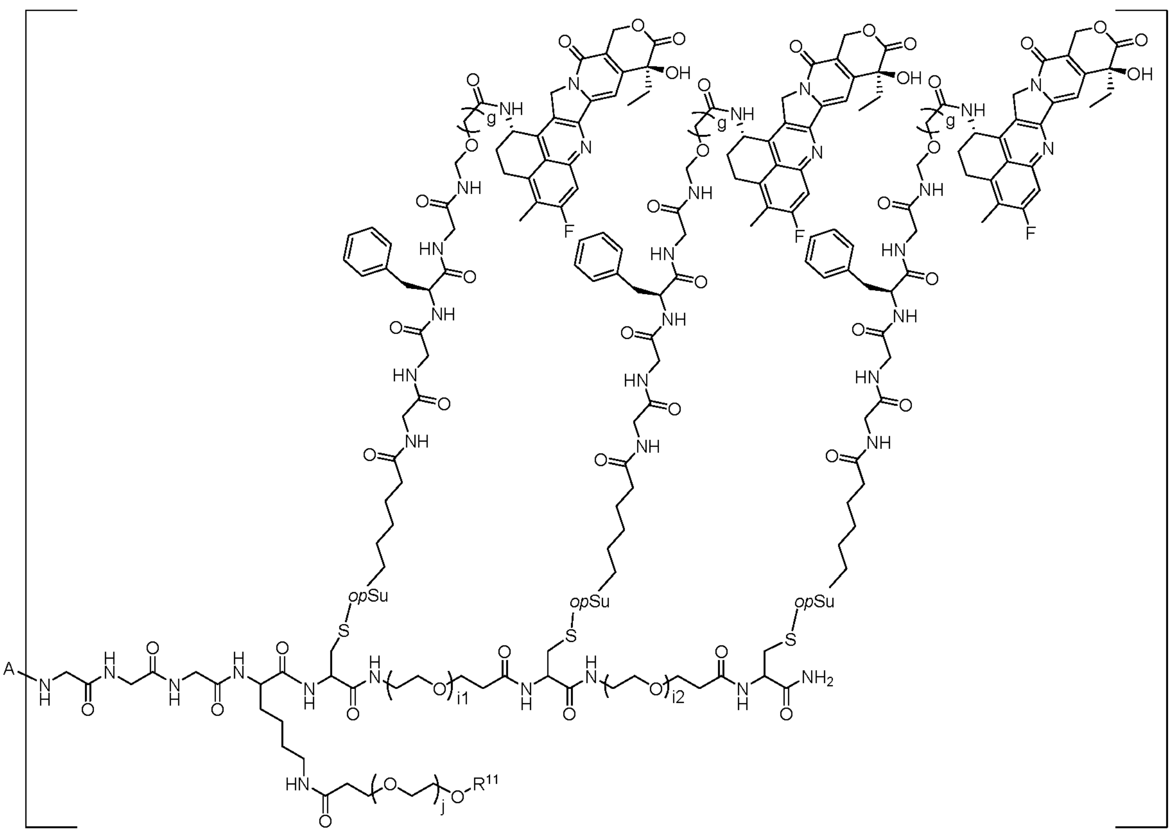

z,

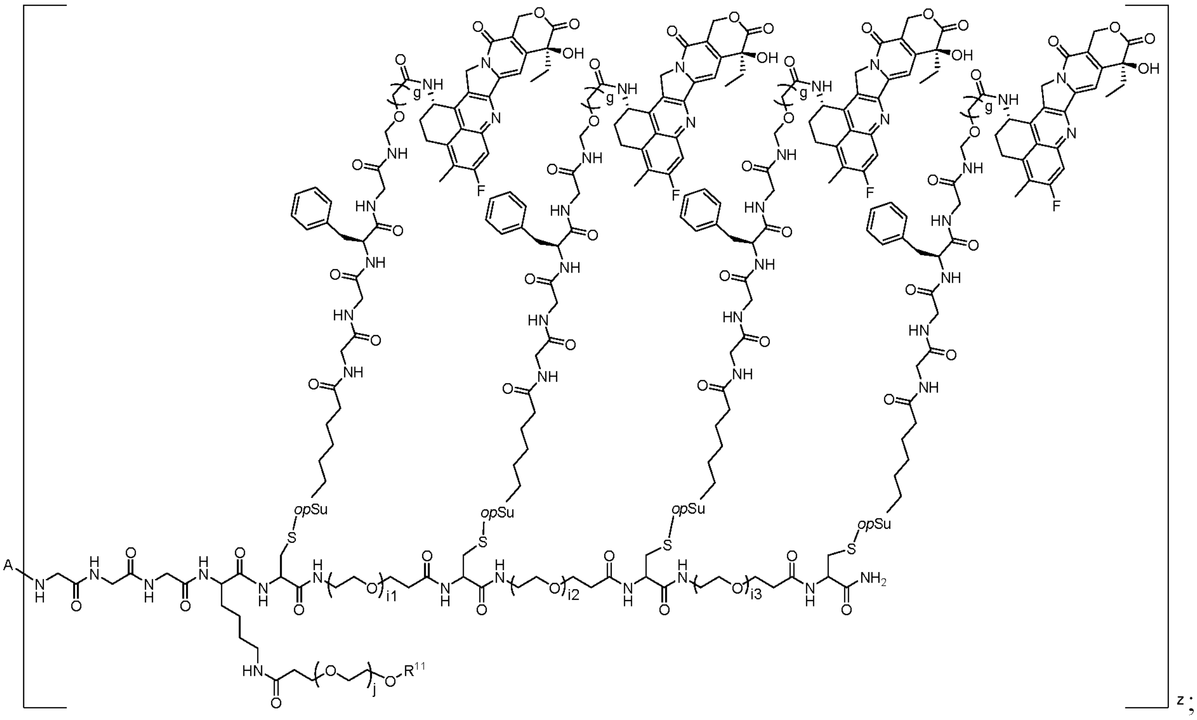

each g is independently an integer from 1 to 6, preferably from 1 to 3, more preferably 1.

**6.** The pharmaceutical composition according to any one of claims 1-5, wherein the buffering agent forms into a buffer, wherein the buffer is selected from an acetic acid buffer, a histidine buffer, a citric acid buffer, and a phosphoric acid buffer; preferably, the buffer is an acetic acid buffer or a histidine buffer, further more preferably a histidine buffer; preferably, the concentration of the buffer is 10-50 mM; preferably, the pH of the buffer is 5.0-6.5 or 5.5-6.4.

**7.** The pharmaceutical composition according to any one of claims 1-6, wherein the stabilizer is selected from sucrose and trehalose, preferably sucrose; preferably, the concentration of the stabilizer is 1-20% (w:v).

**8.** The pharmaceutical composition according to any one of claims 1-8, wherein the pH of the pharmaceutical composition is 5.0-6.5, preferably 5.2-6.0, more preferably about 5.5.

**9.** The pharmaceutical composition according to any one of claims 1-8, wherein the surfactant is a polysorbate; preferably, the concentration of the surfactant is 0.01-10 mg/mL.

**10.** A pharmaceutical formulation comprising an active ingredient of formula (III), comprising:

20 mM acetic acid buffer at pH 5.0, 9% (w:v) sucrose, 20 mg/mL active ingredient; or
20 mM histidine buffer at pH 5.5, 9% (w:v) sucrose, 20 mg/mL active ingredient; or
20 mM histidine buffer at pH 6.0, 9% (w:v) sucrose, 20 mg/mL active ingredient; or
20 mM citric acid buffer at pH 6.0, 9% (w:v) sucrose, 20 mg/mL active ingredient; or
20 mM phosphoric acid buffer at pH 7.0, 9% (w:v) sucrose, 20 mg/mL active ingredient; or
20 mM histidine buffer at pH 5.5, 8.1% (w:v) trehalose, 0.2 mg/mL polysorbate 20, 20 mg/mL active ingredient; or
20 mM histidine buffer at pH 5.5, 9% (w:v) sucrose, 0.2 mg/mL polysorbate 20, 20 mg/mL active ingredient; or
20 mM histidine buffer at pH 5.5, 9% (w:v) sucrose, 0.2 mg/mL polysorbate 80, 20 mg/mL active ingredient;
wherein the pharmaceutical formulation is a powder formulation or a liquid formulation.

**11.** Use of the pharmaceutical composition according to any one of claims 1-10 in the manufacture of a medicament for treating a disease, wherein the disease is a tumor or an autoimmune disease, preferably an HER2-positive tumor; preferably, the HER2-positive tumor is selected from breast cancer, gastric cancer, lung cancer, ovarian cancer, and urothelial cancer.

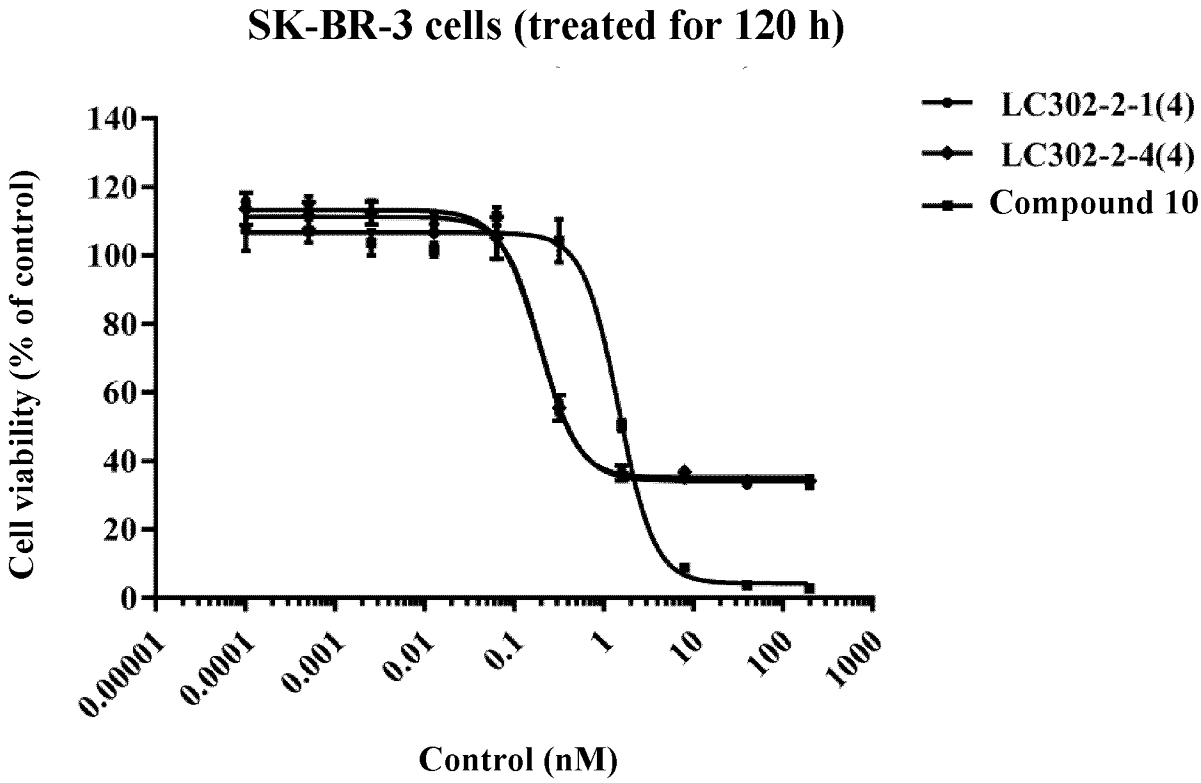
SK-BR-3 cells (treated for 120 h)
LC302-2-1(4)
LC302-2-4(4)
Compound 10
Cell viability (% of control)
140
120
100
80
60
40
20
0
0.00001
0.0001
0.001
0.01
0.1
1
10
100
1000
Control (nM)

FIG. 1

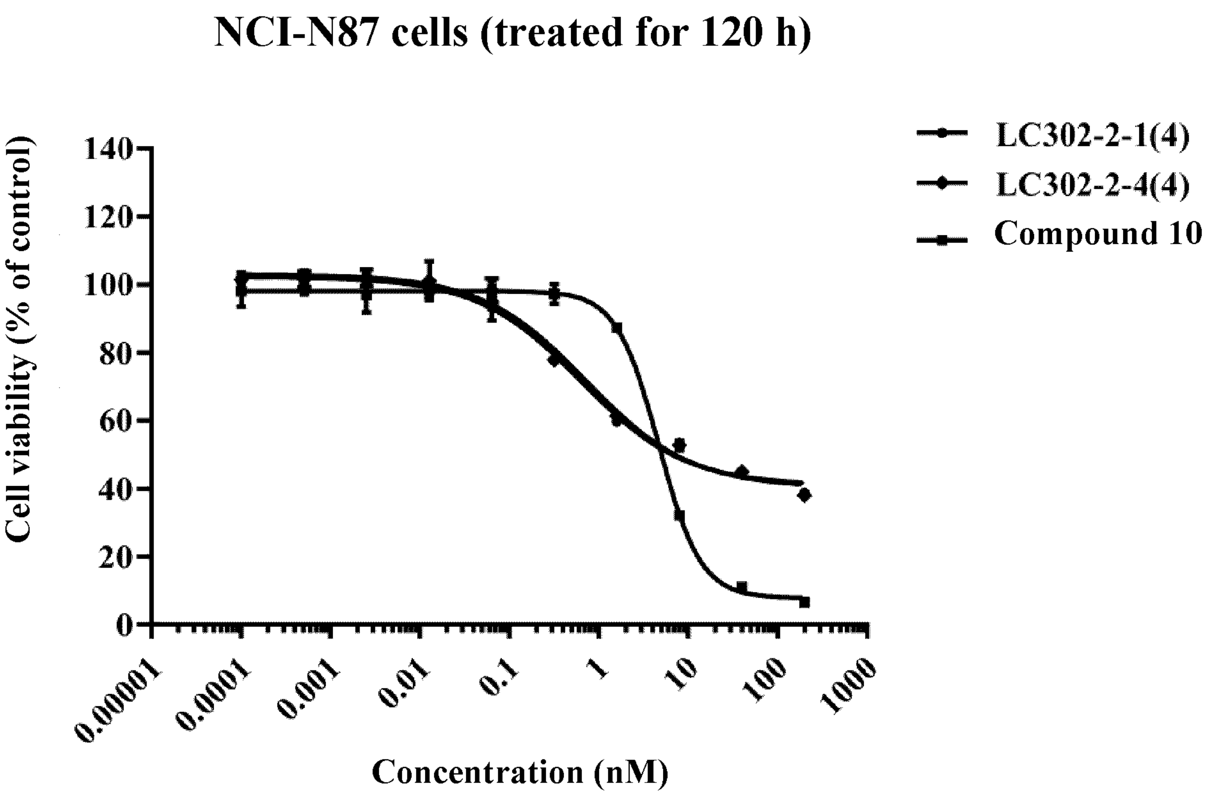
NCI-N87 cells (treated for 120 h)
Cell viability (% of control)
140
120
100
80
60
40
20
0
0.00001
0.0001
0.001
0.01
0.1
1
10
100
1000
Concentration (nM)
LC302-2-1(4)
LC302-2-4(4)
Compound 10

FIG. 2

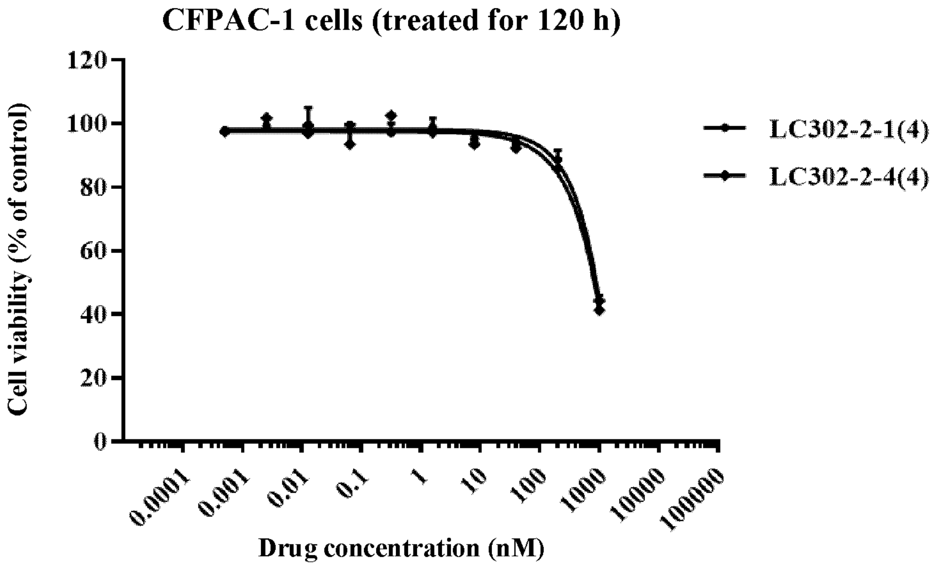
CFPAC-1 cells (treated for 120 h)
Cell viability (% of control)
120
100
80
60
40
20
0
0.0001
0.001
0.01
0.1
1
10
100
1000
10000
100000
Drug concentration (nM)
LC302-2-1(4)
LC302-2-4(4)

FIG. 3

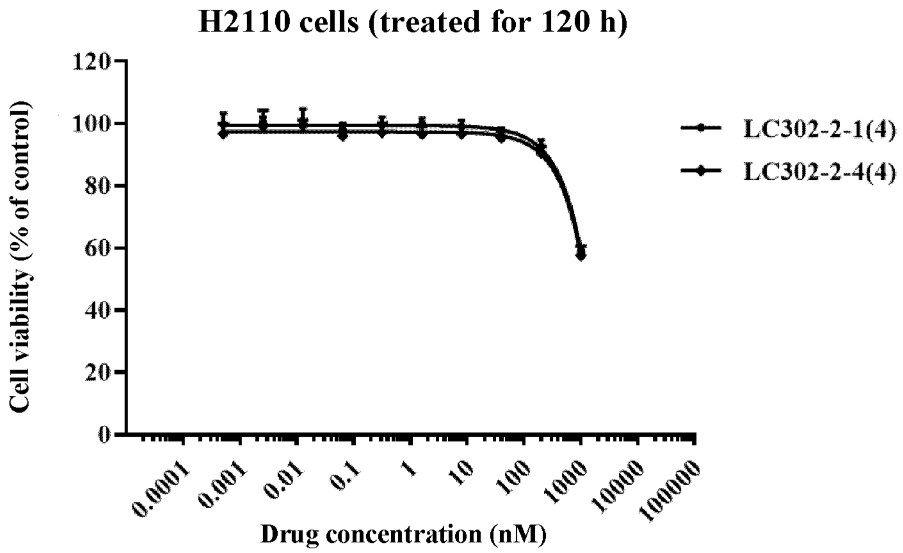
H2110 cells (treated for 120 h)
Cell viability (% of control)
120
100
80
60
40
20
0
0.0001
0.001
0.01
0.1
1
10
100
1000
10000
100000
Drug concentration (nM)
LC302-2-1(4)
LC302-2-4(4)

FIG. 4

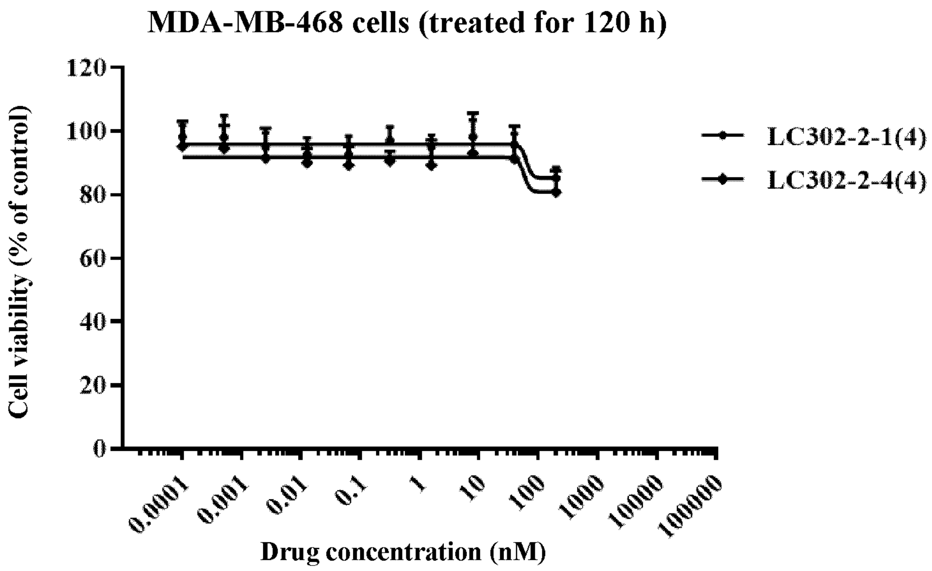
MDA-MB-468 cells (treated for 120 h)
Cell viability (% of control)
120
100
80
60
40
20
0
0.0001
0.001
0.01
0.1
1
10
100
1000
10000
100000
Drug concentration (nM)
LC302-2-1(4)
LC302-2-4(4)

FIG. 5

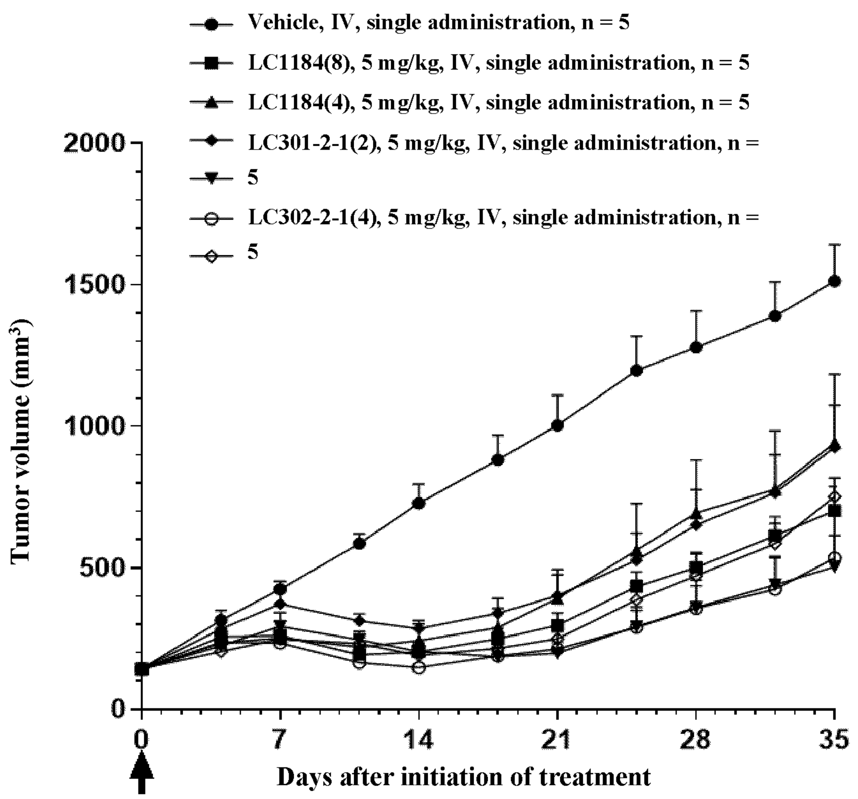
Vehicle, IV, single administration, n = 5
LC1184(8), 5 mg/kg, IV, single administration, n = 5
LC1184(4), 5 mg/kg, IV, single administration, n = 5
LC301-2-1(2), 5 mg/kg, IV, single administration, n =
5
LC302-2-1(4), 5 mg/kg, IV, single administration, n =
5
Tumor volume (mm³)
2000
1500
1000
500
0
0
7
14
21
28
35
Days after initiation of treatment

FIG. 6

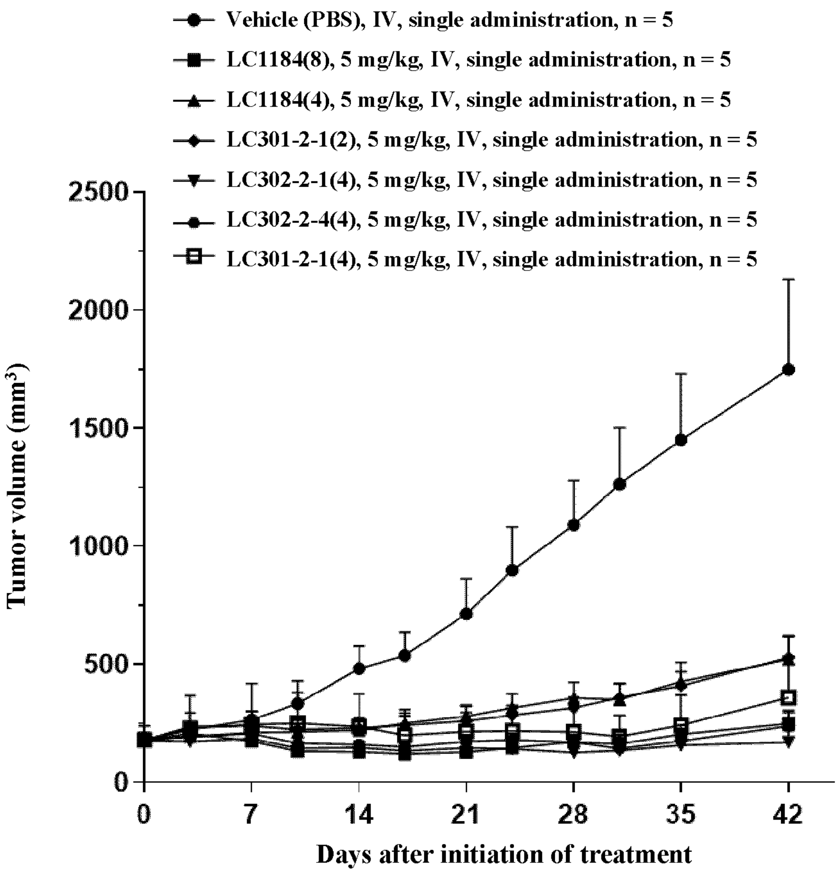
Vehicle (PBS), IV, single administration, n = 5
LC1184(8), 5 mg/kg, IV, single administration, n = 5
LC1184(4), 5 mg/kg, IV, single administration, n = 5
LC301-2-1(2), 5 mg/kg, IV, single administration, n = 5
LC302-2-1(4), 5 mg/kg, IV, single administration, n = 5
LC302-2-4(4), 5 mg/kg, IV, single administration, n = 5
LC301-2-1(4), 5 mg/kg, IV, single administration, n = 5
Tumor volume (mm3)
2500
2000
1500
1000
500
0
0
7
14
21
28
35
42
Days after initiation of treatment

FIG. 7

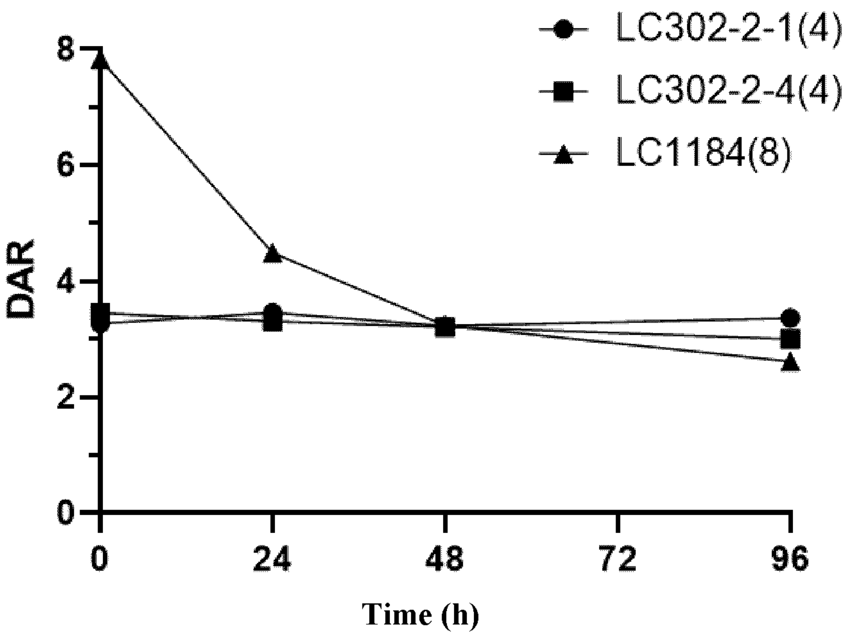
LC302-2-1(4)
LC302-2-4(4)
LC1184(8)
DAR
8
6
4
2
0
0
24
48
72
96
Time (h)

FIG. 8

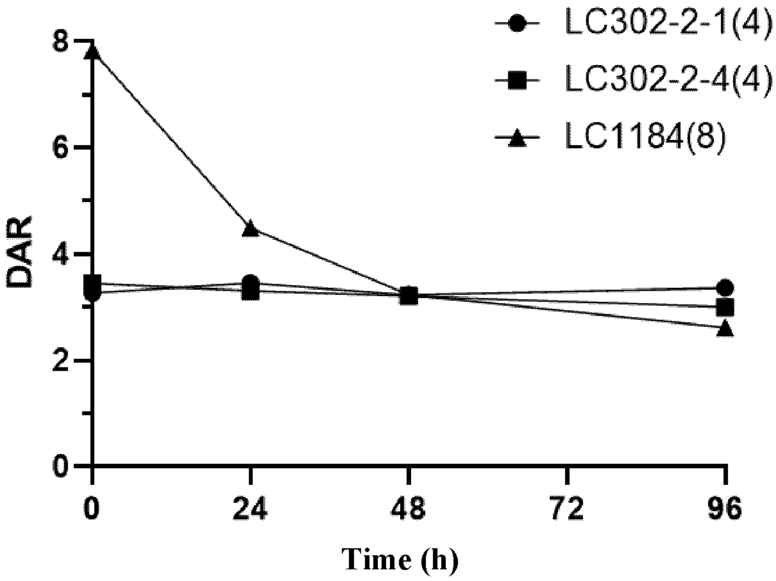
LC302-2-1(4)
LC302-2-4(4)
LC1184(8)
DAR
8
6
4
2
0
0
24
48
72
96
Time (h)

## INTERNATIONAL SEARCH REPORT

International application No.
**PCT/CN2023/114566**

**A. CLASSIFICATION OF SUBJECT MATTER**

A61K47/68(2017.01)i； A61K47/65(2017.01)i； A61K9/08(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K A61P

Documentation searched other than minimum documentation to the extent that such documents are included in the fields searched

Electronic data base consulted during the international search (name of data base and, where practicable, search terms used)

CNABS; CNTXT; ENTXT; DWPI; STNext Registry; STNext Caplus; CNKI: 启德医药; 李雪松; 秦刚; 抗体药物偶联物; 连接子; 结构检索; 组氨酸; 蔗糖; 海藻糖; 聚山梨酯; ADC; linker; GGFC; histidine; sucrose; trehalose; polysorbate; tween

**C. DOCUMENTS CONSIDERED TO BE RELEVANT**

| Category* | Citation of document, with indication, where appropriate, of the relevant passages | Relevant to claim No. |
|---|---|---|
| PY | WO 2022218331 A1 (GENEQUANTUM HEALTHCARE (SUZHOU) CO., LTD.) 20 October 2022 (2022-10-20)<br>claims 1-17 | 1-11 |
| PY | WO 2023088235 A1 (GENEQUANTUM HEALTHCARE (SUZHOU) CO., LTD.) 25 May 2023 (2023-05-25)<br>claims 1-20 | 1-11 |
| Y | WO 2015165413 A1 (QIN GANG et al.) 05 November 2015 (2015-11-05)<br>claims 11-22 and 33-34, and description, page 1, and figures 26 and 32 | 1-11 |
| Y | CN 110944667 A (DAIICHI SANKYO COMPANY, LIMITED) 31 March 2020 (2020-03-31)<br>claims 1 and 7-8, and description, paragraphs 0201 and 0204 | 1-11 |
| Y | CN 112566942 A (DAIICHI SANKYO COMPANY, LIMITED) 26 March 2021 (2021-03-26)<br>claims 42-59 | 1-11 |
| A | WO 2014177042 A1 (QIN GANG) 06 November 2014 (2014-11-06)<br>entire document | 1-11 |

☐ Further documents are listed in the continuation of Box C.　　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 December 2023** | **20 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/114566**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/CN2023/114566**

| Patent document cited in search report | Publication date (day/month/year) | Patent family member(s) | Publication date (day/month/year) |
|---|---|---|---|
| WO 2022218331 A1 | 20 October 2022 | US 2022395581 A1 | 15 December 2022 |
| WO 2023088235 A1 | 25 May 2023 | EP 4211145 A1 | 19 July 2023 |
| | | US 2023271977 A1 | 31 August 2023 |
| | | US 11814394 B2 | 14 November 2023 |
| | | AU 2022393856 A1 | 03 August 2023 |
| | | AU 2022393856 A9 | 24 August 2023 |
| | | AU 2022393856 B2 | 23 November 2023 |
| WO 2015165413 A1 | 05 November 2015 | US 2017112944 A1 | 27 April 2017 |
| | | US 11040084 B2 | 22 June 2021 |
| | | EP 3517114 A1 | 31 July 2019 |
| | | JP 2017514812 A | 08 June 2017 |
| | | JP 6666264 B2 | 13 March 2020 |
| | | JP 2020055828 A | 09 April 2020 |
| | | JP 7100617 B2 | 13 July 2022 |
| | | WO 2015165413 A8 | 14 April 2016 |
| | | ES 2736505 T3 | 02 January 2020 |
| | | EP 3138568 A1 | 08 March 2017 |
| | | EP 3138568 A4 | 13 December 2017 |
| | | EP 3138568 B1 | 17 April 2019 |
| | | US 2021177929 A1 | 17 June 2021 |
| | | AU 2020200975 A1 | 27 February 2020 |
| | | AU 2020200975 B2 | 28 January 2021 |
| | | US 2021401924 A1 | 30 December 2021 |
| | | AU 2015252518 A1 | 15 December 2016 |
| | | AU 2015252518 A2 | 14 November 2019 |
| | | KR 20170020328 A | 22 February 2017 |
| | | KR 102511249 B1 | 20 March 2023 |
| CN 110944667 A | 31 March 2020 | JPWO 2019039483 A1 | 01 October 2020 |
| | | JP 7204651 B2 | 16 January 2023 |
| | | WO 2019039483 A1 | 28 February 2019 |
| | | RU 2020111448 A | 24 September 2021 |
| | | RU 2020111448 A3 | 29 November 2021 |
| | | JP 2023036900 A | 14 March 2023 |
| | | EP 3673918 A1 | 01 July 2020 |
| | | EP 3673918 A4 | 19 May 2021 |
| | | IL 272721 A | 30 April 2020 |
| | | KR 20200044044 A | 28 April 2020 |
| | | MA 49987 A | 01 July 2020 |
| | | US 2021128741 A1 | 06 May 2021 |
| | | TW 201919710 A | 01 June 2019 |
| | | SG 11202000996 UA | 30 March 2020 |
| | | BR 112020003474 A2 | 20 October 2020 |
| | | CA 3073383 A1 | 28 February 2019 |
| | | CA 3073383 C | 31 October 2023 |
| | | AU 2018320470 A1 | 13 February 2020 |
| CN 112566942 A | 26 March 2021 | AU 2019311557 A1 | 04 February 2021 |
| | | CA 3107417 A1 | 30 January 2020 |
| | | CA 3107417 C | 17 October 2023 |
| | | KR 20210038904 A | 08 April 2021 |
| | | IL 280295 A | 25 March 2021 |

Form PCT/ISA/210 (patent family annex) (July 2022)

**INTERNATIONAL SEARCH REPORT**
**Information on patent family members**

International application No.
**PCT/CN2023/114566**

| Patent document cited in search report | Publication date (day/month/year) | Patent family member(s) | Publication date (day/month/year) |
|---|---|---|---|
| | | SG 11202100653 YA | 25 February 2021 |
| | | TW 202019972 A | 01 June 2020 |
| | | US 2021283269 A1 | 16 September 2021 |
| | | EP 3828206 A1 | 02 June 2021 |
| | | EP 3828206 A4 | 20 April 2022 |
| | | JPWO 2020022363 A1 | 02 August 2021 |
| | | BR 112021001194 A2 | 27 April 2021 |
| | | WO 2020022363 A1 | 30 January 2020 |
| WO 2014177042 A1 | 06 November 2014 | GB 201520943 D0 | 13 January 2016 |
| | | GB 2529356 A | 17 February 2016 |
| | | GB 2529356 B | 23 December 2020 |
| | | US 2021187114 A1 | 24 June 2021 |
| | | JP 2018138588 A | 06 September 2018 |
| | | JP 2020079262 A | 28 May 2020 |
| | | JP 2016520574 A | 14 July 2016 |
| | | US 2016193355 A1 | 07 July 2016 |
| | | US 2018104349 A9 | 19 April 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015165413 A1
- US 20110321183 A1
- US 20060229253 A
- EP 2907824 A